(19) Europäisches Patentamt
European Patent Office
Office européen des brevets

(11) EP 2 133 095 A1

(12) **EUROPEAN PATENT APPLICATION**
published in accordance with Art. 153(4) EPC

(43) Date of publication:
**16.12.2009 Bulletin 2009/51**

(21) Application number: **08721329.4**

(22) Date of filing: **05.03.2008**

(51) Int Cl.:
***A61K 45/06*** *(2006.01)* ***A61K 31/136*** *(2006.01)*
***A61K 31/198*** *(2006.01)* ***A61K 31/416*** *(2006.01)*
***A61K 31/436*** *(2006.01)* ***A61K 31/4439*** *(2006.01)*
***A61K 31/454*** *(2006.01)* ***A61K 31/4745*** *(2006.01)*
***A61K 31/475*** *(2006.01)* ***A61K 31/496*** *(2006.01)*
***A61K 31/506*** *(2006.01)* ***A61K 31/513*** *(2006.01)*
***A61K 31/5377*** *(2006.01)* ***A61K 31/69*** *(2006.01)*
***A61K 31/704*** *(2006.01)* ***A61K 31/7048*** *(2006.01)*
***A61K 31/7068*** *(2006.01)* ***A61K 38/00*** *(2006.01)*
***A61K 39/395*** *(2006.01)* ***A61P 35/00*** *(2006.01)*
***A61P 35/02*** *(2006.01)*

(86) International application number:
**PCT/JP2008/053909**

(87) International publication number:
**WO 2008/111441 (18.09.2008 Gazette 2008/38)**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **05.03.2007 JP 2007053676**

(71) Applicant: **Kyowa Hakko Kirin Co., Ltd.**
**Tokyo 100-8185 (JP)**

(72) Inventors:
- **SHIOTSU, Yukimasa**
  **Shizuoka 411-8731 (JP)**
- **ISHII, Kenichi**
  **Shizuoka 411-8731 (JP)**
- **ISHIDA, Hiroyuki**
  **Shizuoka 411-8731 (JP)**
- **SHIMIZU, Makiko**
  **Shizuoka 411-8731 (JP)**

(74) Representative: **Simcox, Michael Thomas et al**
**D Young & Co**
**120 Holborn**
**London EC1N 2DY (GB)**

## (54) PHARMACEUTICAL COMPOSITION

(57) The present invention provides a pharmaceutical composition comprising a combination of an Flt-3 inhibitor and at least one compound, the said pharmaceutical composition wherein an Flt-3 inhibitor is an indazole derivative represented by Formula (I)

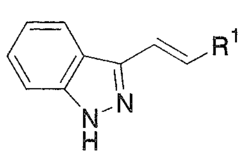
(I)

(wherein $R^1$ represents suctituted or unsubstituted aryl, or the like) or a pharmaceutically acceptable salt thereof, the said pharmaceutical composition wherein an Flt-3 inhibitor is a pyrimidine derivative represented by Formula (II)

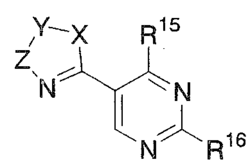
(II)

[wherein -X-Y-Z- represents $-O-CR^{17}=N-$ (wherein $R^{17}$ represents a hydrogen atom, lower alkyl, or the like), $R^{15}$ represents $-NR^{22a}R^{22b}$ (wherein $R^{22a}$ and $R^{22b}$ may be the same or different and each represents a hydrogen atom, substituted or unsubstituted lower alkyl, or the like), and $R^{16}$ represents $-NR^{24a}R^{24b}$ (wherein $R^{24a}$ and $R^{24b}$ may be the same or different and each represents a hydrogen atom, substituted or unsubstituted lower alkyl, or the like), or the like] or a pharmaceutically acceptable salt thereof, or the like.

EP 2 133 095 A1

Figure 10

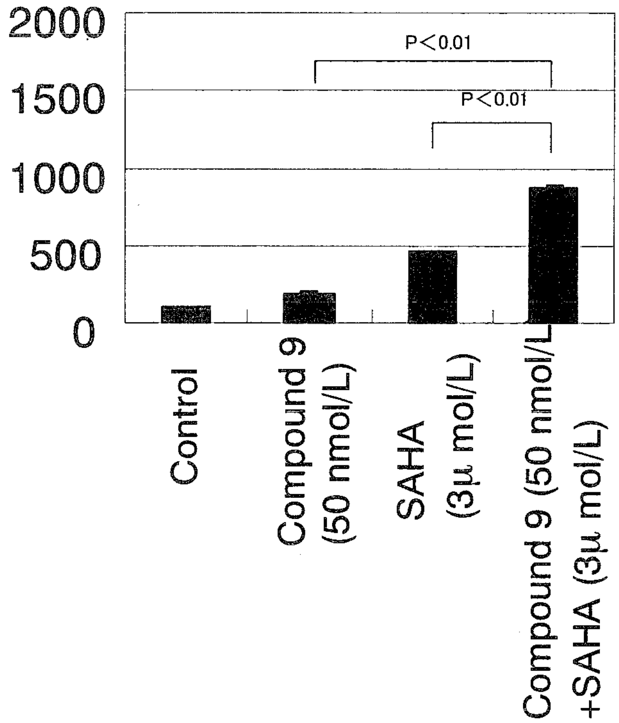
2000
1500
1000
500
0
P<0.01
P<0.01
Control
Compound 9 (50 nmol/L)
SAHA (3μ mol/L)
Compound 9 (50 nmol/L +SAHA (3μ mol/L)

## Description

### Technical Field

The present invention relates to a pharmaceutical composition comprising a combination of an Fms like tyrosine kinase 3 (Flt-3) inhibitor and at least one compound, and the like.

### Background Art

Fms like tyrosine kinase 3 (hereinafter referred to as "Flt-3") is a receptor-type protein tyrosine kinase (PTK) which belongs to the platelet-derived growth factor receptor (PDGFR) family, and is an enzyme which is activated by dimerization by the binding of its ligand, i.e., Flt-3 ligand, and which phosphorylates various proteins that are intracellular substrates, thus participating in cell growth and differentiation. It is known that the Flt-3 is specifically expressed in hematopoietic stem cells, and the Flt-3 or Flk-2 (fetal liver kinase-2) plays an important role in the growth thereof [Cell, vol. 65, 1143 (1991)]. Furthermore, recently, as a result of the examination of samples obtained from leukemia patients, it has been shown that the Flt-3 is activated, without binding of the ligand, by tyrosine residue internal tandem duplication (ITD) mutations in the Flt-3 juxtamembrane domain [Leukemia, vol. 11, 1447 (1997)]. It has also been shown that the Flt-3 is activated in a similar manner by mutations in which the amino acid sequence is lengthened or shortened in the Flt-3 juxtamembrane domain [Blood, vol. 96, 3907 (2000)]. In addition, it has been shown that the Flt-3 is constitutively activated by point mutations of amino acids in the Flt-3 kinase domain [Blood, vol. 97, 2434 (2001)]. Constitutive activation due to the mutations in the Flt-3 is considered to induce infinite cell growth by transmission of cell growth signals and be a major cause of leukemia.

As described above, examples of currently known mutations in the Flt-3 include tyrosine residue internal tandem duplications in the juxtamembrane domain, changes in the length of the juxtamembrane domain, point mutations of amino acids in the Flt-3 kinase domain, and the like. It is known that introduction of these mutant genes into cytokine-dependent cell lines, such as 32D cells, can provide cytokine-independent growth potency. Consequently, Flt-3 inhibitors are considered to be useful as therapeutic agents for treating various cancers, such as leukemia.

Indazole derivatives used in the present invention are known as protein kinase inhibitors, antitumor agents, etc. (Patent Documents 1 and 2). Furthermore, related indazole derivatives are known [Japanese Published Unexamined Patent Application No. 32059/1990, WO01/53268, WO02/10137, WO01/02369, WO02/083648, WO03/101968, W02004/094388, W02004/050088, W02005/0137171, WO2005/094823, and Khimiya Geterotsiklicheskikh Soedinenii, Vol. 7, 957 (1978)].

Pyrimidine derivatives used in the present invention are known as antitumor agents (Patent Document 3). Furthermore, related pyrimidine derivatives are known (WO92/01675, WO94/14780, Japanese Published Unexamined Patent Application No. 87492/1998, WO99/19305, WO99/32117, US5,935,966, WO99/41253, WO00/39101, WO01/17995, WO02/20495, WO02/22601, WO02/22602, WO02/22608, WO03/30909, WO02/62789, WO02/30358, WO01/00213, and Japanese translation of PCT international application No. 2003-523942).

Isoindolinone and phthalimide derivatives used in the present invention are known as protein kinase inhibitors, antitumor agents, etc. (Patent Document 4). Furthermore, related isoindolinone and phthalimide derivatives are known [German Published Patent Application No. 2141063, WO04/108672, WO05/039564, Heterocycles, vol. 45, 2217 (1997); and Bioorganic & Medicinal Chemistry Letters, vol. 14, 4505 (2004)].

At present, in most cases, treatment of cancers with a single anticancer agent produces limited effect, and therefore, in order to achieve maximum therapeutic effect, multiagent chemotherapy is used. The aims of multiagent chemotherapy are as follows: firstly, to enhance the antitumor effect by use of a plurality of agents; secondly, in the case where a plurality of cancer cells have emerged, to administer an effective second agent to cancer cells in which a first agent is ineffective, i.e., to broaden a spectrum of anticancer activity against a diversity of cancer cells; and thirdly, as a result of the first and second points, to avoid or delay the emergence of resistant cells (Clinical oncology, third edition, Japanese Journal of Cancer and Chemotherapy Publishers Inc). For example, it has been reported that, by using Lestaurtinib (CEP-701), PKC412, or the like, which is an indolocarbazole derivative, in combination with chemotherapy, an HDAC inhibitor, or an m-TOR inhibitor against acute myelogenous leukemia cells, combined effects are demonstrated [Clinical Cancer Research, vol. 10, 4991 (2004); Blood, vol. 104, 1145 (2004); and Proceeding of National Academy of Science of the United States of America, vol. 101, 3130 (2004)]. The efficacy of combining Sunitinib (SU011248), which is an indolinone derivative, with chemotherapy or a molecular targeted drug against blood cancers and solid cancers has been reported [Blood, vol. 104, 4202 (2004), and WO06/120557].

Patent Document 1: WO2005/012257
Patent Document 2: WO2005/012258
Patent Document 3: WO2005/095382

Patent Document 4: WO2005/095341

Disclosure of the Invention

Problems to be solved by the Invention

An object of the present invention is to provide a pharmaceutical composition comprising a combination of an Flt-3 inhibitor and at least one compound, and the like.

Means for Solving the Problems

The present invention relates to the following (1) to (66).

(1) A pharmaceutical composition comprising a combination of an Flt-3 inhibitor and at least one compound.
(2) A pharmaceutical composition for administering a combination of an Flt-3 inhibitor and at least one compound.
(3) A pharmaceutical composition for administering an Flt-3 inhibitor and at least one compound simultaneously or successively.
(4) The pharmaceutical composition according to any of the above (1) to (3), wherein the Flt-3 inhibitor is an indazole derivative represented by Formula (I)

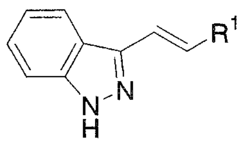
(I)

(wherein $R^1$ represents substituted or unsubstituted aryl or a substituted or unsubstituted heterocyclic group) or a pharmaceutically acceptable salt thereof.
(5) The pharmaceutical composition according to any of the above (1) to (3), wherein the Flt-3 inhibitor is an indazole derivative represented by Formula (Ia)

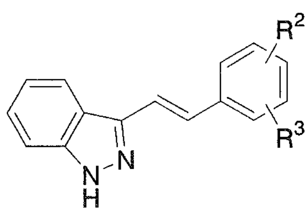
(Ia)

[wherein $R^2$ represents $CONR^{4a}R^{4b}$ (wherein $R^{4a}$ and $R^{4b}$ may be the same or different and each represents a hydrogen atom, substituted or unsubstituted lower alkyl, substituted or unsubstituted aryl, substituted or unsubstituted aralkyl, or a substituted or unsubstituted heterocyclic group, or $R^{4a}$ and $R^{4b}$ are combined together with the adjacent nitrogen atom thereto to form a substituted or unsubstituted heterocyclic group) or $NR^{5a}R^{5b}$ (wherein $R^{5a}$ represents substituted or unsubstituted lower alkylsulfonyl or substituted or unsubstituted arylsulfonyl and $R^{5b}$ represents a hydrogen atom or substituted or unsubstituted lower alkyl) and $R^3$ represents a hydrogen atom, halogen, cyano, nitro, hydroxy, carboxy, lower alkoxycarbonyl, substituted or unsubstituted lower alkyl, substituted or unsubstituted lower alkoxy, substituted or unsubstituted lower alkanoyl, $CONR^{6a}R^{6b}$ (wherein $R^{6a}$ and $R^{6b}$ may be the same or different and each represents a hydrogen atom, substituted or unsubstituted lower alkyl, substituted or unsubstituted aryl, substituted or unsubstituted aralkyl or a substituted or unsubstituted heterocyclic group, or $R^{6a}$ and $R^{6b}$ are combined together with the adjacent nitrogen atom thereto to form a substituted or unsubstituted heterocyclic group) or $NR^{7a}R^{7b}$ (wherein $R^{7a}$ and $R^{7b}$ may be the same or different and each represents a hydrogen atom, substituted or unsubstituted lower alkyl, substituted or unsubstituted lower alkanoyl, substituted or unsubstituted aroyl, substituted or unsubstituted heteroaroyl, substituted or unsubstituted aralkyl, substituted or unsubstituted lower alkylsulfonyl or substituted or unsubstituted arylsulfonyl)] or a pharmaceutically acceptable salt thereof.

(6) The pharmaceutical composition according to the above (5), wherein $R^2$ is $CONR^{4a}R^{4b}$ (wherein $R^{4a}$ and $R^{4b}$ have the same meanings as defined above, respectively) and $R^3$ is a hydrogen atom.

(7) The pharmaceutical composition according to the above (5), wherein $R^2$ is $NR^{5a}R^{5b}$ (wherein $R^{5a}$ and $R^{5b}$ have the same meanings as defined above, respectively) and $R^3$ is substituted or unsubstituted lower alkoxy.

(8) The pharmaceutical composition according to the above (5), wherein $R^2$ is $NR^{5a}R^{5b}$ (wherein $R^{5a}$ and $R^{5b}$ have the same meanings as defined above, respectively) and $R^3$ is a hydrogen atom.

(9) The pharmaceutical composition according to any of the above (1) to (3), wherein the Flt-3 inhibitor is an indazole derivative represented by Formula (Ib)

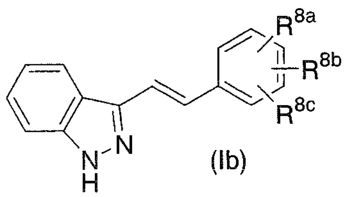

(Ib)

[wherein $R^{8a}$, $R^{8b}$ and $R^{8c}$ may be the same or different and each represents a hydrogen atom, halogen, nitro, nitroso, carboxy, cyano, substituted or unsubstituted lower alkyl, substituted or unsubstituted lower alkanoyl, substituted or unsubstituted lower alkoxycarbonyl, substituted or unsubstituted aryl, $NR^{9a}R^{9b}$ (wherein $R^{9a}$ and $R^{9b}$ may be the same or different and each represents a hydrogen atom, substituted or unsubstituted lower alkyl, substituted or unsubstituted lower alkenyl, substituted or unsubstituted lower alkynyl, substituted or unsubstituted lower alkoxy, substituted or unsubstituted lower alkanoyl, substituted or unsubstituted aryl, substituted or unsubstituted aroyl, a substituted or unsubstituted heterocyclic group or substituted or unsubstituted heteroaroyl, or $R^{9a}$ and $R^{9b}$ are combined together with the adjacent nitrogen atom thereto to form a substituted or unsubstituted heterocyclic group) or $OR^{10}$ (wherein $R^{10}$ represents a hydrogen atom, substituted or unsubstituted lower alkyl, substituted or unsubstituted aryl, substituted or unsubstituted aroyl or a substituted or unsubstituted heterocyclic group)] or a pharmaceutically acceptable salt thereof.

(10) The pharmaceutical composition according to the above (9), wherein at least one of $R^{8a}$, $R^{8b}$ and $R^{8c}$ is $NR^{9a}R^{9b}$ (wherein $R^{9a}$ and $R^{9b}$ have the same meanings as defined above, respectively).

(11) The pharmaceutical composition according to the above (9), wherein at least one of $R^{8a}$, $R^{8b}$ and $R^{8c}$ is $NR^{9c}R^{9d}$ (wherein $R^{9c}$ and $R^{9d}$ may be the same or different and each represents a hydrogen atom or substituted or unsubstituted lower alkanoyl, or $R^{9c}$ and $R^{9d}$ are combined together with the adjacent nitrogen atom thereto to form a substituted or unsubstituted heterocyclic group).

(12) The pharmaceutical composition according to the above (9), wherein at least one of $R^{8a}$, $R^{8b}$ and $R^{8c}$ is $OR^{10}$ (wherein $R^{10}$ has the same meaning as defined above).

(13) The pharmaceutical composition according to the above (9), wherein at least one of $R^{8a}$, $R^{8b}$ and $R^{8c}$ is $OR^{10a}$ (wherein $R^{10a}$ represents substituted or unsubstituted lower alkyl).

(14) The pharmaceutical composition according to any of the above (1) to (3), wherein the Flt-3 inhibitor is an indazole derivative represented by Formula (Ib-1)

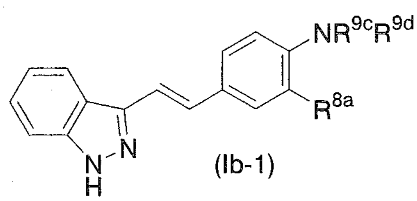

(Ib-1)

(wherein $R^{8a}$, $R^{9c}$ and $R^{9d}$ have the same meanings as defind above, respectively) or a pharmaceutically acceptable salt thereof.

(15) The pharmaceutical composition according to any of the above (1) to (3), wherein the Flt-3 inhibitor is an indazole derivative represented by Formula (Ib-2)

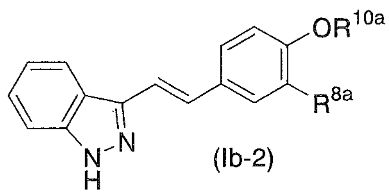

(Ib-2)

(wherein $R^{8a}$ and $R^{10a}$ have the same meanings as defind above, respectively) or a pharmaceutically acceptable salt thereof.
(16) The pharmaceutical composition according to any of the above (1) to (3), wherein the Flt-3 inhibitor is an indazole derivative represented by Formula (Ic)

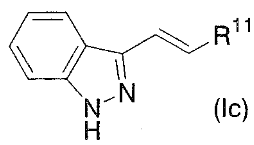

(Ic)

{wherein $R^{11}$ represents a substituted or unsubstituted heterocyclic group [substituent(s) in the substituted heterocyclic group may be the same or different, are 1 to 3 in number, and are oxo, formyl, carboxy, lower alkoxycarbonyl, substituted or unsubstituted lower alkyl, substituted or unsubstituted lower alkoxy, $CONR^{12a}R^{12b}$ (wherein $R^{12a}$ and $R^{12b}$ may be the same or different and each represents a hydrogen atom or substituted or unsubstituted lower alkyl), $NR^{13a}R^{13b}$ (wherein $R^{13a}$ and $R^{13b}$ may be the same or different and each represents a hydrogen atom, lower alkanoyl, lower alkoxycarbonyl, aralkyl, substituted or unsubstituted lower alkyl, substituted or unsubstituted aryl, substituted or unsubstituted aroyl or a substituted or unsubstituted heterocyclic group) or - $O(CR^{14a}R^{14b})_nO$- (wherein $R^{14a}$ and $R^{14b}$ may be the same or different and each represents a hydrogen atom or lower alkyl, n represents 2 or 3, and two terminal oxygen atoms are bonded to the same carbon atom in the heterocyclic group of the substituted heterocyclic group)]} or a pharmaceutically acceptable salt thereof.
(17) The pharmaceutical composition according to the above (16), wherein substituents in the substituted heterocyclic group are amino, oxo, lower alkoxy, lower alkoxycarbonylamino, aroylamino or lower alkoxycarbonyl-substituted lower alkyl.
(18) The pharmaceutical composition according to the above (16), wherein $R^{11}$ is 3-pyridyl.
(19) The pharmaceutical composition according to any of the above (1) to (3), wherein the Flt-3 inhibitor is a pyrimidine derivative represented by Formula (II)

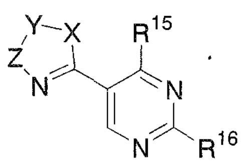

(II)

[wherein -X-Y-Z- represents -O-$CR^{17}$=N- {wherein $R^{17}$ represents a hydrogen atom, hydroxy, carboxy, lower alkyl, lower alkyl substituted with one to four substituents, which may be the same or different and selected from the following substituent group A [substituent group A: halogen, amino, aminosulfonyl, nitro, hydroxy, mercapto, cyano, formyl, carboxy, carbamoyl, lower alkanoyloxy, lower alkanoylamino, mono- or di-(lower alkyl)aminocarbonyl, lower alkoxycarbonyl, mono- or di-(lower alkyl)amino, N-aryl-N-(lower alkyl)amino, lower alkylsulfonyl, lower alkylsulfinyl, mono- or di-(lower alkylsulfonyl)amino, mono-or di-(arylsulfonyl)amino, tri-(lower alkyl)silyl, lower alkylthio, aromatic heterocycle-alkylthio, lower alkanoyl, lower alkanoyl substituted with one to three substituents, which may be the same or different and selected from the following substituent group a (substituent group a: halogen and hydroxy), lower alkoxy, lower alkoxy substituted with one to three substituents, which may be the same or different and selected from the substituent group a, aryloxy, aryloxy substituted with one to three substituents, which may be the same or different and selected from the substituent group a, aralkyloxy, and aralkyloxy substituted with one to three substituents, which may be the same or different and selected from the substituent group a; wherein, when the substituted

lower alkyl is substituted methyl, substituted ethyl, or substituted propyl, the substituent may be $-NR^{18a}R^{18b}$ (wherein $R^{18a}$ and $R^{18b}$ may be the same or different, and each represents a hydrogen atom, substituted or unsubstituted lower alkyl, substituted or unsubstituted cycloalkyl, substituted or unsubstituted lower alkenyl, substituted or unsubstituted lower alkynyl, substituted or unsubstituted aralkyl, substituted or unsubstituted aromatic heterocycle-alkyl, substituted or unsubstituted aliphatic heterocycle-alkyl, substituted or unsubstituted aryl, a substituted or unsubstituted aromatic heterocyclic group, or a substituted or unsubstituted aliphatic heterocyclic group)], lower cycloalkyl, lower cycloalkyl substituted with one to four substituents, which may be the same or different and selected from the above substituent group A, substituted or unsubstituted lower alkenyl, substituted or unsubstituted lower alkynyl, substituted or unsubstituted aralkyl, substituted or unsubstituted aromatic heterocycle-alkyl, substituted or unsubstituted aliphatic heterocycle-alkyl, substituted or unsubstituted aryl, a substituted or unsubstituted aromatic heterocyclic group, a substituted or unsubstituted aliphatic heterocyclic group, substituted or unsubstituted lower alkoxy, substituted or unsubstituted lower alkoxycarbonyl, substituted or unsubstituted lower alkylthio, substituted or unsubstituted lower alkanoyl, or $-C(=O)NR^{19a}R^{19b}$ (wherein $R^{19a}$ and $R^{19b}$ may be the same or different, and each represents a hydrogen atom, substituted or unsubstituted lower alkyl, substituted or unsubstituted lower cycloalkyl, substituted or unsubstituted lower alkenyl, substituted or unsubstituted lower alkynyl, substituted or unsubstituted aralkyl, substituted or unsubstituted aromatic heterocycle-alkyl, substituted or unsubstituted aliphatic heterocycle-alkyl, substituted or unsubstituted aryl, a substituted or unsubstituted aromatic heterocyclic group, or a substituted or unsubstituted aliphatic heterocyclic group, or $R^{19a}$ and $R^{19b}$ are combined together with the adjacent nitrogen atom thereto to form a substituted or unsubstituted aliphatic heterocyclic group)}, $-N=CR^{17a}-O-$ (wherein $R^{17a}$ has the same meaning as $R^{17}$ defined above), $-O-N=CR^{17b}-$(wherein $R^{17b}$ has the same meaning as $R^{17}$ defined above), $-O-C(=O)-NR^{20}-$ (wherein $R^{20}$ represents a hydrogen atom, lower alkyl, lower alkyl substituted with one to four substituents, which may be the same or different and selected from the above substituent group A, lower cycloalkyl, lower cycloalkyl substituted with one to four substituents, which may be the same or different and selected from the substituent group A, or substituted or unsubstituted aliphatic heterocycle-alkyl), $-N=N-NR^{21}-$ (wherein $R^{21}$ represents substituted or unsubstituted lower alkyl, substituted or unsubstituted lower cycloalkyl or substituted or unsubstituted aliphatic heterocycle-alkyl), or $-NR^{21a}-N=N-$ (wherein $R^{21a}$ has the same definition as $R^{21}$ described above);

$R^{15}$ represents $-NR^{22a}R^{22b}$ (wherein $R^{22a}$ and $R^{22b}$ may be the same or different, and each represents a hydrogen atom, substituted or unsubstituted lower alkyl, substituted or unsubstituted lower cycloalkyl, substituted or unsubstituted lower alkenyl, substituted or unsubstituted lower alkynyl, substituted or unsubstituted aralkyl, substituted or unsubstituted aromatic heterocycle-alkyl, substituted or unsubstituted aliphatic heterocycle-alkyl, substituted or unsubstituted monocyclic aryl, a substituted or unsubstituted aromatic monoheterocyclic group, or a substituted or unsubstituted aliphatic heterocyclic group, or $R^{22a}$ and $R^{22b}$ are combined together with the adjacent nitrogen atom thereto to form a substituted or unsubstituted aliphatic heterocyclic group; wherein, when one of $R^{22a}$ and $R^{22b}$ is a hydrogen atom, the other of $R^{22a}$ and $R^{22b}$ is not a group selected from substituted or unsubstituted pyrazol-3-yl and substituted or unsubstituted 1,2,4-triazol-3-yl), or $-OR^{23}$ (wherein $R^{23}$ represents a hydrogen atom, substituted or unsubstituted lower alkyl, substituted or unsubstituted lower cycloalkyl, substituted or unsubstituted lower alkenyl, substituted or unsubstituted lower alkynyl, substituted or unsubstituted aralkyl, substituted or unsubstituted aromatic heterocycle-alkyl, substituted or unsubstituted aliphatic heterocycle-alkyl, substituted or unsubstituted aryl, a substituted or unsubstituted aromatic heterocyclic group, or a substituted or unsubstituted aliphatic heterocyclic group);

and $R^{16}$ represents $-NR^{24a}R^{24b}$ {wherein $R^{24a}$ and $R^{24b}$ may be the same or different, and each represents a hydrogen atom, lower alkyl, lower alkyl substituted with one to four substituents, which may be the same or different and selected from the following substituent group B [substituent group B: halogen, amino, aminosulfonyl, nitro, hydroxy, mercapto, cyano, formyl, carboxy, carbamoyl, lower alkanoyloxy, lower alkanoylamino, mono- or di-(lower alkyl)aminocarbonyl, lower alkoxycarbonyl, mono- or di-(lower alkyl)amino, N-aryl-N-(lower alkyl)amino, lower alkylsulfonyl, lower alkylsulfinyl, mono- or di-(lower alkylsulfonyl)amino, mono-or di-(arylsulfonyl)amino, tri-(lower alkyl) silyl, lower alkylthio, aromatic heterocycle-alkylthio, lower alkanoyl, lower alkanoyl substituted with one to three substituents, which may be the same or different and selected from the above substituent group a, lower alkoxy, lower alkoxy substituted with one to three substituents, which may be the same or different and selected from the above substituent group a, aryloxy, aryloxy substituted with one to three substituents, which may be the same or different and selected from the above substituent group a, aralkyloxy, and aralkyloxy substituted with one to three substituents, which may be the same or different and selected from the above substituent group a], lower cycloalkyl, lower cycloalkyl substituted with one to four substituents, which may be the same or different and selected from the above substituent group B, substituted or unsubstituted lower alkenyl, substituted or unsubstituted lower alkynyl, substituted or unsubstituted aliphatic heterocycle-alkyl, substituted or unsubstituted monocyclic aryl, or a substituted or unsubstituted aliphatic heterocyclic group, or $R^{24a}$ and $R^{24b}$ are combined together with the adjacent nitrogen atom thereto to form a substituted or unsubstituted aliphatic heterocyclic group or a substituted or unsubstituted aromatic heterocyclic group; wherein $R^{24a}$ and $R^{24b}$ do not simultaneously represent a hydrogen atom}, $-NR^{25}CR^{26a}R^{26b}-Ar$ {wherein $R^{25}$ represents a hydrogen atom, lower alkyl or lower cycloalkyl, $R^{26a}$ and $R^{26b}$ may

be the same or different, and each represents a hydrogen atom, lower alkyl, lower alkyl substituted with one to three substituents, which may be the same or different and selected from the following substituent group b (substituent group b: halogen, hydroxy and hydroxymethyl), lower cycloalkyl, or lower cycloalkyl substituted with one to three substituents, which may be the same or different and selected from the above substituent group b, and Ar represents aryl, aryl substituted with one to three substituents, which may be the same or different and selected from the following substituent group C [substituent group C: halogen, amino, nitro, hydroxy, mercapto, cyano, carboxy, aminosulfonyl, lower alkyl, lower alkyl substituted with one to three substituents, which may be the same or different and selected from the above substituent group b, lower cycloalkyl, lower cycloalkyl substituted with one to three substituents, which may be the same or different and selected from the above substituent group b, lower alkoxy, lower slkylthio, mono- or di-(lower alkyl)amino, lower alkanoylamino, mono- or di-(lower alkylsulfonyl)amino, lower alkoxycarbonylamino, aliphatic heterocycle-alkyloxy and alkylenedioxy], an aromatic heterocyclic group or an aromatic heterocyclic group substituted with one to three substituents, which may be the same or different and selected from the above substituent group C} or - $NR^{25}CR^{26a}R^{26b}CR^{27a}R^{27b}$-Ar (wherein $R^{25}$, $R^{26a}$, $R^{26b}$ and Ar have the same meanings as defined above, respectively, and $R^{27a}$ and $R^{27b}$ have the same meanings as $R^{26a}$ and $R^{26b}$ defined above, respectively)] or a pharmaceutically acceptable salt thereof.

(20) The pharmaceutical composition according to any of the above (1) to (3), wherein the Flt-3 inhibitor is a nitrogen-containing heterocyclic compound represented by Formula (III)

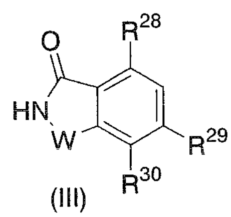

(III)

[wherein W represents -C(=O)- or -$CHR^{31}$- (wherein $R^{31}$ represents a hydrogen atom, hydroxy, substituted or unsubstituted lower alkyl, or substituted or unsubstituted lower alkoxy), $R^{28}$ represents

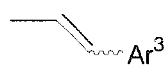

{wherein $Ar^3$ represents aryl, aryl substituted with one or two substituents, which may be the same or different and selected from the following substituent group D, an aromatic monoheterocyclic group, an aromatic monoheterocyclic group substituted with one or two substituents, which may be the same or different and selected from the following substituent group D [substituent group D: halogen, nitro, hydroxy, cyano, carboxy, lower alkoxycarbonyl, substituted or unsubstituted lower alkyl, substituted or unsubstituted lower alkoxy, substituted or unsubstituted lower alkanoyl, -$CONR^{32a}R^{32b}$ (wherein $R^{32a}$ and $R^{32b}$ may be the same or different, and each represents a hydrogen atom, substituted or unsubstituted lower alkyl, substituted or unsubstituted aryl, or substituted or unsubstituted aralkyl, or $R^{32a}$ and $R^{32b}$ are combined together with the adjacent nitrogen atom thereto to form a substituted or unsubstituted heterocyclic group) and -$NR^{33a}R^{33b}$ (wherein $R^{33a}$ and $R^{33b}$ may be the same or different, and each represents a hydrogen atom, substituted or unsubstituted lower alkyl, substituted or unsubstituted lower alkanoyl, substituted or unsubstituted lower alkylsulfonyl, substituted or unsubstituted aryl, substituted or unsubstituted aralkyl, substituted or unsubstituted aroyl, substituted or unsubstituted arylsulfonyl, or substituted or unsubstituted heteroaroyl)]}, $R^{29}$ represents a hydrogen atom or

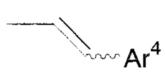

(wherein $Ar^4$ has the same meaning as $Ar^3$ defined above), and $R^{30}$ represents a hydrogen atom, halogen, substituted or unsubstituted lower alkyl, substituted or unsubstituted lower alkanoyl, substituted or unsubstituted aryl, -$NR^{34a}R^{34b}$ [(wherein $R^{34a}$ and $R^{34b}$ may be the same or different, and each represents a hydrogen atom, substituted or unsubstituted lower alkyl, or - $C(=O)R^{35}$ (wherein $R^{35}$ represents a hydrogen atom, substituted or unsubstituted lower alkyl, substituted or unsubstituted lower alkoxy, or substituted or unsubstituted aralkyl)] or

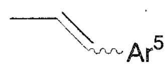

(wherein $Ar^5$ has the same meaning as $Ar^3$ defined above); provided that, when $R^{29}$ is a hydrogen atom and $Ar^3$ is aryl, aryl substituted with two lower alkoxy, or aryl substituted with one lower alkyl or one lower alkoxy only, $R^{30}$ is not a hydrogen atom] or a phrmaceutically acceptable salt thereof.

(21) The pharmaceutical composition according to any of the above (1) to (20), wherein the target disease is cancer.

(22) The pharmaceutical composition according to the above (21), wherein the cancer is cancer derived from hematopoietic tumor, breast cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, head and neck cancer, osteosarcoma, melanoma, or cancer derived from brain tumor.

(23) The pharmaceutical composition according to the above (21), wherein the cancer is leukemia, myeloma or lymphoma.

(24) The pharmaceutical composition according to the above (21), wherein the cancer is acute myeloid leukemia.

(25) The pharmaceutical composition according to the above (21), wherein the cancer is solid cancer.

(26) The pharmaceutical composition according to the above (21), wherein the cancer is pancreatic cancer.

(27) The pharmaceutical composition according to the above (21), wherein the cancer is renal cancer.

(28) The pharmaceutical composition according to the above (21), wherein the cancer is colon cancer.

(29) The pharmaceutical composition according to any of the above (3) to (28), wherein the compound to be administered in combination, simultaneously or successively, with the Flt-3 inhibitor is a protein or a low molecular compound.

(30) The pharmaceutical composition according to the above (29), wherein the compound to be combined with the Flt-3 inhibitor is a protein and the protein is an antibody.

(31) The pharmaceutical composition according to the above (30), wherein the antibody is Cetuximab.

(32) The pharmaceutical composition according to the above (29), wherein the compound to be combined with the Flt-3 inhibitor is a low molecular compound and the low molecular compound is a chemotherapeutic agent or a molecular targeted drug.

(33) The pharmaceutical composition according to the above (32), wherein the low molecular compound is a chemotherapeutic agent and the chemotherapeutic agent is selected from Cytarabine, Daunorubicin, Idarubicin, Vincristine, Etoposide, Vindesine, Doxorubicin, Mitoxantrone, Fluorouracil, Irinotecan and Gemcitabine.

(34) The pharmaceutical composition according to the above (32), wherein the low molecular compound is a molecular targeted drug and the molecular targeted drug is a farnesyltransferase inhibitor.

(35) The pharmaceutical composition according to the above (34), wherein the farnesyltransferase inhibitor is Zanestra.

(36) The pharmaceutical composition according to the above (32), wherein the low molecular compound is a molecular targeted drug and the molecular targeted drug is a proteasome inhibitor.

(37) The pharmaceutical composition according to the above (36), wherein the proteasome inhibitor is Bortezomib.

(38) The pharmaceutical composition according to the above (32), wherein the low molecular compound is a molecular targeted drug and the molecular targeted drug is a kinase inhibitor.

(39) The pharmaceutical composition according to the above (38), wherein the kinase inhibitor is Erlotinib.

(40) The pharmaceutical composition according to the above (32), wherein the low molecular compound is a molecular targeted drug and the molecular targeted drug is a heat shock protein 90 (Hsp90) inhibitor.

(41) A method of treating cancer, which comprises the step of administering an Flt-3 inhibitor and at least one compound simultaneously or separately with an interval.

(42) The method of treating cancer according to the above (41), wherein the Flt-3 inhibitor is an indazole derivative represented by Formula (I)

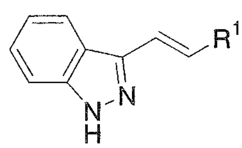 (I)

(wherein $R^1$ has the same meaning as defined above) or a pharmaceutically acceptable salt thereof.

(43) The method of treating cancer according to the above (41), wherein the Flt-3 inhibitor is an indazole derivative represented by Formula (Ia)

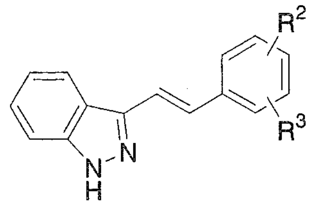

(Ia)

(wherein $R^2$ and $R^3$ have the same meanings as defined above, respectively) or a pharmaceutically acceptable salt thereof.

(44) The method of treating cancer according to the above (41), wherein the Flt-3 inhibitor is an indazole derivative represented by Formula (Ib)

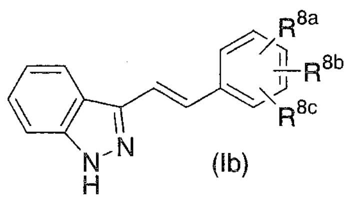

(Ib)

(wherein $R^{8a}$, $R^{8b}$ and $R^{8c}$ have the same meanings as defined above, respectively) or a pharmaceutically acceptable salt thereof.

(45) The method of treating cancer according to the above (41), wherein the Flt-3 inhibitor is an indazole derivative represented by Formula (Ib-1)

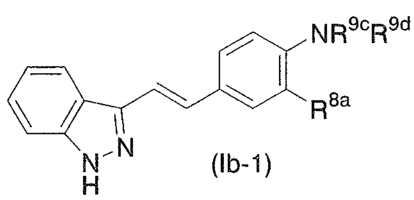

(Ib-1)

(wherein $R^{8a}$, $R^{9c}$ and $R^{9d}$ have the same meanings as defined above, respectively) or a pharmaceutically acceptable salt thereof.

(46) The method of treating cancer according to the above (41), wherein the Flt-3 inhibitor is an indazole derivative represented by Formula (Ib-2)

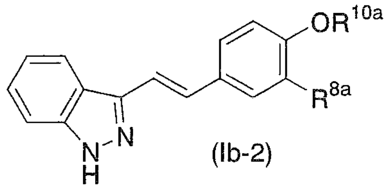

(Ib-2)

(wherein $R^{8a}$ and $R^{10a}$ have the same meanings as defined above, respectively) or a pharmaceutically acceptable salt thereof.

(47) The method of treating cancer according to the above (41), wherein the Flt-3 inhibitor is an indazole derivative represented by Formula (Ic)

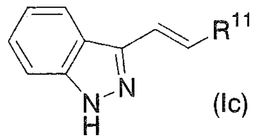

(Ic)

(wherein $R^{11}$ has the same meanings as defined above) or a pharmaceutically acceptable salt thereof.

(48) The method of treating cancer according to the above (41), wherein the Flt-3 inhibitor is a pyrimidine derivative represented by Formula (II)

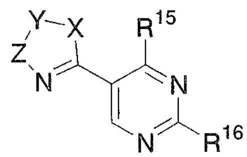

(II)

(wherein -X-Y-Z-, $R^{15}$ and $R^{16}$ have the same meanings as defined above, respectively) or a pharmaceutically acceptable salt thereof.

(49) The method of treating cancer according to the above (41), wherein the Flt-3 inhibitor is a nitrogen-containing heterocyclic compound represented by Formula (III)

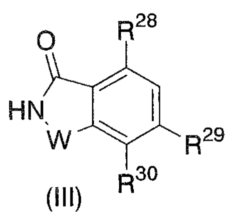

(III)

(wherein W, $R^{28}$, $R^{29}$ and $R^{30}$ have the same meanings as defined above, respectively) or a pharmaceutically acceptable salt thereof.

(50) Use of an Flt-3 inhibitor and at least one compound for the manufacture of an anticancer agent.

(51) The use according to the above (50), wherein the Flt-3 inhibitor is an indazole derivative represented by formula (I)

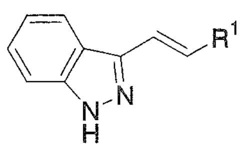

(I)

(wherein $R^1$ has the same meaning as defined above) or a pharmaceutically acceptable salt thereof.

(52) The use according to the above (50), wherein the Flt-3 inhibitor is an indazole derivative represented by Formula (Ia)

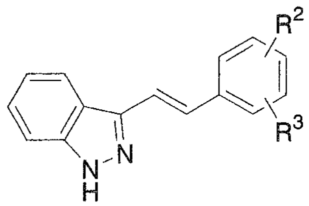

(Ia)

(wherein $R^2$ and $R^3$ have the same meanings as defined above, respectively) or a pharmaceutically acceptable salt thereof.

(53) The use according to the above (50), wherein the Flt-3 inhibitor is an indazole derivative represented by Formula (Ib)

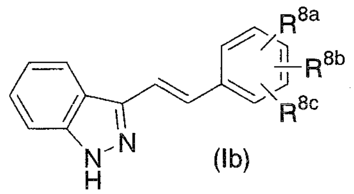

(Ib)

(wherein $R^{8a}$, $R^{8b}$ and $R^{8c}$ have the same meanings as defined above, respectively) or a pharmaceutically acceptable salt thereof.

(54) The use according to the above (50), wherein the Flt-3 inhibitor is an indazole derivative represented by Formula (Ib-1)

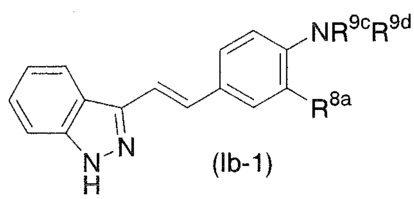

(Ib-1)

(wherein $R^{8a}$, $R^{9c}$ and $R^{9d}$ have the same meanings as defined above, respectively) or a pharmaceutically acceptable salt thereof.

(55) The use according to the above (50), wherein the Flt-3 inhibitor is an indazole derivative represented by Formula (Ib-2)

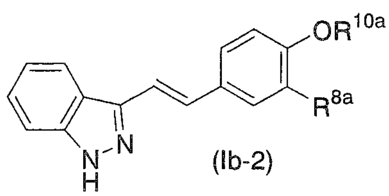

(Ib-2)

(wherein $R^{8a}$ and $R^{10a}$ have the same meanings as defined above, respectively) or a pharmaceutically acceptable salt thereof.

(56) The use according to the above (50), wherein the Flt-3 inhibitor is an indazole derivative represented by Formula (Ic)

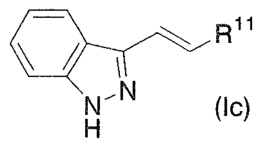

(Ic)

(wherein $R^{11}$ has the same meanings as defined above) or a pharmaceutically acceptable salt thereof.

(57) The use according to the above (50), wherein the Flt-3 inhibitor is a pyrimidine derivative represented by Formula (II)

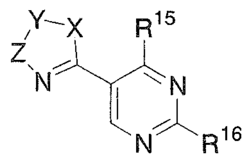

(II)

(wherein -X-Y-Z-, $R^{15}$ and $R^{16}$ have the same meanings as defined above, respectively) or a pharmaceutically acceptable salt thereof.

(58) The use according to the above (50), wherein the Flt-3 inhibitor is a nitrogen-containing heterocyclic compound represented by Formula (III)

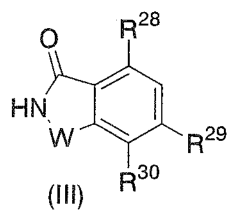

(III)

(wherein W, $R^{28}$, $R^{29}$ and $R^{30}$ have the same meanings as defined above, respectively) or a pharmaceutically acceptable salt thereof.

(59) A kit which comprises a first component comprising an Flt-3 inhibitor and a second component comprising an antitumor agent.

(60) The kit according to the above (59), wherein the Flt-3 inhibitor is the indazole derivative described in any of the above (4) to (18) or a pharmaceutically acceptable salt thereof.

(61) The kit according to the above (59), wherein the Flt-3 inhibitor is the pyrimidine derivative described in the above (19) or a pharmaceutically acceptable salt thereof.

(62) The kit according to the above (59), wherein the Flt-3 inhibitor is the nitrogen-containing heterocyclic compound described in the above (20) or a pharmaceutically acceptable salt thereof.

(63) An antitumor agent for administering an Flt-3 inhibitor and at least one compound as active ingredients simultaneously or successively.

(64) The antitumor agent according to the above (63), wherein the Flt-3 inhibitor is the indazole derivative described in any of the above (4) to (18), or a pharmaceutically acceptable salt thereof.

(65) The antitumor agent according to the above (63), wherein the Flt-3 inhibitor is the pyrimidine derivative described in the above (19) or a pharmaceutically acceptable salt thereof.

(66) The antitumor agent according to the above (63), wherein the Flt-3 inhibitor is the nitrogen-containing heterocyclic compound described in the above (20) or a pharmaceutically acceptable salt thereof.

Effect of the Invention

The present invention provides a pharmaceutical composition comprising a combination of an Flt-3 inhibitor and at least one compound, and the like.

Description of Drawings

[Fig. 1] Fig. 1 is a graph showing growth inhibitory effects obtained by a combined use of Compound 9 and cytarabine on mouse models transplanted with human acute myeloid leukemia MOLM-13 cells. The vertical axis represents the ratio (V/V0) of change in tumor volume, assuming that the tumor volume on day zero is V0. The horizontal axis represents the number of days. Solid diamonds represent the growth inhibitory effect of non-administration of Compound 9 and cytarabine, solid circles represent the growth inhibitory effect of administration of Compound 9, solid triangles represent the growth inhibitory effect of administration of cytarabine, and crosses represent the growth inhibitory effect of combined administration of Compound 9 and cytarabine.

[Fig. 2] Fig. 2 is a graph showing growth inhibitory effects obtained by a combined use of Compound 9 and cetuximab on mouse models transplanted with human pancreatic cancer MIA-PaCa-2 cells.

The vertical axis represents the ratio (V/V0) of change in tumor volume, assuming that the tumor volume on day zero is V0. The horizontal axis represents the number of days. Solid diamonds represent the growth inhibitory effect of non-administration of Compound 9 and cetuximab, solid circles represent the growth inhibitory effect of administration of Compound 9, solid triangles represent the growth inhibitory effect of administration of cetuximab, and crosses represent the growth inhibitory effect of combined administration of Compound 9 and cetuximab.

[Fig. 3] Fig. 3 is a graph showing growth inhibitory effects obtained by a combined use of Compound 9 and gemcitabine on mouse models transplanted with human pancreatic cancer MIA-PaCa-2 cells.

The vertical axis represents the ratio (V/V0) of change in tumor volume, assuming that the tumor volume on day zero is V0. The horizontal axis represents the number of days. Solid diamonds represent the growth inhibitory effect of non-administration of Compound 9 and gemcitabine, solid circles represent the growth inhibitory effect of administration of Compound 9, solid triangles represent the growth inhibitory effect of administration of gemcitabine, and crosses represent the growth inhibitory effect of combined administration of Compound 9 and gemcitabine.

[Fig. 4] Fig. 4 is a graph showing growth inhibitory effects obtained by a combined use of Compound 9 and 5-FU on mouse models transplanted with human colon cancer Colo205 cells.

The vertical axis represents the ratio (V/V0) of change in tumor volume, assuming that the tumor volume on day zero is V0. The horizontal axis represents the number of days. Solid diamonds represent the growth inhibitory effect of non-administration of Compound 9 and 5-FU, solid circles represent the growth inhibitory effect of administration of Compound 9, solid triangles represent the growth inhibitory effect of administration of 5-FU, and crosses represent the growth inhibitory effect of combined administration of Compound 9 and 5-FU.

[Fig. 5] Fig. 5 is a graph showing growth inhibitory effects obtained by a combined use of Compound 9 and irinotecan on mouse models transplanted with human colon cancer Colo205 cells.

The vertical axis represents the ratio (V/V0) of change in tumor volume, assuming that the tumor volume on day zero is V0.

The horizontal axis represents the number of days. Solid diamonds represent the growth inhibitory effect of non-administration of Compound 9 and irinotecan, solid circles represent the growth inhibitory effect of administration of Compound 9, solid triangles represent the growth inhibitory effect of administration of irinotecan, and crosses represent the growth inhibitory effect of combined administration of Compound 9 and irinotecan.

[Fig. 6] Fig. 6 is a graph showing growth inhibitory effects obtained by a combined use of Compound 9 and erlotinib on mouse models transplanted with human renal cancer Caki-1 cells.

The vertical axis represents the ratio (V/V0) of change in tumor volume, assuming that the tumor volume on day zero is V0. The horizontal axis represents the number of days. Solid diamonds represent the growth inhibitory effect of non-administration of Compound 9 and erlotinib, solid circles represent the growth inhibitory effect of administration of Compound 9, solid triangles represent the growth inhibitory effect of administration of erlotinib, and crosses represent the growth inhibitory effect of combined administration of Compound 9 and erlotinib.

[Fig. 7] Fig. 7 is a graph showing growth inhibitory effects obtained by a combined use of Compound 9 and sunitinib on mouse models transplanted with human renal cancer Caki-1 cells.

The vertical axis represents the ratio (V/V0) of change in tumor volume, assuming that the tumor volume on day zero is V0. The horizontal axis represents the number of days. Solid diamonds represent the growth inhibitory effects of non-administration of Compound 9 and sunitinib, solid circles represent the growth inhibitory effects of administration of Compound 9, solid triangles represent the growth inhibitory effects of administration of sunitinib, and crosses represent the growth inhibitory effects of combined administration of Compound 9 and sunitinib.

[Fig. 8] Fig. 8 is a graph showing growth inhibitory effects obtained by a combined use of Compound P and Compound 9 on mouse models transplanted with human acute myeloid leukemia MOLM-13 cells.

The vertical axis represents the ratio (V/V0) of change in tumor volume, assuming that the tumor volume on day zero is V0. The horizontal axis represents the number of days. Solid diamonds represent the growth inhibitory effects

of non-administration of Compound P and Compound 9, solid circles represent the growth inhibitory effects of administration of Compound P, solid triangles represent the growth inhibitory effects of administration of Compound 9, and crosses represent the growth inhibitory effects of combined administration of Compound P and Compound 9.

[Fig. 9] Fig. 9 is a graph showing growth inhibitory effects obtained by a combined use of Compound 9 and daunorubicin on mouse models transplanted with human acute myeloid leukemia HL-60 cells.

The vertical axis represents the ratio (V/V0) of change in tumor volume, assuming that the tumor volume on day zero is V0. The horizontal axis represents the number of days. Solid diamonds represent the growth inhibitory effects of non-administration of Compound 9 and daunorubicin, solid circles represent the growth inhibitory effects of administration of Compound 9, solid triangles represent the growth inhibitory effects of administration of daunorubicin, and crosses represent the growth inhibitory effects of combined administration of Compound 9 and daunorubicin.

[Fig. 10] Fig. 10 is a graph showing the apoptosis-inducing activity of Compound 9, suberanilohydroxamic acid (SAHA), and combined use of Compound 9 and SAHA against MOLM-13 cells. The vertical axis represents the VEVDase activity (%), and P indicates the statistical significance level.

[Fig. 11] Fig. 11 is a graph showing the apoptosis-inducing activity of Compound 9, valproic acid (VPA), and combined use of Compound 9 and VPA against MOLM-13 cells. The vertical axis represents the VEVDase activity (%), and P indicates the statistical significance level.

[Fig. 12] Fig. 12 is a graph showing the apoptosis-inducing activity of Compound 9, 17-allylamino-17-demethoxygeldanamycin (17-AAG), and combined use of Compound 9 and 17-AAG against MOLM-13 cells. The vertical axis represents the VEVDase activity (%), and P indicates the statistical significance level.

[Fig. 13] Fig. 13 is a graph showing the apoptosis-inducing activity of Compound 9, 17-dimethylaminoethylamino-17-demethoxygeldanamycin (17-DMAG), and combined use of Compound 9 and 17-DMAG against MOLM-13 cells. The vertical axis represents the VEVDase activity (%), and P indicates the statistical significance level.

[Fig. 14] Fig. 14 is a graph showing the apoptosis-inducing activity of Compound 9, decitabine, and combined use of Compound 9 and decitabine against MOLM-13 cells. The vertical axis represents the VEVDase activity (%), and P indicates the statistical significance level.

[Fig. 15] Fig. 15 is a graph showing the apoptosis-inducing activity of Compound 9, 5-azacitidine, and combined use of Compound 9 and 5-azacitidine against MOLM-13 cells. The vertical axis represents the VEVDase activity (%), and P indicates the statistical significance level.

[Fig. 16] Fig. 16 is a graph showing the apoptosis-inducing activity of Compound 9, fludarabine, and combined use of Compound 9 and fludarabine against MOLM-13 cells. The vertical axis represents the VEVDase activity (%), and P indicates the statistical significance level.

## Best Mode for Carrying Out the Invention

Hereinafter, the compounds represented by general formulae (I), (Ia), (Ib), (Ib-1), (Ib-2), (Ic), (II) and (III) are referred to as Compounds (I), (Ia), (Ib), (Ib-1), (Ib-2), (Ic), (II) and (III) and the same applies to compounds of other formula numbers.

In the definitions of the groups in general formula (I), (Ia), (Ib), (Ib-1), (Ib-2), (Ic), (II) and (III):

(i) The halogen includes fluorine, chlorine, bromine, and iodine atoms.

(ii) Examples of the lower alkyl and the lower alkyl moieties of the lower alkoxy, the lower alkoxycarbonyl, the lower alkoxycarbonylamino, the lower alkoxycarbonyl-substituted lower alkyl and the lower alkylsulfonyl include, for example, linear, branched or cyclic alkyl or alkyl consisting of these alkyls in combination, having 1 to 10 carbon atom(s). More specific examples thereof are as follows.

(ii-a) Examples of the linear or branched lower alkyl include, for example, methyl, ethyl, *n*-propyl, isopropyl, *n*-butyl, isobutyl, *sec*-butyl, *tert*-butyl, *n*-pentyl, neopentyl, *n*-hexyl, *n*-heptyl, *n*-octyl, *n*-nonyl, *n*-decyl and the like;

(ii-b) examples of the cyclic lower alkyl include, for example, cyclopropyl, cyclobutyl, cyclopentyl, cyclohexyl, cycloheptyl, cyclooctyl, cyclodecyl, noradamantyl, adamantyl, bicyclo[2.2.1]heptyl, bicyclo[2.2.2]octyl, bicyclo[3.3.0]octyl, bicyclo[3.3.1]nonyl and the like; and

(ii-c) examples of the lower alkyl consisting of linear or branched alkyl and cyclic alkyl in combination include, for example, cyclopropylmethyl, cyclopentylmethyl, cyclooctylethyl and the like.

(iii) The alkylene moiety of the lower alkoxycarbonyl-substituted lower alkyl and the aralkyl has the same meaning as the group formed by removing one hydrogen atom from the linear or branched lower alkyl (ii-a) defined above.

(iv) Examples of the lower alkenyl include, for example, linear or branched alkenyl having 2 to 10 carbon atoms such as vinyl, allyl, 1-propenyl, 1-butenyl, 3-butenyl, 2-pentenyl, 4-pentenyl, 2-hexenyl, 5-hexenyl, 2-decenyl or 9-decenyl.

(v) Examples of the lower alkynyl include, for example, linear or branched alkynyl having 2 to 10 carbon atoms such as ethynyl, 2-propynyl, 3-butynyl, 4-pentynyl, 5-hexynyl or 9-decynyl.

(vi) Examples of the aryl and the aryl moieties of the aralkyl, the aroyl, the aroylamino and the arylsulfonyl include, for example, monocyclic aryl or fused aryl in which two or more rings are fused, and more specific examples include aryl having 6 to 14 carbon atoms as ring-constituting members, such as phenyl, naphthyl, indenyl or anthranyl.

(vii) Examples of the lower alkanoyl include, for example, linear, branched, or cyclic lower alkanyol, or lower alkanoyl consisting of these lower alkanoyls in combination, having 1 to 8 carbon atom(s), such as formyl, acetyl, propionyl, butyryl, isobutyryl, valeryl, isovaleryl, pivaloyl, hexanoyl, heptanoyl, octanoyl, cyclopropylcarbonyl, cyclobutylcarbonyl, cyclopentylcarbonyl, cyclopropylmethylcarbonyl, cyclohexylcarbonyl, 1-methylcyclopropylcarbonyl or cycloheptylcarbonyl.

(viii) Examples of the heterocyclic group include, for example, aromatic heterocyclic group, aliphatic heterocyclic group and the like.

(viii-a) Examples of the aromatic heterocyclic group include, for example, monocyclic aromatic heterocyclic group, fused aromatic heterocyclic group in which two or more rings are fused or the like. The type and number of the heteroatom contained in aromatic heterocyclic group are not specifically limited and the aromatic heterocyclic group may contain, for example, one or more heteroatoms selected from the group consisting of a nitrogen atom, a sulfur atom and an oxygen atom. More specific examples include aromatic heterocyclic group having 5 to 14 atoms as ring-constituting members, such as furyl, thienyl, pyrrolyl, imidazolyl, pyrazolyl, triazolyl, tetrazolyl, oxazolyl, oxadiazolyl, thiazolyl, pyridyl, pyrazinyl, pyrimidinyl, pyridazinyl, triazinyl, indolyl, indazolyl, benzimidazolyl, benzoxazolyl, benzothiazolyl, quinolyl, isoquinolyl, phthalazinyl, naphthyridinyl, quinoxalinyl, quinazolinyl, cinnolinyl, purinyl or coumarinyl.

(viii-b) Examples of the aliphatic heterocyclic group include, for example, monocyclic aliphatic heterocyclic group or fused aliphatic heterocyclic group in which two or more rings are fused. The type and number of the heteroatom contained in aliphatic heterocyclic groups are not specifically limited and the aliphatic heterocyclic group may contain, for example, one or more heteroatoms selected from the group consisting of a nitrogen atom, a sulfur atom and an oxygen atom. More specific examples include, for example, pyrrolidinyl, 2,5-dioxopyrrolidinyl, thiazolidinyl, oxazolidinyl, piperidyl, 1,2-dihydropyridyl, piperazinyl, homopiperazinyl, morpholinyl, thiomorpholinyl, pyrazolinyl, oxazolinyl, dioxolanyl, tetrahydropyranyl, tetrahydrothiopyranyl, tetrahydrofuryl, tetrahydroquinolyl, tetrahydroisoquinolyl, tetrahydroquinoxalinyl, octahydroquinolyl, dihydroindolyl, 1,3-dioxoisoindolinyl and the like.

(ix) Examples of the heterocyclic group formed together with the adjacent nitrogen atom include 5- or 6-membered monocyclic heterocyclic group containing at least one nitrogen atom (the monocyclic heterocyclic group may further contain any other of a nitrogen atom, an oxygen atom and a sulfur atom) and bicyclic or tricyclic fused heterocyclic group containing at least one nitrogen atom in which 3- to 8-membered rings are fused (the fused heterocyclic group may further contain any other of a nitrogen atom, an oxygen atom and a sulfur atom). More specific examples include, for example, pyrrolidinyl, piperidino, piperazinyl, morpholino, thiomorpholino, homopiperidino, homopiperazinyl, tetrahydropyridyl, tetrahydroquinolyl, tetrahydroisoquinolyl and the like.

(x) The heteroaryl moiety in the heteroaroyl has the same meaning as the aromatic heterocyclic group (viii-a) defined above.

(xi) Examples of the substituents in the substituted lower alkyl, the substituted lower alkoxy, the substituted lower alkenyl, the substituted lower alkynyl, the substituted lower alkanoyl, the substituted lower alkoxycarbonyl and the substituted lower alkylsulfonyl which may be the same or different and in number of 1 to 3, include

(xi-a) hydroxy,

(xi-b) lower alkoxy,

(xi-c) oxo,

(xi-d) carboxy,

(xi-e) lower alkoxycarbonyl,

(xi-f) heteroaroyl,

(xi-g) arylsulfonyl,

(xi-h) substituted or unsubstituted aryl (the substituent(s) in the substituted aryl is carboxy, lower alkoxy, lower alkoxycarbonyl or the like),

(xi-i) a substituted or unsubstituted heterocyclic group (the substituent(s) in the substituted heterocyclic group is carboxy, lower alkoxy, lower alkoxycarbonyl or the like),

(xi-j) $CONR^{36a}R^{36b}$ {wherein $R^{36a}$ and $R^{36b}$ may be the same or different and each represents a hydrogen atom or substituted or unsubstituted lower alkyl [the substituent(s) in the substituted lower alkyl, which is 1 to 3 in number, is for example, halogen, hydroxy, oxo, nitro, cyano, carboxy, lower alkanoyl, lower alkoxycarbonyl, aroyl or substituted or unsubstituted lower alkoxy (the substituent(s) in the substituted lower alkoxy, which is 1 to 3 in number, is for example, hydroxy or the like) or the like] or $R^{36a}$ and $R^{36b}$ are combined together with the adjacent nitrogen atom thereto to form a substituted or unsubstituted heterocyclic group [the substituent(s) in the substituted heterocyclic group formed together with the adjacent nitrogen atom, which is 1 to 3 in number, is for example, halogen, hydroxy, oxo, nitro, cyano, carboxy, lower alkanoyl, lower alkoxycarbonyl, aralkyl, aroyl, substituted or unsubstituted lower

alkyl (the substituent(s) in the substituted lower alkyl, which is 1 to 3 in number, is for example, hydroxy or the like), substituted or unsubstituted lower alkoxy (the substituent(s) in the substituted lower alkoxy, which is 1 to 3 in number, is for example, hydroxy or the like) or the like]),

(xi-k) $NR^{37a}R^{37b}$ (wherein $R^{37a}$ and $R^{37b}$ have the same meanings as $R^{36d}$ and $R^{36b}$ defined above, respectively),

(xi-l) lower alkanoylamino,

(xi-m) N-(lower alkanoyl)-N-(lower alkyl)amino, and the like.

In the definition of the substituents (xi) in the substituted lower alkyl, the substituted lower alkoxy, the substituted lower alkenyl, the substituted lower alkynyl, the substituted lower alkanoyl, the substituted lower alkoxycarbonyl and the substituted lower alkylsulfonyl, the halogen has the same meaning as (i) defined above; the lower alkyl and the lower alkyl moiety of the lower alkoxy, the lower alkoxycarbonyl and the N-(lower alkanoyl)-N-(lower alkyl)amino have the same meanings as (ii) defined above; the alkylene moiety of the aralkyl has the same meaning as (iii) defined above; the aryl and the aryl moiety of the aralkyl, the aroyl and the arylsulfonyl have the same meanings as (vi) defined above; the lower alkanoyl and the lower alkanoyl moiety of the lower alkanoylamino and the N-(lower alkanoyl)-N-(lower alkyl)amino have the same meanings as (vii) defined above; the heterocyclic group has the same meaning as (viii) defined above; the heterocyclic group formed together with the adjacent nitrogen atom has the same meaning as (ix) defined above; and the heteroaroyl has the same meaning as (x) defined above.

(xii) Examples of the substituents in the substituted aryl, the substituted aroyl, the substituted aralkyl, the substituted arylsulfonyl, the substituted heteroaroyl, the substituted heterocyclic group and the substituted heterocyclic group formed together with the adjacent nitrogen atom, which may be the same or different and is 1 to 3 in number, include

(xii-a) halogen,

(xii-b) nitro,

(xii-c) nitroso,

(xii-d) carboxy,

(xii-e) substituted or unsubstituted lower alkyl [the substituent(s) in the substituted lower alkyl has the same meaning as (xi) defined above],

(xii-f) substituted or unsubstituted lower alkenyl [the substituent(s) in the substituted lower alkenyl has the same meaning as (xi) defined above],

(xii-g) substituted or unsubstituted lower alkynyl [the substituent(s) in the substituted lower alkynyl has the same meaning as (xi) defined above],

(xii-h) substituted or unsubstituted lower alkoxycarbonyl [the substituent(s) in the substituted lower alkoxycarbonyl has the same meaning as (xi) defined above],

(xii-i) substituted or unsubstituted lower alkanoyl [the substituent(s) in the substituted lower alkanoyl has the same meaning as (xi) defined above],

(xii-j) substituted or unsubstituted aryl [the substituent(s) in the substituted aryl, which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example hydroxy or the like), substituted or unsubstituted lower alkoxy (the substituent(s) in the substituted lower alkoxy, which is 1 to 3 in number, is for example hydroxy or the like) or the like],

(xii-k) $NR^{38a}R^{38b}$ {wherein $R^{38a}$ and $R^{38b}$ may be the same or different and each represents a hydrogen atom, lower alkylsulfonyl, substituted or unsubstituted lower alkyl [the substituent(s) in the substituted lower alkyl has the same meaning as (xi) defined above], substituted or unsubstituted lower alkenyl [the substituent(s) in the substituted lower alkenyl has the same meaning as (xi) defined above], substituted or unsubstituted lower alkynyl [the substituent(s) in the substituted lower alkynyl has the same meaning as (xi) defined above], substituted or unsubstituted lower alkoxy [the substituent(s) in the substituted lower alkoxy has the same meaning as (xi) defined above], substituted or unsubstituted lower alkanoyl [the substituent(s) in the substituted lower alkanoyl has the same meaning as (xi) defined above], substituted or unsubstituted aryl [the substituent(s) in the substituted aryl, which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example hydroxy or the like), substituted or unsubstituted lower alkoxy (the substituent(s) in the substituted lower alkoxy, which is 1 to 3 in number, is for example hydroxy or the like) or the like], substituted or unsubstituted aroyl [the substituent(s) in the substituted aroyl, which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent (s) in the substituted lower alkyl, which is 1 to 3 in number, is for example hydroxy or the like), substituted or unsubstituted lower alkoxy (the substituent(s) in the substituted lower alkoxy, which is 1 to 3 in number, is for example hydroxy or the like) or the like] or a substituted or unsubstituted heterocyclic group [the substituent(s) in the substituted heterocyclic group, which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example hydroxy or the like), substituted or unsubstituted lower alkoxy

(the substituent(s) in the substituted lower alkoxy, which is 1 to 3 in number, is for example hydroxy or the like) or the like], or $R^{38a}$ and $R^{38b}$ are combined together with the adjacent nitrogen atom thereto to form a substituted or unsubstituted heterocyclic group [the substituent(s) in the substituted heterocyclic group formed with the adjacent nitrogen atom, which is 1 to 3 in number, is for example, halogen, amino, nitro, hydroxy, oxo, cyano, carboxy, lower alkoxycarbonyl, aralkyl, aroyl, heteroaroyl, substituted or unsubstituted lower alkyl (the substituent(s) in the substituted lower alkyl, which is 1 to 3 in number, is for example, hydroxy, lower alkoxy or the like), substituted or unsubstituted lower alkoxy (the substituent(s) in the substituted lower alkoxy, which is 1 to 3 in number, is for example, hydroxy, lower alkoxy or the like), substituted or unsubstituted lower alkanoyl (the substituent(s) in the substituted lower alkanoyl, which is 1 to 3 in number, is for example, amino, hydroxy, lower alkoxy, lower alkanoylamino, N-(lower alkanoyl)-N-(lower alkyl)amino or the like), substituted or unsubstituted aliphatic heterocycle-carbonyl (the substituent(s) in the substituted aliphatic heterocycle-carbonyl, which is 1 to 3 in number, is for example, halogen, hydroxy, oxo, lower alkyl, lower alkoxy or the like) or the like]},

(xii-l) $CONR^{39a}R^{39b}$ (wherein $R^{39a}$ and $R^{39b}$ have the same meanings as $R^{38a}$ and $R^{38b}$ defined above, respectively),

(xii-m) $OR^{40}$ {wherein $R^{40}$ represents a hydrogen atom, substituted or unsubstituted lower alkyl [the substituent(s) in the substituted lower alkyl has the same meaning as (xi) defined above], substituted or unsubstituted aryl [the substituent(s) in the substituted aryl, which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent (s) in the substituted lower alkyl, which is 1 to 3 in number, is for example hydroxy or the like), substituted or unsubstituted lower alkoxy (the substituent(s) in the substituted lower alkoxy, which is 1 to 3 in number, is for example hydroxy or the like) or the like] or a substituted or unsubstituted heterocyclic group [the substituent(s) in the substituted heterocyclic group, which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example hydroxy or the like), substituted or unsubstituted lower alkoxy (the substituent(s) in the substituted lower alkoxy, which is 1 to 3 in number, is for example hydroxy or the like) or the like] or the like },

(xii-n) heteroaroyl,

(xii-o) substituted or unsubstituted aliphatic heterocycle-carbonyl [the substituent(s) in the substituted aliphatic heterocycle-carbonyl, which is 1 to 3 in number, is for example, halogen, hydroxy, oxo, lower alkyl, lower alkoxy or the like], or the like.

The substituent(s) in the substituted heterocyclic group, and the substituent(s) in the substituted heterocyclic group formed together with the adjacent nitrogen atom may be, in addition to the above (xii-a) to (xii-o), the following (xii-p) or (xii-q):

(xii-p) oxo

(xii-q) $-O(CR^{41a}R^{41b})_{na}O-$ (wherein $R^{41a}$ and $R^{41b}$ may be the same or different and each represents a hydrogen atom or lower alkyl, na represents 2 or 3, and the two terminal oxygen atoms are combined to the same carbon atom in the heterocyclic group of the substituted heterocyclic group or the substituted heterocyclic group formed with the adjacent nitrogen atom)

In the definition of the substituents (xii) in the substituted aryl, the substituted aroyl, the substituted aralkyl, the substituted arylsulfonyl, the substituted heteroaroyl, the substituted heterocyclic group and the substituted heterocyclic group formed together with the adjacent nitrogen atom, the halogen has the same meaning as (i) defined above; the lower alkyl and the lower alkyl moiety of the N-(lower alkanoyl)-N-(lower alkyl)amino, the lower alkoxy, the lower alkoxycarbonyl and the lower alkylsulfonyl have the same meanings as (ii) defined above; the alkylene moiety of the aralkyl has the same meaning as (iii) defined above; the lower alkenyl has the same meaning as (iv) defined above; the lower alkynyl has the same meaning as (v) defined above; the aryl and the aryl moiety of the aroyl and the aralkyl have the same meanings as (vi) defined above; the lower alkanoyl and the lower alkanoyl moity of the lower alkanoylamino and the N-(lower alkanoyl)-N-(lower alkyl)amino have the same meaning as (vii) defined above; the heterocyclic group has the same meaning as (viii) defined above; the aliphatic heterocyclic moiety of the aliphatic heterocycle-carbonyl has the same meaning as (viii-b) defined above; the heterocyclic group formed with the adjacent nitrogen atom has the same meaning as (ix) defined above; the heteroaroyl has the same meaning as (x) defined above.

Examples of the pharmaceutically acceptable salts of Compound (I), (Ia), (Ib), (Ib-1), (Ib-2), (Ic), (II) and (III) include, for example, pharmaceutically acceptable acid addition salts, metal salts, ammonium salts, organic amine addition salts, amino acid addition salts and the like. The acid addition salts include, for example, inorganic acid salts such as hydrochlorides, sulfates and phosphates; and organic acid salts such as acetate, maleate, fumarate, tartrates, citrates, lactates, aspartates, and glutamates. The metal salts include, for example, alkali metal salts such as sodium salts and potassium salts; alkaline earth metal salts such as magnesium salts and calcium salts; as well as aluminum salts, zinc salts and the like. The ammonium salts include, for example, salts of ammonium, tetramethylammonium and the like. The organic amine addition salts include, for example, morpholine salts, piperidine salts and the like. The amino

acid addition salts include, for example, lysine salts, glycine salts, phenylalanine salts and the like.

When it is desired to obtain salts of Compounds (I), (Ia), (Ib), (Ib-1), (Ib-2), (Ic), (II) and (III), the salt may be purified as it is, if Compounds (I), (Ia), (Ib), (Ib-1), (Ib-2), (Ic), (II) and (III) are obtained in a form of a salt; and if Compounds (I), (Ia), (Ib), (Ib-1), (Ib-2), (Ic), (II) and (III) is obtained in a free form, it is dissolved or suspended in an appropriate solvent followed by adding an acid or a base thereto to form a salt.

There can be isomers such as positional isomers, geometrical isomers or optical isomers in Compounds (I), (Ia), (Ib), (Ib-1), (Ib-2), (Ic), (II) and (III). All possible isomers including these isomers, and mixtures of the isomers in any ratio can be used as pharmaceutical composition of the present invention.

Compounds (I), (Ia), (Ib), (Ib-1), (Ib-2), (Ic), (II) and (III) or the pharmaceutically acceptable salts thereof may exist in a form of adducts to water or various solvents. These adducts can also be used as pharmaceutical composition of the present inhibition.

In the pharmaceutical composition of the present invention, examples of the compound used in combination with the Flt-3 inhibitor include antitumor agents and proteins or low molecular compounds other than antitumor agents. Examples of antitumor agents include protein drugs, chemotherapeutic agents, hormonal therapeutic agents, molecular targeted drugs, differentiation-inducing agents, osteoclastic inhibitors, nucleic acid drugs (siRNA and antisense oligos), and other compounds used for treating cancers. Furthermore, as the therapeutic method used in combination with the Flt-3 inhibitor, radiation irradiation may be mentioned.

Examples of protein drugs include cytokines, antibodies, and the like.

Examples of cytokines include interleukin-2 (IL-2), IFN-$\alpha$, IFN-$\gamma$, GM-CSF, G-CSF, TNF-$\alpha$, IL-1$\beta$, and the like.

Examples of antibodies include anti-EGFR antibodies [cetuximab (Erbitux)], anti-ErbB2 antibodies [trastuzumab (Herceptin)], anti-VEGF antibodies [bevacizumab (Avastin)], anti-CD20 antibodies [rituximab (Rituxan)], anti-CD33 antibodies [gemtuzumab ozogamicin (Mylotarg)], anti-CD52 antibodies [alemtuzumab (Campath)], anti-TRAIL antibodies, and the like.

Examples of chemotherapeutic agents include tubulin acting agents, DNA acting agents, antimetabolites, and the like.

Examples of hormonal therapeutic agents include anti-androgen drugs, anti-estrogen drugs, androgen preparations, estrogen preparations, LH-RH agonists (chemical castration drugs), progestin, aromatase inhibitors, steroid sulfatase inhibitors, and the like.

Examples of molecular targeted drugs include Bcr-Abl inhibitors, EGFR inhibitors, JAK inhibitors, multikinase inhibitors, kinesin Eg5 inhibitors, Flt-3 inhibitors, mTOR inhibitors, proteasome inhibitors, HDAC inhibitors, DNA methylation inhibitors, heat shock protein 90 (Hsp90) inhibitors, farnesyltransferase inhibitors, Bcl-2 inhibitors, and the like.

Examples of tubulin acting agents include vinblastine, vindesine, vincristine, vinorelbine, paclitaxel (Taxol), docetaxel (Taxotere), and the like.

Examples of DNA acting agents include chlorambucil, cyclophosphamide, melpharan, cisplatin, carboplatin, dacarbazine (DTIC), oxaloplatin, bleomycin, doxorubicin (adriamycin), doxorubicin lipo (doxil), daunorubicin, idarubicin, mitomycin, mitoxantrone, etoposide, camptothecin, CPT-11,10-hydroxy-7-ethyl-camptothecin (SN38), irinotecan, topotecan, 5-azacytidine, decitabine, and the like.

Examples of antimetabolites include 5-fluorouracil, fludarabine, hydroxyurea, cytarabine, methotrexate, capecitabine, gemcitabine (gemzar), tegafur-uracil (UFT), clofarabine, nelarabine, and the like.

Examples of hormonal therapeutic agents include leuprolide, goserelin, megestrol, tamoxifen, ICI182780, Tremifene, fadrozole, letrozole, flutamide, bicalutamide, testolactone, mitotane, prednisolone, dexamethasone, and the like.

Examples of molecular targeted drugs include gefitinib (Iressa); Erlotinib (Tarceva); lapatinib (Tykerb) [HKI-272, BIBW-2992, BMS-599626]; imatinib (Gleevec) [STI571]; dasatinib (Sprycel) [BMS-354825]; nilotinib (Tasigna) [AMN107]; sunitinib (SUTENT) [SU11248]; sorafenib (Nexabar) [BAY43-9006]; CHIR-258; vatalanib (PTK-787); R-1155777 (tipifarnib, zarnestra); rapamycin; temsirolimus (CCI-779); bortezomib (Velcade) [PS-341]; asparaginase; pegaspargase; and the like. Examples of Flt-3 inhibitors include CEP-701, PKC412, MLN518, CHIR-258, and the like. Examples of HDAC inhibitors include Vorinostat (suberanilohydroxamic acid, SAHA, Zolinza), valproic acid (VPA), MS-275, and the like. Examples of Hsp90 inhibitors include Radicicol, Geldanamycin, 17-allylamino-17-demethoxygeldanamycin (17-AAG), 17-dimethylaminoethylamino-17-demethoxygeldanamycin (17-DMAG), Herbimycin A, Novobiocin, a benzoyl compound represented by formula (IV)

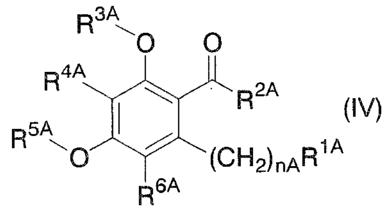

(IV)

[wherein nA represents an integer of 1 to 5; $R^{1A}$ represents substituted or unsubstituted lower alkyl, substituted or unsubstituted lower alkoxy, substituted or unsubstituted cycloalkyl, substituted or unsubstituted lower alkoxycarbonyl, substituted or unsubstituted heterocycle-alkyl, substituted or unsubstituted aryl, $CONR^{7A}R^{8A}$ (wherein $R^{7A}$ and $R^{8A}$ may be the same or different, and each represents a hydrogen atom, substituted or unsubstituted lower alkyl, substituted or unsubstituted cycloalkyl, substituted or unsubstituted lower alkanoyl, substituted or unsubstituted aryl, a substituted or unsubstituted heterocyclic group, substituted or unsubstituted aralkyl, substituted or unsubstituted heterocycle-alkyl, or substituted or unsubstituted aroyl, or $R^{7A}$ and $R^{8A}$ are combined together with the adjacent nitrogen atom thereto to form a substituted or unsubstituted heterocyclic group), or $NR^{9A}R^{10A}$ (wherein $R^{9A}$ and $R^{10A}$ have the same meanings as the above $R^{7A}$ and $R^{8A}$, respectively);
$R^{2A}$ represents substituted or unsubstituted aryl or a substituted or unsubstituted aromatic heterocyclic group;
$R^{3A}$ and $R^{5A}$ may be the same or different, and each represents a hydrogen atom, substituted or unsubstituted lower alkyl, substituted or unsubstituted lower alkenyl, substituted or unsubstituted lower alkanoyl, substituted or unsubstituted cycloalkyl, substituted or unsubstituted aralkyl, or substituted or unsubstituted aroyl;
$R^{4A}$ represents a hydrogen atom, hydroxy, or halogen; and
$R^{6A}$ represents a hydrogen atom, halogen, cyano, nitro, substituted or unsubstituted lower alkyl, substituted or unsubstituted lower alkenyl, substituted or unsubstituted lower alkynyl, substituted or unsubstituted lower alkoxy, substituted or unsubstituted cycloalkyl, amino, lower alkylamino, di(lower alkyl)amino, carboxy, substituted or unsubstituted lower alkoxycarbonyl, substituted or unsubstituted lower alkanoyl, substituted or unsubstituted aryloxy, substituted or unsubstituted aryl, a substituted or unsubstituted heterocyclic group, substituted or unsubstituted aralkyl, or substituted or unsubstituted heterocycle-alkyl;
with the proviso that

(i) when $R^{3A}$ and $R^{5A}$ are methyl, and $R^{4A}$ and $R^{6A}$ are hydrogen atoms, and

(a) when $-(CH_2)_{nA}R^{1A}$ is methoxycarbonylmethyl,
$R^{2A}$ is not a group selected from 2,4,6-trimethoxy-5-methoxycarbonyl-3-nitrophenyl, 3-cyano-2,4,6-trimethoxyphenyl, 5-cyano-2-ethoxy-4,6-dimethoxy-3-nitrophenyl, 2,6-dimethoxyphenyl, 2-chloro-6-methoxyphenyl and 2-chloro-4,6-dimethoxy-5-methoxycarbonyl-3-nitropluenyl,
(b) when $-(CH_2)_{nA}R^{1A}$ is ethoxycarbonylmethyl, $R^{2A}$ is not 2,4,6-trimethoxy-3-methoxycarbonylphenyl, and
(c) when $-(CH_2)_{nA}R^{1A}$ is N,N-dimethylaminomethyl, $R^{2A}$ is not phenyl,

(ii) when $R^{3A}$, $R^{4A}$, $R^{5A}$ and $R^{6A}$ are hydrogen atoms, and

(a) when $-(CH_2)_{nA}R^{1A}$ is 2-(acetoxymethyl)heptyl, 3-oxopentyl or pentyl, $R^{2A}$ is not 6-hydroxy-4-methoxy-3-methoxycarbonyl-2-pentylphenyl,
(b) when $-(CH_2)_{nA}R^{1A}$ is 3-oxopentyl, $R^{2A}$ is not a group selected from 3-benzyloxycarbonyl-6-hydroxy-4-methoxy-2-pentylphenyl and 3-carboxy-6-hydroxy-4-methoxy-2-pentylphenyl, and
(c) when $-(CH_2)_{nA}R^{1A}$ is n-propyl, $R^{2A}$ is not 2,4-dihydroxy-6-[(4-hydroxy-2-oxopyran-6-yl)methyl]phenyl,

(iii) when $R^{3A}$ and $R^{4A}$ are hydrogen atoms, $R^{5A}$ is methyl, $R^{6A}$ is methoxycarbonyl and $-(CH_2)_{nA}R^{1A}$ is pentyl, $R^{2A}$ is not a group selected from 6-[2-(acetoxymethyl)heptyl]-2,4-dihydroxyphenyl, 2,4-dihyroxy-6-pentylphenyl and 2,4-dihydroxy-6-(3-oxopentyl)phenyl,
(iv) when $R^{3A}$ and $R^{5A}$ are benzyl, $R^{4A}$ and $R^{6A}$ are hydrogen atoms, and $-(CH_2)_{nA}R^{1A}$ is 3-oxopentyl, $R^{2A}$ is not a group selected from 6-benzyloxy-4-methoxy-3-methoxycarbonyl-2-pentylphenyl and 6-benzyloxy-3-benzyloxycarbonyl-4-methoxy-2-pentylphenyl,
(v) when $R^{3A}$ is benzyl, $R^{4A}$ is a hydrogen atom, $R^{5A}$ is methyl, - $(CH_2)_{nA}R^{1A}$ is pentyl and $R^{6A}$ is methoxycarbonyl or benzyloxycarbonyl, $R^{2A}$ is not 2,4-bis(benzyloxy)-6-(3-oxopentyl)phenyl,
(vi) when $R^{3A}$ and $R^{4A}$ are hydrogen atoms, $R^{5A}$ is methyl, $-(CH_2)_{nA}R^{1A}$ is pentyl, and $R^{6A}$ is carboxy or benzyloxycarbonyl, $R^{2A}$ is not 2,4-dihydroxy-6-(3-oxopentyl)phenyl, and

(vii) when $R^{3A}$, $R^{4A}$, and $R^{6A}$ are hydrogen atoms, $R^{5A}$ is n-propyl, and $-(CH_2)_{nA}R^{1A}$ is 5-(1,1-dimetylpropyl)-4-(2-hydrobenzotriazol-2-yl)-2-hydroxyphenylmethyl,

$R^2$ is not phenyl], or a prodrug thereof; or a pharmaceutically acceptable salt thereof, a benzene derivative represented by formula (V)

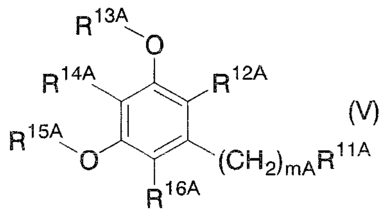

(V)

{wherein mA represents an integer of 0 to 10;

$R^{11A}$ represents a hydrogen atom, hydroxy, cyano, carboxy, nitro, halogen, substituted or unsubstituted lower alkyl, substituted or unsubstituted lower alkenyl, substituted or unsubstituted lower alkynyl, substituted or unsubstituted cycloalkyl, substituted or unsubstituted lower alkoxycarbonyl, substituted or unsubstituted aroyl, substituted or unsubstituted lower alkanoyl, substituted or unsubstituted heterocycle-alkyl, substituted or unsubstituted aryl, substituted or unsubstituted aralkyl, substituted or unsubstituted arylsulfonyl, a substituted or unsubstituted heterocyclic group, $CONR^{17A}R^{18A}$ (wherein $R^{17A}$ and $R^{18A}$ may be the same or different, and each represents a hydrogen atom, substituted or unsubstituted lower alkyl, substituted or unsubstituted cycloalkyl, substituted or unsubstituted lower alkanoyl, substituted or unsubstituted aryl, a substituted or unsubstituted heterocyclic group, substituted or unsubstituted aralkyl, substituted or unsubstituted heterocycle-alkyl, or substituted or unsubstituted aroyl, or $R^{17A}$ and $R^{18A}$ are combined together with the adjacent nitrogen atom thereto to form a substituted or unsubstituted heterocyclic group), or $NR^{19A}R^{20A}$ [wherein $R^{19A}$ and $R^{20A}$ may be the same or different, and each represents a hydrogen atom, substituted or unsubstituted lower alkylsulfonyl, substituted or unsubstituted lower alkyl, substituted or unsubstituted cycloalkyl, substituted or unsubstituted lower alkanoyl, substituted or unsubstituted aryl, a substituted or unsubstituted heterocyclic group, substituted or unsubstituted aralkyl, substituted or unsubstituted heterocycle-alkyl, substituted or unsubstituted aroyl, or $CONR^{21A}R^{22A}$ (wherein $R^{21A}$ and $R^{22A}$ have the same meanings as $R^{17}$ and $R^{18}$ defined above, respectively), or $R^{19A}$ and $R^{20A}$ are combined together with the adjacent nitrogen atom thereto to form a substituted or unsubstituted heterocyclic group] or $OR^{23A}$ (wherein $R^{23A}$ represents substituted or unsubstituted lower alkyl, substituted or unsubstituted lower alkenyl, substituted or unsubstituted lower alkanoyl, substituted or unsubstituted aryl, a substituted or unsubstituted heterocyclic group, substituted or unsubstituted aralkyl, or substituted or unsubstituted heterocycle-alkyl; $R^{12A}$ represents substituted or unsubstituted lower alkyl, substituted or unsubstituted lower alkenyl, substituted or unsubstituted lower alkynyl, substituted or unsubstituted aryl or a substituted or unsubstituted heterocyclic group (wherein substituted or unsubstituted pyrazolyl is not included);

$R^{13A}$ and $R^{15A}$ may be the same or different, and each represents a hydrogen atom, substituted or unsubstituted lower alkyl, substituted or unsubstituted lower alkenyl, substituted or unsubstituted lower alkanoyl, substituted or unsubstituted cycloalkyl, substituted or unsubstituted lower alkylsulfonyl, substituted or unsubstituted arylsulfonyl, carbamoyl, sulfamoyl, substituted or unsubstituted lower alkylaminocarbonyl, substituted or unsubstituted di(lower alkyl)aminocarbonyl, substituted or unsubstituted lower alkoxycarbonyl, substituted or unsubstituted heterocycle-carbonyl, substituted or unsubstituted aralkyl, or substituted or unsubstituted aroyl; $R^{14A}$ and $R^{16A}$ may be the same or different, and each represents a hydrogen atom, hydroxy, halogen, cyano, nitro, substituted or unsubstituted lower alkyl, substituted or unsubstituted lower alkenyl, substituted or unsubstituted lower alkynyl, substituted or unsubstituted lower alkoxy, substituted or unsubstituted cycloalkyl, amino, lower alkylamino, di(lower alkyl)amino, carboxy, substituted or unsubstituted lower alkoxycarbonyl, substituted or unsubstituted aryloxy, substituted or unsubstituted aryl, a substituted or unsubstituted heterocyclic group (wherein substituted or unsubstituted pyrazoryl is not included), substituted or unsubstituted lower alkanoyl, substituted or unsubstituted aralkyl, or substituted or unsubstituted heterocycle-alkyl}, or a prodrug thereof; or a pharmaceutically acceptable salt thereof, and the like.

In the definitions of the groups in general formula (IV) and (V):

Examples of the lower alkyl and the lower alkyl moieties of the lower alkoxy, the lower alkoxycarbonyl, the lower alkylamino, the di(lower alkyl)amino, the lower alkylaminocarbonyl, the di(lower alkyl)aminocarbonyl, and the lower alkylsulfonyl include, for example, linear or branched alkyl having 1 to 8 carbon atom(s), such as methyl, ethyl, propyl, isopropyl, butyl, *sec*-butyl, *tert*-butyl, pentyl, isopentyl, neopentyl, hexyl, heptyl, octyl, and the like. The two lower alkyl moieties of the di(lower alkyl)amino and the di(lower alkyl)aminocarbonyl may be the same or different.

Examples of the lower alkenyl include straight-chain or branched alkenyl having 2 to 8 carbon atoms, such as vinyl, allyl, 1-propenyl, methacryl, crotyl, 1-butenyl, 3-butenyl, 2-pentenyl, 4-pentenyl, 2-hexenyl, 5-hexenyl, 2-heptenyl and 2-octenyl.

Examples of the lower alkynyl include straight-chain or branched alkynyl having 2 to 8 carbon atoms, such as ethynyl, propynyl, butynyl, pentynyl, hexynyl, heptynyl and octynyl.

Examples of the lower alkanoyl include straight-chain or branched alkanoyl having 1 to 7 carbon atoms, such as formyl, acetyl, propionyl, butyryl, isobutyryl, valeryl, isovaleryl, pivaloyl, hexanoyl and heptanoyl.

Examples of the cycloalkyl include cycloalkyl having 3 to 8 carbon atoms, such as cyclopropyl, cyclobutyl, cyclopentyl, cyclohexyl, cycloheptyl and cyclooctyl.

Examples of the aryl and aryl moieties of the arylsulfonyl, aryloxy and aroyl include monocyclic, bicyclic or tricyclic aryl having 6 to 14 carbon atoms, such as phenyl, indenyl, naphthyl and anthryl.

Examples of the aralkyl include aralkyl having 7 to 15 carbon atoms, such as benzyl, phenethyl, benzhydryl and naphthylmethyl.

Examples of the aromatic heterocyclic group include 5- or 6- membered monocyclic aromatic heterocyclic groups containing at least one atom selected from a nitrogen atom, an oxygen atom and a sulfur atom, and bicyclic or tricyclic fused aromatic heterocyclic groups containing at least one atom selected from a nitrogen atom, an oxygen atom and a sulfur atom in which 3- to 8-membered rings are fused, such as pyridyl, pyrazinyl, pyrimidinyl, pyridazinyl, quinolinyl, isoquinolinyl, phthalazinyl, quinazolinyl, quinoxalinyl, naphthyridinyl, cinnolinyl, pyrrolyl, pyrazolyl, imidazolyl, triazolyl, tetrazolyl, thienyl, furyl, thiazolyl, oxazolyl, indolyl, indazolyl, benzimidazolyl, benzotriazolyl, benzothiazolyl, benzoxazolyl, purinyl and benzodioxolanyl.

Examples of the heterocyclic group and heterocyclic moieties of the heterocycle-carbonyl and the heterocycle-alkyl include groups described in the above definition of the aromatic heterocyclic groups and also aliphatic heterocyclic groups. Examples of the aliphatic heterocyclic group include 5- or 6- membered monocyclic aliphatic heterocyclic groups containing at least one atom selected from a nitrogen atom, an oxygen atom and a sulfur atom, and bicyclic or tricyclic fused aliphatic heterocyclic groups containing at least one atom selected from a nitrogen atom, an oxygen atom and a sulfur atom in which 3- to 8- membered rings are fused, such as pyrrolidinyl, piperidino, piperazinyl, piperazinyl, morpholino, morpholinyl, thiomorpholino, thiomorpholinyl, homopiperidino, homopiperazinyl, tetrahydropyridinyl, tetrahydroquinolinyl, tetrahydroisoquinolinyl, tetrahydrofuranyl, tetrahydropyranyl, dihydrobenzofuranyl, oxopiperazinyl and 2-oxopyrrolidinyl.

Examples of the heterocyclic group formed together with the adjacent nitrogen atom include 5- or 6-membered monocyclic heterocyclic groups containing at least one nitrogen atom (the monocyclic heterocyclic groups may also contain another nitrogen atom, an oxygen atom or a sulfur atom), and bicyclic or tricyclic fused heterocyclic groups containing at least one nitrogen atom in which 3- to 8-membered rings are fused (the fused heterocyclic groups may also contain another nitrogen atom, an oxygen atom or a sulfur atom), such as pyrrolidinyl, piperidino, piperazinyl, morpholino, thiomorpholino, homopiperidino, homopiperazinyl, tetrahydropyridinyl, tetrahydroquinolinyl, tetrahydroisoquinolinyl, oxopiperazinyl and 2-oxopyrrolidinyl.

The alkylene moiety of the heterocycle-alkyl has the same meaning as a group formed by removing one hydrogen atom from the lower alkyl defined above.

The halogen means each atoms of fluorine, chlorine, bromine and iodine.

Examples of the substituents in the substituted lower alkyl, the substituted lower alkoxy, the substituted lower alkoxycarbonyl, the substituted lower alkylaminocarbonyl, the substituted di(lower alkyl)aminocarbonyl, the substituted lower alkylsulfonyl, the substituted lower alkenyl, and the substituted lower alkynyl include 1 to 3 substituents which may be the same or different, such as hydroxy, oxo, cyano, nitro, carboxy, amino, halogen, substituted or unsubstituted lower alkoxy [the substituent(s) in the substituted lower alkoxy, which is 1 to 3 in number, is for example, hydroxy, halogen or the like], cycloalkyl, lower alkanoyl, lower alkoxycarbonyl, lower alkylamino and di(lower alkyl)amino [the two lower alkyl moieties of the di(lower alkyl)amino may be the same or different]. The position(s) to be substituted by the substituent (s) is/are not particularly limited. The halogen, the lower alkoxy, the cycloalkyl, the lower alkanoyl, the lower alkoxycarbonyl, the lower alkylamino and the di(lower alkyl)amino have the same meanings as defined above, respectively.

Examples of substituents in the substituted lower alkanoyl, the substituted cycloalkyl, the substituted aryl, the substituted arylsulfonyl, the substituted aryloxy, the substituted aralkyl, the substituted aroyl, the substituted heterocycle-alkyl, the substituted heterocyclic group, the substituted heterocycle-carbonyl, the substituted aromatic heterocyclic group and the substituted heterocyclic group formed together with the adjacent nitrogen atom include 1 to 3 substituents which may be the same or different, such as hydroxy, halogen, nitro, cyano, amino, carboxy, carbamoyl, substituted or unsubstituted lower alkyl, substituted or unsubstituted lower alkoxy, aralkyloxy, lower alkylsulfonyl, lower alkylsulfanyl, cycloalkyl, lower alkoxycarbonyl, lower alkylamino, di(lower alkyl)amino, lower alkanoyl, a heterocyclic group, substituted or unsubstituted aryl, substituted or unsubstituted heterocycle-alkyloxy, and substituted or unsubstituted heterocycle-carbonylalkyloxy. The position(s) to be substituted by substituent(s) is/are not particularly limited. The halogen, the lower alkyl, the lower alkoxy, the cycloalkyl, the lower alkoxycarbonyl, the lower alkylamino, the di(lower alkyl)amino, the lower

alkanoyl, the heterocyclic group and the aryl have the same meanings as defined above,respectively. The lower alkyl moieties of the lower alkylsulfonyl and the lower alkylsulfanyl have the same meanings as the lower alkyl defined above. The aralkyl moiety of the aralkyloxy has the same meaning as the aralkyl defined above. The heterocyclic moieties and the alkylenes of the heterocycle-alkyloxy and heterocycle-carbonylalkyloxy have the same meanings as the heterocyclic group and the group produced by removing a hydrogen atom from the lower alkyl defined above, respectively. Examples of the substituents in the substituted lower alkyl, the substituted lower alkoxy and the substituted aryl include 1 to 3 substituents which may be the same or different, such as hydroxy, halogen, lower alkoxy, cyano, lower alkylamino and di(lower alkyl)amino. Examples of the substituents in the substituted heterocycle-alkyloxy and the substituted heterocycle-carbonylalkyloxy include 1 to 3 substituents which may be the same or different, such as hydroxy, halogen, lower alkyl, lower alkoxy and a heterocyclic group.

The prodrugs of Compounds (IV) and (V) include compounds which are converted in vivo, for example, by various mechanisms such as hydrolysis in blood to form Compounds (IV) and (V) of the present invention, and the like. Such compounds can be specified by techniques well known in the art (e.g. J. Med. Chem., 1997, Vol. 40, p. 2011-2016; Drug Dev. Res., 1995, Vol. 34, p. 220-230; Advances in Drug Res., 1984, Vol. 13, p. 224-331; Bundgaard, Design of Prodrugs, 1985, Elsevier Press and the like).

Examples of the differentiation-inducing agents include, for example, all-trans retinoic acid, arsenic trioxide, thalidomide, lenalidomide, bexarotene (targretin) and the like.

Examples of the osteoclastic inhibitors include, for example, bisphosphonate (Zoledronic acid, Zometa).

There are concerns that when the above compounds may not give sufficient treatment results in the single administration. Also, high-dose administration of the above compounds may cause side effects. By combining the above compounds together with the Flt-3 inhibitors, the present invention provides better treatment results than administering compounds alone, and then the above compounds can be used in a low dosage. Therefore, the present invention not only provides sufficient effect of treatment but also decreases side effects.

Compounds (I), (Ia), (Ib), (Ib-1), (Ib-2), (Ic), (II) and (III) used in the present invention can be synthesized according to the method described in, for example, W02005/012257, WO2005/012258, WO2005/095382 and W02005/095341.

Specific examples of the Compounds (I), (Ia), (Ib), (Ib-1), (Ib-2), (Ic), (II) and (III) used in the present invention will be described in the following Table 1, 2 and 3, but the present invention is not limited to them.

Compounds 1, 2, 3, 4, 5, 7, 8, 9, 10, 12, 13 and 14 can be synthesized according to the Examples 5, 2, 22, 38, 54, 69, 70, 64, 74, 59, 51 and 29 in WO2005/012258. Compounds 6 and 11 can be synthesized according to the Examples 13 and 158 in WO2005/012257. Compounds 16, 17, 18, 19, 20, 21, 22 and 23 can be synthesized according to the Examples 1, 1, 2, 3, 3, 4, 17 and 21 in WO2005/095382. Compounds 24, 25, 26, 27, 28, 29, 30, 31, 32 and 33 can be synthesized according to the Examples 1, 3, 11, 14, 23, 26, 33, 40, 50 and 51 in W02005/095341.

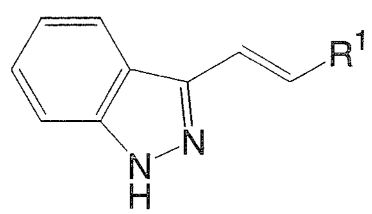

Table 1-1

| Compound No. | $R^1$ | salt |
|---|---|---|
| 1 | 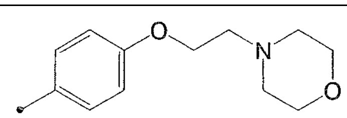 | HCl |
| 2 | 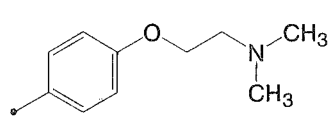 | HCl |

(continued)

| Compound No. | $R^1$ | salt |
|---|---|---|
| 3 | 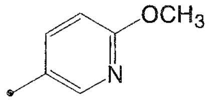 | |
| 4 | 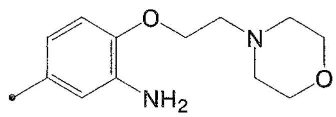 | |
| 5 | 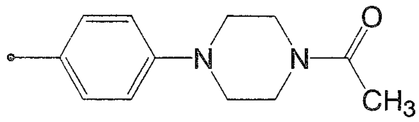 | |
| 6 | 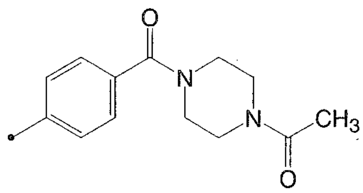 | |
| 7 | 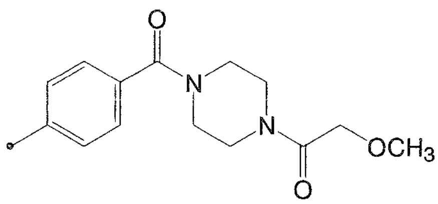 | |

Table 1-2

| Compound No. | $R^1$ | salt |
|---|---|---|
| 8 | 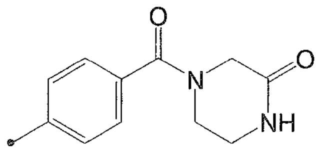 | |
| 9 | 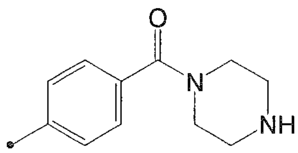 | |
| 10 | 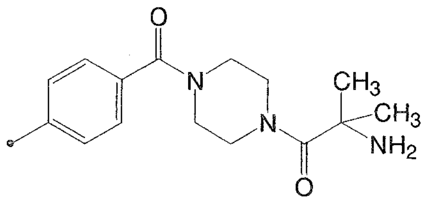 | HCl |

(continued)

| Compound No. | $R^1$ | salt |
|---|---|---|
| 11 | 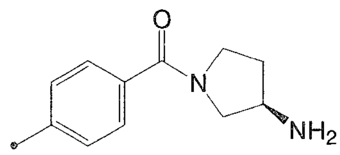 | |
| 12 | 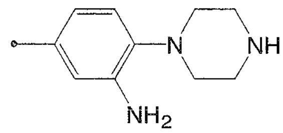 | |
| 13 | 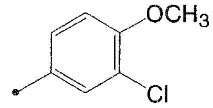 | |
| 14 | 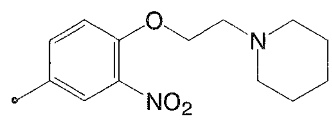 | |

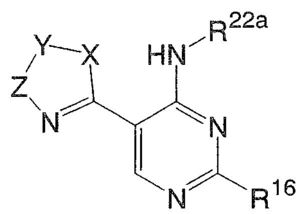

Table 2

| Compound No. | $R^{22a}$ | $R^{16}$ | 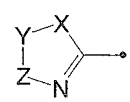 |
|---|---|---|---|
| 16 | $-(CH_2)_2CH_3$ | 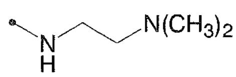 | 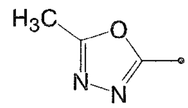 |
| 17 | $-(CH_2)_2CH_3$ | 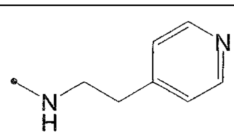 | 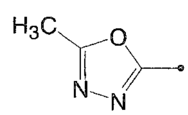 |
| 18 | $-CH_3$ | 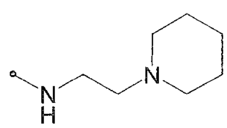 | 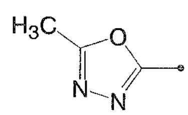 |

(continued)

| Compound No. | $R^{22a}$ | $R^{16}$ | 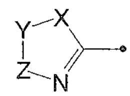 |
|---|---|---|---|
| 19 | 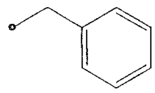 | 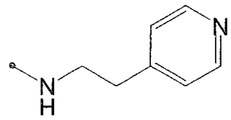 | 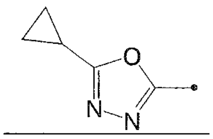 |
| 20 | 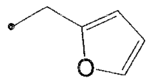 | 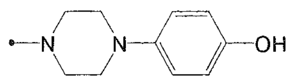 | 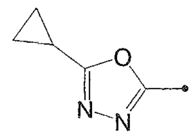 |
| 21 | 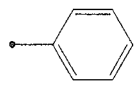 | 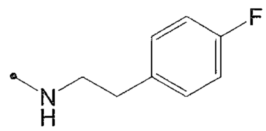 | 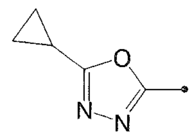 |
| 22 | $-(CH_2)_2CH_3$ | 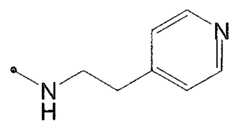 | 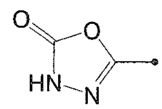 |
| 23 | $-(CH_2)_2CH_3$ | 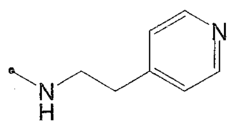 | 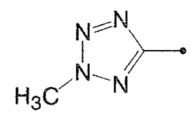 |

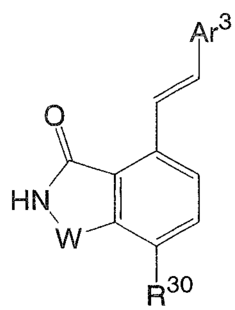

Table 3-1

| Compound No. | W | $Ar^3$ | $R^{30}$ |
|---|---|---|---|
| 24 | C=O | 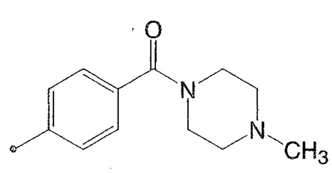 | H |

(continued)

| Compound No. | W | $Ar^3$ | $R^{30}$ |
|---|---|---|---|
| 25 | C=O | 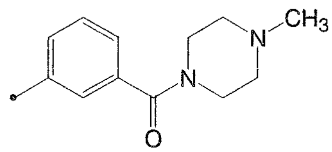 | H |
| 26 | CH(OH) | 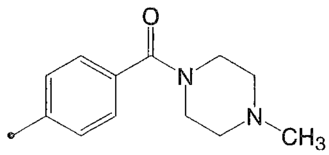 | H |
| 27 | $CH_2$ | 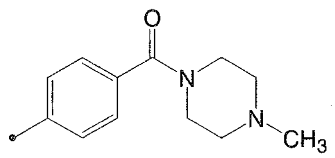 | H |
| 28 | $CH_2$ | 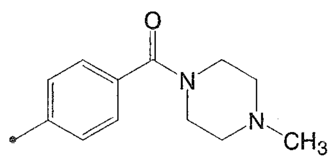 | Cl |
| 29 | $CH_2$ | 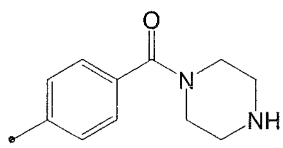 | Cl |

Table 3-2

| Compound No. | $W^A$ | $R^E$ | $R^F$ |
|---|---|---|---|
| 30 | C=O | 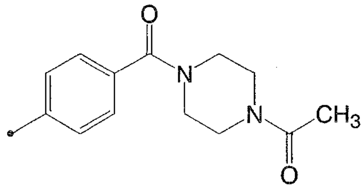 | $NH_2$ |
| 31 | C=O | 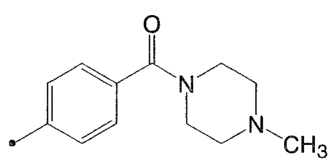 | $NH(COCH_3)$ |

(continued)

| Compound No. | $W^A$ | $R^E$ | $R^F$ |
|---|---|---|---|
| 30 | C=O | 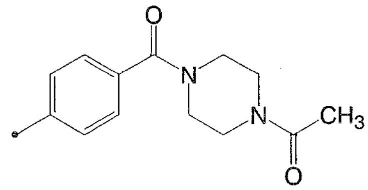 | $NH_2$ |
| 32 | $CH_2$ | 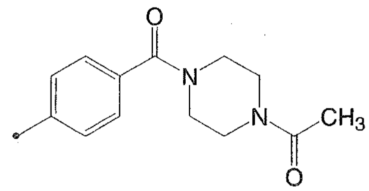 | $NH_2$ |
| 33 | C=O | 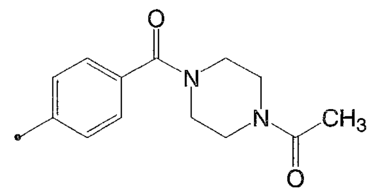 | 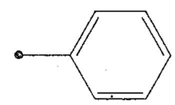 |

The pharmaceutical composition of the present invention can be used in treatment of any cancer, such as cancer derived from hematopoietic tumor (for example, acute myeloid leukemia, chronic myeloid leukemia, acute lymphocytic leukemia, chronic lymphocytic leukemia, multiple myeloma, and lymphoma), breast cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma, or cancer derived from brain tumor. Among them, the pharmaceutical composition is preferably used for acute myeloid leukemia, multiple myeloma, lung cancer, and breast cancer.

The effect of the pharmaceutical composition of the present invention can be examined by analyzing the results of in vitro cell growth inhibitory activity assays using an isobologram method (International Journal of Radiation Oncology, Biology, Physics, 5, 85-91, 1979).

The effect of the pharmaceutical composition of the present invention can be examined by measuring the in vivo antitumor activity using an animal model.

As the animal model, for example, immunodeficient mice, such as nude mice, transplanted with a cultured cell line derived from a cancer tissue may be used.

By comparing the effect of single administration of an indazole derivative, the effect of single administration of a compound to be combined, and the effect of a pharmaceutical composition of the present invention, using the animal model, the effect of the pharmaceutical composition of the present invention can be evaluated.

As the cultured cells to be used, MOLM-13 cells may be used. MOLM-13 cells are derived from a patient with human acute myeloid leukemia, and can be used as a model of acute myeloid leukemia.

As the cultured cells to be used, MIA-Paca-2 cells may be used. MIA-Paca-2 cells are derived from a patient with human pancreas cancer and can be used as a model of pancreas cancer.

As the cultured cells to be used, Colo205 cells may be used. Colo205 cells are derived from a patient with human colon cancer, and can be used as a model of colon cancer.

As the cultured cells to be used, Caki-1 cells may be used. Caki-1 cells are derived from a patient with human kidney cancer, and can be used as a model of kidney cancer.

As long as the pharmaceutical composition of the present invention is formed into a preparation so as to contain an Flt-3 inhibitor and at least one compound to be combined therewith, it can be used, administered, or produced as a single agent (mixture) or as a combination of a plurality of preparations. The pharmaceutical compositions desirably have a unit dose form suitable for oral administration or parenteral administration, such as injections. When a combination of a plurality of preparations is used or administered, the preparations may be used together or separately at intervals.

These preparations can be produced by an ordinary method using, in addition to the active ingredients, a pharmaceutically acceptable diluent, excipient, disintegrant, lubricant, binder, surfactant, water, physiological saline, vegetable oil solubilizer, isotonizing agent, preservative, antioxidant, and the like.

When tablets are prepared, for example, an excipient such as lactose, a disintegrant such as starch, a lubricant such as magnesium stearate, a binder such as hydroxypropyl cellulose, a surfactant such as a fatty ester, a plasticizer such as glycerin, etc. may be used in the ordinary manner.

When injections are prepared, for example, water, physiological saline, vegetable oil, a solvent, a solubilizer, an isotonizing agent, a preservative, an antioxidant, etc. may be used in the ordinary manner.

When Compound (I), (Ia), (Ib), (Ib-1), (Ib-2), (Ic), (II), (III), or a pharmaceutically acceptable salt thereof is used for the purposes described above, it can be administered orally or parenterally as an injection or the like. The effective dose and number of doses thereof may vary depending on the administration form, and the age, body weight, symptom, etc. of patients. The recommended daily dose is usually 0.01 to 20 mg/kg.

The pharmacological action of the pharmaceutical composition of the present invention will now be described more specifically with reference to test examples. The compounds used in the test examples to be combined with the Flt-3 inhibitors were obtained as commercial products or synthesized by known methods.

Test Example 1: In vitro cell growth inhibitory activity assays and analysis of combined effects using isobologram method

The cell growth inhibitory ratios of test compounds against human acute myeloid leukemia cell line (MOLM-13), human acute lymphocytic leukemia cell line (RS4;11), and human multiple myeloma cell line (NCI-H929) were measured by the method described below.

For the culture of MOLM-13 cells, Roswell Park Memorial Institute's Medium (RPMI) 1640 (Gibco Corp.) containing 10% fetal calf serum (FCS, Invitrogen) was used. For the culture of RS4;11 cells, Roswell Park Memorial Institute's Medium (RPMI) 1640 containing 10% fetal calf serum (FCS), 1 mmol/L sodium pyruvate (Invitrogen), 10 mmol/L HEPES (Invitrogen), and 4.5 g/L D-glucose (Sigma-Aldrich) was used. In the culture of NCI-H929 cells, RPMI 1640 medium (Invitrogen) containing 10% FCS, 10 mmol/L HEPES (Invitrogen), 1 mmol/L sodium pyruvate, 4.5 g/L glucose, and 50 μmol/L 2-mercaptoethanol (Sigma-Aldrich) was used.

MOLM-13 cells prepared at $3.8 \times 10^4$ cells/mL (RS4;11 cells at $25 \times 10^4$ cells/mL, NCI-H929 cells at $12.5 \times 10^4$ cells/mL) were seeded in each well of a TC MICROWELL 96U plate (Nalge Nunc International) in the amount of 80 μL per well, and culturing was performed in a 5% carbon dioxide incubator at 37˚C for 24 hours. A solution of each test compound prepared by stepwise dilution with the corresponding cell culture medium was added in the amount of 20 μL per well, and culturing was performed again in a 5% carbon dioxide incubator at 37˚C for 72 hours. The WST-1 reagent {4-[3-(4-Iodophenyl)-2-(4-nitrophenyl)-2H-5-tetrazolio]-1,3-benzene disulfonate sodium salt} (Roche Diagnostic Corporation) in the amount of 10 μL was added to each well, and incubation was performed at 37˚C for 2 hours. Then, using a microplate spectrophotometer, SPECTRA max 340PC (Molecular Devices), the absorbance at 450 nm (reference wavelength of 690 nm or 655 nm) was measured. With respect to the cell growth inhibitory ratio of each compound-added group (test compound alone, compound to be combined alone, or combined group), the absorbance of the well was measured in the case where only the solvent of the compound solution was added and culturing was performed for 72 hours as in the compound-added group, and the absorbance of the well was measured as in the compound group immediately after addition of the solvent. The calculation was made according to the equation shown below.

$$\text{Cell growth inhibition rate (\%)} = 100 - \left( \frac{\text{absorbance at 72 hours after addition of test compound} - \text{absorbance of blank plate}}{\text{absorbance of well added with solvent} - \text{absorbance of blank plate}} \right) \times 100$$

Using the method described above, the 5% to 70% growth inhibitory concentration ($IC_5$ to $IC_{70}$) was calculated with respect to the case where Compound 8, 9, or 11 only, shown in Table 1, or the compound to be combined alone was added, and the $IC_{50}$ and $IC_{70}$ were calculated with respect to the case where the test compound and the compound to be combined were used in combination. The combined effects were analyzed using the isobologram method [International Journal of Radiation Oncology, Biology, Physics, vol. 5, 85 (1979)]. The efficacy of the combination therapy was determined, according to the method described in International Journal of Radiation Oncology, Biology, Physics, 85 (1979), 1145 (1979), or the like, by classifying as supra-additive (synergistic effect), envelope of additivity (additive effect), sub-additive (additive tendency), and protection. The combinations of compounds classified as supra-additive, envelope of additivity, and sub-additive were determined as having combined effects, and the combinations of compounds classified as protection were determined as having no combined effects. The results for each cell are shown in Tables 4 and 5. As the compound to be combined, cytarabine, daunorubicin, etoposide, idarubicin, vincristine, vindesine, doxorubicin, mitoxantrone, R-1155777, bortezomib, 2-{2-ethyl-3,5-dihydroxy-6-[3-methoxy-4-(2-morpholino ethoxy) benzoyl]phenyl}-N,N-bis(2-methoxyethyl)acetamide (WO2005/000778, hereinafter referred to as "Compound P"), melphalan, rapamycin, and lenalidomide were used. In any combination of the compounds, the combination was determined to have the combined effects. It is evident from the above analysis that in any of the compounds to be combined, the effectiveness is enhanced by combination with any of Compounds 8, 9, and 11.

Table 4: Combined effects against MOLM-13 cells due to combinations of compounds

| MOLM-13 | | | |
|---|---|---|---|
| Compound to be combined/Test compound | Compound 8 | Compound 9 | Compound 11 |
| Cytarabine | Sub-additive | Sub-additive | Sub-additive |
| Daunorubicin | Envelope of additivity | Envelope of additivity | Sub-additive |
| Etoposide | Envelope of additivity | Supra-additive | Sub-additive |
| Idarubicin | Envelope of additivity | Sub-additive | Envelope of additivity |
| Vincristine | Envelope of additivity | Envelope of additivity | Sub-additive |
| Vindesine | Envelope of additivity | Envelope of additivity | Sub-additive |
| Doxorubicin | Envelope of additivity | Envelope of additivity | Envelope of additivity |
| Mitoxantrone | Envelope of additivity | Envelope of additivity | Sub-additive |
| R-1155777 | Sub-additive | Envelope of additivity | Sub-additive |
| Bortezomib | Envelope of additivity | Envelope of additivity | Envelope of additivity |
| Compound P | Envelope of additivity | Sub-additive | Sub-additive |

Table 5: Combined effects against RS4;11 cells due to combinations of compounds

| RS-4;11 | | | |
|---|---|---|---|
| Compound to be combined/Test compound | Compound 8 | Compound 9 | Compound 11 |
| Cytarabine | Envelope of additivity | Sub-additive | Envelope of additivity |
| Daunorubicin | Envelope of additivity | Envelope of additivity | Envelope of additivity |
| Etoposide | Envelop of additivity | Envelops of additivity | Envelope of additivity |
| Idarubicin | Envelope of additivity | Envelope of additivity | Envelope of additivity |
| Vincristine | Envelope of additivity | Envelope of additivity | Envelope of additivity |
| Vindesine | Envelope of additivity | Envelope of additivity | Envelope of additivity |
| Doxorubicin | Envelope of additivity | Envelope of additivity | Envelope of additivity |
| Mitoxantrone | Envelope of additivity | Envelope of additivity | Envelope of additivity |
| R-1155777 | Sub-additive | Envelope of additivity | Envelope of additivity |
| Bortezomib | Envelope of additivity | Envelope of additivity | Envelope of additivity |
| Compound P | Protection | Protection | Sub-additive |

Table 6: Combined effects against NCI-H929 cells due to combinations of compounds

| NCI-H929 | |
|---|---|
| Compound to be combined/Test compound | Compound 9 |
| Melphalan | Envelope of additivity |
| Rapamycin | Envelope of additivity |
| Lenalidomide | Envelope of additivity |

Test Example 2: Antitumor effect by combined use of Flt-3 inhibitor and cytarabine in mouse models transplanted with human acute myeloid leukemia MOLM-13 cells

One day before the transplantation of MOLM-13 cells, an anti-asialo GM1 antibody was intraperitoneally injected to Fox C.B-17/Icr-scidJc1 mice (CLEA Japan) in an amount of 0.3 mg per mouse. MOLM-13 cells were cultured and grown in RPMI1640 medium containing 10% fetal calf serum (FCS) in a 5% carbon dioxide incubator at 37°C, and the cultured cells ($1 \times 10^7$ cells/mouse) were subcutaneously, ventrally transplanted into the mice. Five days after the transplantation, the major axis and minor axis of the tumors subcutaneously grown were measured with slide calipers, and the tumor volume was determined according to the equation shown below.

$$\underset{(\mathrm{mm}^3)}{\text{Tumor volume}} = \underset{(\mathrm{mm})}{\text{major axis}} \times \underset{(\mathrm{mm})}{\text{minor axis}} \times \underset{(\mathrm{mm})}{\text{minor axis}} \times \frac{1}{2}$$

At the same time, the body weight of each mouse was measured, and the mice were separated into the groups described below, each consisting of five mice, such that the groups had the similar average tumor volume and the similar average body weight. This day was defined as day zero of the administration test, and pharmaceutical administration was started in the following manner.

Compound 9 was suspended at a concentration of 0.5 mg/mL in distilled water containing 0.5% Methyl Cellulose 400cp. The resulting suspension was orally administered to each mouse in a dose of 0.01 mL per gram of the body weight of the mouse (5 mg/kg) daily, twice a day, from days zero to 13 after the start of administration.

Cytarabine was dissolved in physiological saline (manufactured by Otsuka Pharmaceutical Co., Ltd.) at a concentration of 7.5 mg/mL. The resulting solution was intravenously administered from the caudal vein to each mouse in a dose of 0.01 mL per gram of the body weight of the mouse (75 mg/kg) daily, once a day, from days zero to 4 after the start of administration.

A. Negative control group (Control): non-administration of Compound 9 and cytarabine
B. Compound 9 alone group: 5 mg/kg (twice a day × 14 days)
C. Cytarabine alone group: 75 mg/kg (once a day × 5 days)
D. Compound 9 + cytarabine: 5 mg/kg (twice a day × 14 days) for Compound 9, and 75 mg/kg (once a day × 5 days) for cytarabine

The tumor volume was measured twice a week after day zero. In order to determine the antitumor effect, the average tumor volume was calculated for each group, and the ratios (V/V0) of change in tumor volume were compared, assuming that the tumor volume on day zero was V0. The ratios (V/V0) for the individual groups measured daily are shown in Fig. 1.

As shown in Fig. 1, the combined use of Compound 9 and cytarabine shows a higher growth inhibitory effect than single administration of Compound 9 or cytarabine.

The value (T/C) obtained by dividing the ratio V/V0 of each group by the ratio V/V0 of the negative control group on day 14 is shown in Table 7. In comparison with the theoretical T/C value in the case where the drug efficacy of Compound 9 and the drug efficacy of cytarabine are simply added, i.e., the value obtained by multiplying the T/C value of the Compound 9 alone group by the T/C value of the cytarabine alone group, the T/C in the group of actually combined use (D in Table) is lower (0.097) than 0.18 which is the theoretical value on day 14.

Table 7: T/C of individual groups

| A. Control | B. Compound 9 | C. Cytarabine | D. Compound 9 + Cytarabine | Theoretical value (B × C) |
|---|---|---|---|---|
| 1 | 0.29 | 0.62 | 0.097 | 0.18 |

As is evident from the above, the combined administration of Compound 9 and cytarabine has a higher antitumor effect than the single administration of each of the compounds, and shows a synergistic effect. This result suggests that combined administration of an Flt-3 inhibitor and an antimetabolite has higher antitumor activity than single administration of each of the compounds, and shows a synergistic effect.

Test Example 3: Antitumor effect by combined use of Flt-3 inhibitor and cetuximab in mouse models transplanted with human pancreatic cancer MIA-Paca-2 cells

One day before the transplantation of MIA-Paca-2 cells, an anti-asialo GM1 antibody was intraperitoneally injected to Fox C.B-17/Icr-scidJc1 mice (CLEA Japan) in an amount of 0.3 mg per mouse. MIA-PaCa-2 cells were cultured and grown in MEM medium containing 10% fetal calf serum (FCS) in a 5% carbon dioxide incubator at 37°C, and the cultured cells ($1 \times 10^7$ cells/mouse) were subcutaneously, ventrally transplanted into the mice. Ten days after the transplantation, the major axis and minor axis of the tumors subcutaneously grown were measured with slide calipers, and the tumor volume was determined according to the equation shown below.

$$\text{Tumor volume (mm}^3\text{)} = \text{major axis (mm)} \times \text{minor axis (mm)} \times \text{minor axis (mm)} \times \frac{1}{2}$$

At the same time, the body weight of each mouse was measured, and the mice were separated into the groups described below, each consisting of five mice, such that the groups had the similar average tumor volume and the similar average body weight. This day was defined as day zero of the administration test, and pharmaceutical administration was started in the following manner.

Compound 9 was suspended at a concentration of 5 mg/mL in distilled water containing 0.5% Methyl Cellulose 400cp. The resulting suspension was orally administered to each mouse in a dose of 0.01 mL per gram of the body weight of the mouse (50 mg/kg) daily, twice a day, from days zero to 4 after the start of administration.

Cetuximab was dissolved in physiological saline (manufactured by Otsuka Pharmaceutical Co., Ltd.) at a concentration of 1 mg/mL. The resulting solution was intravenously administered from the caudal vein to each mouse in a dose of 0.25 mL per mouse (2.5 mg/head) on days zero, 3, 6, 9, and 12 after the start of administration, once a day.

A. Negative control group (Control): non-administration of Compound 9 and cetuximab
B. Compound 9 alone group: 50 mg/kg (twice a day × 5 days)
C. Cetuximab alone group: 2.5 mg/head (once a day/days zero, 3, 6, 9, and 12 after the start of administration)
D. Compound 9 + cetuximab: 50 mg/kg (twice a day × 5 days) for Compound 9, and 2.5 mg/head, once a day/days zero, 3, 6, 9, and 12 after the start of administration for cetuximab

The tumor volume was measured twice a week after day zero. In order to determine the antitumor effect, the average tumor volume was calculated for each group, and the ratios (V/V0) of change in tumor volume were compared, assuming that the tumor volume on day zero was V0. The ratios (V/V0) for the individual groups measured daily are shown in Fig. 2.

As shown in Fig. 2, the combined use of Compound 9 and cetuximab shows a higher growth inhibitory effect than single administration of Compound 9 or cetuximab.

The value (T/C) obtained by dividing the ratio V/V0 of each group by the ratio V/V0 of the negative control group on day 24 is shown in Table 8. In comparison with the theoretical T/C value in the case where the drug efficacy of Compound 9 and the drug efficacy of cetuximab are simply added, i.e., the value obtained by multiplying the T/C value of the Compound 9 alone group by the T/C value of the cetuximab alone group, the T/C in the group of actually combined use (D in Table) is lower (0.30) than 0.31 which is the theoretical value on day 24.

Table 8: T/C of individual groups

| A. Control | B. Compound 9 | C. Cetuximab | D. Compound 9 + Cetuximab | Theoretical value (B × C) |
|---|---|---|---|---|
| 1 | 0.59 | 0.53 | 0.30 | 0.31 |

As is evident from the above, the combined administration of Compound 9 and cetuximab has a higher antitumor effect than the single administration of each of the compounds, and shows a synergistic effect. This result suggests that combined administration of an Flt-3 inhibitor and an anti-EGFR antibody has higher antitumor activity than single administration of each of the compounds, and shows a synergistic effect.

Test Example 4: Antitumor effect by combined use of Flt-3 inhibitor and gemcitabine in mouse models transplanted with human pancreatic cancer MIA-Paca-2 cells

One day before the transplantation of MIA-Paca-2 cells, an anti-asialo GM1 antibody was intraperitoneally injected to Fox C.B-17/Icr-scidJc1 mice (CLEA Japan) in an amount of 0.3 mg per mouse. MIA-PaCa-2 cells were cultured and grown in MEM medium containing 10% fetal calf serum (FCS) in a 5% carbon dioxide incubator at 37°C, and the cultured cells ($1 \times 10^7$ cells/mouse) were subcutaneously, ventrally transplanted into the mice. Ten days after the transplantation, the major axis and minor axis of the tumors subcutaneously grown were measured with slide calipers, and the tumor volume was determined according to the equation shown below.

$$\underset{(mm^3)}{\text{Tumor volume}} = \underset{(mm)}{\text{major axis}} \times \underset{(mm)}{\text{minor axis}} \times \underset{(mm)}{\text{minor axis}} \times \frac{1}{2}$$

At the same time, the body weight of each mouse was measured, and the mice were separated into the groups described below, each consisting of five mice, such that the groups had the similar average tumor volume and the similar average body weight. This day was defined as day zero of the administration test, and pharmaceutical administration was started in the following manner.

Compound 9 was suspended at a concentration of 2.5 mg/mL in distilled water containing 0.5% Methyl Cellulose 400cp. The resulting suspension was orally administered to each mouse in a dose of 0.01 mL per gram of the body weight of the mouse (25 mg/kg) daily, twice a day, from days zero to 4 after the start of administration.

Gemcitabine was dissolved in physiological saline (manufactured by Otsuka Pharmaceutical Co., Ltd.) at a concentration of 6 mg/mL. The resulting solution was intravenously administered from the caudal vein to each mouse in a dose of 0.01 mL per gram of the body weight of the mouse (60 mg/kg) on days zero, 3, 7, and 10 after the start of administration, once a day.

A. Negative control group (Control): non-administration of Compound 9 and gemcitabine
B. Compound 9 alone group: 25 mg/kg (twice a day × 5 days)
C. Gemcitabine alone group: 60 mg/kg (once a day/days zero, 3, 7, and 10 after the start of administration)
D. Compound 9 + gemcitabine: 25 mg/kg (twice a day × 5 days) for Compound 9, and 60 mg/kg, once a day/days zero, 3, 7, and 10 after the start of administration for gemcitabine

The tumor volume was measured twice a week after day zero. In order to determine the antitumor effect, the average tumor volume was calculated for each group, and the ratios (V/V0) of change in tumor volume were compared, assuming that the tumor volume on day zero was V0. The ratios (V/V0) for the individual groups measured daily are shown in Fig. 3.

As shown in Fig. 3, the combined use of Compound 9 and gemcitabine shows a higher growth inhibitory effect than single administration of Compound 9 or gemcitabine.

The value (T/C) obtained by dividing the ratio V/V0 of each group by the ratio V/V0 of the negative control group on day 24 is shown in Table 9. In comparison with the theoretical T/C value in the case where the drug efficacy of Compound 9 and the drug efficacy of gemcitabine are simply added, i.e., the value obtained by multiplying the T/C value of the Compound 9 alone group by the T/C value of the gemcitabine alone group, the T/C in the group of actually combined use (D in Table) is lower (0.47) than 0.48 which is the theoretical value on day 24.

Table 9: T/C of individual groups

| A. Control | B. Compound 9 | C. Gemcitabine | D. Compound 9 + Gemcitabine | Theoretical value (B×C) |
|---|---|---|---|---|
| 1 | 0.84 | 0.57 | 0.47 | 0.48 |

As is evident from the above, the combined administration of Compound 9 and gemcitabine has a higher antitumor effect than the single administration of each of the compounds, and shows a synergistic effect. This result suggests that combined administration of an Flt-3 inhibitor and an antimetabolite has higher antitumor activity than single administration of each of the compounds, and shows a synergistic effect.

Test Example 5: Antitumor effect by combined use of Flt-3 inhibitor and 5-fluorouracil (5-FU) in mouse models transplanted with human colon cancer Colo205 cells

Colo205 cells were cultured and grown in RPMI1640 medium containing 10% fetal calf serum (FCS) in a 5% carbon dioxide incubator at 37°C, and the cultured cells ($1 \times 10^7$ cells/mouse) were subcutaneously, ventrally transplanted into BALB/cAJcl-nu mice (CLEA Japan). Ten days after the transplantation, the major axis and minor axis of the tumors subcutaneously grown were measured with slide calipers, and the tumor volume was determined according to the equation shown below.

$$\underset{(mm^3)}{\text{Tumor volume}} = \underset{(mm)}{\text{major axis}} \times \underset{(mm)}{\text{minor axis}} \times \underset{(mm)}{\text{minor axis}} \times \frac{1}{2}$$

At the same time, the body weight of each mouse was measured, and the mice were separated into the groups described below, each consisting of five mice, such that the groups had the similar average tumor volume and the similar average body weight. This day was defined as day zero of the administration test, and pharmaceutical administration was started in the following manner.

Compound 9 was suspended at a concentration of 2.0 mg/mL in distilled water containing 0.5% Methyl Cellulose 400cp. The resulting suspension was orally administered to each mouse in a dose of 0.01 mL per gram of the body weight of the mouse (20 mg/kg) daily, twice a day, from days zero to 13 after the start of administration.

5-FU was suspended in physiological saline at a concentration of 1 mg/mL. The resulting suspension was intravenously administered from the caudal vein to each mouse in a dose of 0.01 mL per gram of the body weight of the mouse (10 mg/kg) daily on days zero to 4 after the start of administration, once a day.

A. Negative control group (Control): non-administration of Compound 9 and 5-FU
B. Compound 9 alone group: 20 mg/kg (twice a day × 5 days)
C. 5-FU alone group: 10 mg/kg (once a day × 5 days)
D. Compound 9 + 5-FU: 20 mg/kg (twice a day × 5 days) for Compound 9, and 10 mg/kg (once a day × 5 days) for 5-FU

The tumor volume was measured twice a week after day zero. In order to determine the antitumor effect, the average tumor volume was calculated for each group, and the ratios (V/V0) of change in tumor volume were compared, assuming that the tumor volume on day zero was V0. The ratios (V/V0) for the individual groups measured daily are shown in Fig. 4.

As shown in Fig. 4, the combined use of Compound 9 and 5-FU shows a higher growth inhibitory effect than single administration of Compound 9 or 5-FU.

The value (T/C) obtained by dividing the ratio V/V0 of each group by the ratio V/V0 of the negative control group on day 7 is shown in Table 10. In comparison with the theoretical T/C value in the case where the drug efficacy of Compound 9 and the drug efficacy of 5-FU are simply added, i.e., the value obtained by multiplying the T/C value of the Compound 9 alone group by the T/C value of the 5-FU alone group, the T/C in the group of actually combined use (D in Table) is equal to 0.55 which is the theoretical value on day 7.

Table 10: T/C of individual groups

| A. Control | B. Compound 9 | C. 5-FU | D. Compound 9 + 5-FU | Theoretical value (B × C) |
|---|---|---|---|---|
| 1 | 0.68 | 0.81 | 0.55 | 0.55 |

As is evident from the above, the combined administration of Compound 9 and 5-FU has a higher antitumor effect than the single administration of each of the compounds, and shows a synergistic effect. This result suggests that combined administration of an Flt-3 inhibitor and an antimetabolite has higher antitumor activity than single administration of each of the compounds, and shows a synergistic effect.

Test Example 6: Antitumor effect by combined use of Flt-3 inhibitor and irinotecan in mouse models transplanted with human colon cancer Colo205 cells

Colo205 cells were cultured and grown in RPMI1640 medium containing 10% fetal calf serum (FCS) in a 5%

carbon dioxide incubator at 37°C, and the cultured cells ($1 \times 10^7$ cells/mouse) were subcutaneously, ventrally transplanted into BALB/cAJcl-nu mice (CLEA Japan). Ten days after the transplantation, the major axis and minor axis of the tumors subcutaneously grown were measured with slide calipers, and the tumor volume was determined according to the equation shown below.

$$\underset{(mm^3)}{\text{Tumor volume}} = \underset{(mm)}{\text{major axis}} \times \underset{(mm)}{\text{minor axis}} \times \underset{(mm)}{\text{minor axis}} \times \frac{1}{2}$$

At the same time, the body weight of each mouse was measured, and the mice were separated into the groups described below, each consisting of five mice, such that the groups had the similar average tumor volume and the similar average body weight. This day was defined as day zero of the administration test, and pharmaceutical administration was started in the following manner.

Compound 9 was suspended at a concentration of 2.0 mg/mL in distilled water containing 0.5% Methyl Cellulose 400cp. The resulting suspension was orally administered to each mouse in a dose of 0.01 mL per gram of the body weight of the mouse (20 mg/kg) daily, twice a day, from days zero to 4 after the start of administration.

Irinotecan was suspended in physiological saline at a concentration of 6.7 mg/mL. The resulting suspension was intravenously administered from the caudal vein to each mouse in a dose of 0.01 mL per gram of the body weight of the mouse (67 mg/kg) on days zero and 3 after the start of administration, once a day.

A. Negative control group (Control): non-administration of Compound 9 and irinotecan
B. Compound 9 alone group: 20 mg/kg (twice a day × 5 days)
C. Irinotecan alone group: 67 mg/kg (once a day/days zero and 3 after the start of administration)
D. Compound 9 + irinotecan: 20 mg/kg (twice a day × 5 days) for Compound 9, and 67 mg/kg (once a day/days zero and 3 after the start of administration) for irinotecan

The tumor volume was measured twice a week after day zero. In order to determine the antitumor effect, the average tumor volume was calculated for each group, and the ratios (V/V0) of change in tumor volume were compared, assuming that the tumor volume on day zero was V0. The ratios (V/V0) for the individual groups measured daily are shown in Fig. 5.

As shown in Fig. 5, the combined use of Compound 9 and irinotecan shows a higher growth inhibitory effect than single administration of Compound 9 or irinotecan.

The value (T/C) obtained by dividing the ratio V/V0 of each group by the ratio V/V0 of the negative control group on day 11 is shown in Table 11. In comparison with the theoretical T/C value in the case where the drug efficacy of Compound 9 and the drug efficacy of irinotecan are simply added, i.e., the value obtained by multiplying the T/C value of the Compound 9 alone group by the T/C value of the irinotecan alone group, the T/C in the group of actually combined use (D in Table) is lower (0.44) than 0.49 which is the theoretical value on day 11.

Table 11: T/C of individual groups

| A. Control | B. Compound 9 | C. Irinotecan | D. Compound 9 + Irinotecan | Theoretical value (B × C) |
|---|---|---|---|---|
| 1 | 0.75 | 0.65 | 0.44 | 0.49 |

As is evident from the above, the combined administration of Compound 9 and irinotecan has a higher antitumor effect than the single administration of each of the compounds, and shows a synergistic effect. This result suggests that combined administration of an Flt-3 inhibitor and a DNA agonist has higher antitumor activity than single administration of each of the compounds, and shows a synergistic effect.

<u>Test Example 7: Antitumor effect by combined use of Flt-3 inhibitor and erlotinib in mouse models transplanted with human renal cancer Caki-1 cells</u>

Caki-1 cells were cultured and grown in MEM1640 medium containing 10% fetal calf serum (FCS), 1 mmol/L sodium pyruvate, and 10 mmol/L HEPES in a 5% carbon dioxide incubator at 37°C, and the cultured cells ($1 \times 10^7$ cells/mouse) were subcutaneously, ventrally transplanted into BALB/cAJcl-nu mice (CLEA Japan). From the mice in which tumors were formed, the tumors were removed. The tumor tissues were cut into small pieces of about 8 $mm^3$, which

were subcutaneously, ventrally transplanted, using a trocar needle, into BALB/cAJcl-nu mice (CLEA Japan) for testing. Twelve days after the transplantation, the major axis and minor axis of the tumors subcutaneously grown were measured with slide calipers, and the tumor volume was determined according to the equation shown below.

$$\underset{(mm^3)}{\text{Tumor volume}} = \underset{(mm)}{\text{major axis}} \times \underset{(mm)}{\text{minor axis}} \times \underset{(mm)}{\text{minor axis}} \times \frac{1}{2}$$

At the same time, the body weight of each mouse was measured, and the mice were separated into the groups described below, each consisting of five mice, such that the groups had the similar average tumor volume and the similar average body weight. This day was defined as day zero of the administration test, and pharmaceutical administration was started in the following manner.

Compound 9 was suspended at a concentration of 2.5 mg/mL in distilled water containing 0.5% Methyl Cellulose 400cp. The resulting suspension was orally administered to each mouse in a dose of 0.01 mL per gram of the body weight of the mouse (25 mg/kg) daily, twice a day, from days zero to 4 after the start of administration.

Erlotinib was suspended at a concentration of 5 mg/mL in distilled water containing 0.5% Methyl Cellulose 400cp. The resulting suspension was orally administered to each mouse in a dose of 0.01 mL per gram of the body weight of the mouse (50 mg/kg) daily, once a day, from days zero to 4 after the start of administration.

A. Negative control group (Control): non-administration of Compound 9 and erlotinib
B. Compound 9 alone group: 25 mg/kg (twice a day × 5 days)
C. Erlotinib alone group: 50 mg/kg (once a day × 5 days)
D. Compound 9 + erlotinib: 25 mg/kg (twice a day × 5 days) for Compound 9, and 50 mg/kg (once a day × 5 days) for erlotinib

The tumor volume was measured twice a week after day zero. In order to determine the antitumor effect, the average tumor volume was calculated for each group, and the ratios (V/V0) of change in tumor volume were compared, assuming that the tumor volume on day zero was V0. The ratios (V/V0) for the individual groups measured daily are shown in Fig. 6.

As shown in Fig. 6, the combined use of Compound 9 and erlotinib shows a higher growth inhibitory effect than single administration of Compound 9 or erlotinib.

The value (T/C) obtained by dividing the ratio V/V0 of each group by the ratio V/V0 of the negative control group on day 10 is shown in Table 12. In comparison with the theoretical T/C value in the case where the drug efficacy of Compound 9 and the drug efficacy of erlotinib are simply added, i.e., the value obtained by multiplying the T/C value of the Compound 9 alone group by the T/C value of the erlotinib alone group, the T/C in the group of actually combined use (D in Table) is lower (0.44) than 0.63 which is the theoretical value on day 10.

Table 12: T/C of individual groups

| A. Control | B. Compound 9 | C. Erlotinib | D. Compound 9 + Erlotinib | Theoretical value (B × C) |
|---|---|---|---|---|
| 1 | 0.71 | 0.89 | 0.44 | 0.63 |

As is evident from the above, the combined administration of Compound 9 and erlotinib has a higher antitumor effect than the single administration of each of the compounds, and shows a synergistic effect. This result suggests that combined administration of an Flt-3 inhibitor and an epidermal growth factor receptor (EGFR) tyrosine kinase inhibitor has higher antitumor activity than single administration of each of the compounds, and shows a synergistic effect.

<u>Test Example 8: Antitumor effect by combined use of Flt-3 inhibitor and sunitinib in mouse models transplanted with human renal cancer Caki-1 cells</u>

Caki-1 cells were cultured and grown in MEM1640 medium containing 10% fetal calf serum (FCS), 1 mmol/L sodium pyruvate, and 10 mmol/L HEPES in a 5% carbon dioxide incubator at 37°C, and the cultured cells (1 × $10^7$ cells/mouse) were subcutaneously, ventrally transplanted into BALB/cAJcl-nu mice (CLEA Japan). From the mice in which tumors were formed, the tumors were removed. The tumor tissues were cut into small pieces of about 8 $mm^3$, which were subcutaneously, ventrally transplanted, using a trocar needle, into BALB/cAJc1-nu mice (CLEA Japan) for tasting.

Twelve days after the transplantation, the major axis and minor axis of the tumors subcutaneously grown were measured with slide calipers, and the tumor volume was determined according to the equation shown below.

$$\underset{(\text{mm}^3)}{\text{Tumor volume}} = \underset{(\text{mm})}{\text{major axis}} \times \underset{(\text{mm})}{\text{minor axis}} \times \underset{(\text{mm})}{\text{minor axis}} \times \frac{1}{2}$$

At the same time, the body weight of each mouse was measured, and the mice were separated into the groups described below, each consisting of five mice, such that the groups had the similar average tumor volume and the similar average body weight. This day was defined as day zero of the administration test, and pharmaceutical administration was started in the following manner.

Compound 9 was suspended at a concentration of 5.0 mg/mL in distilled water containing 0.5% Methyl Cellulose 400cp. The resulting suspension was orally administered to each mouse in a dose of 0.01 mL per gram of the body weight of the mouse (50 mg/kg) daily, twice a day, from days zero to 4 after the start of administration.

Sunitinib was suspended at a concentration of 4 mg/mL in distilled water containing 0.5% Methyl Cellulose 400cp. The resulting suspension was orally administered to each mouse in a dose of 0.01 mL per gram of the body weight of the mouse (40 mg/kg) daily, twice a day, from days zero to 4 after the start of administration. In the case of combined adminisration of sunitinib and Compound 9, sunitinib was administered from days 7 to 11 after the start of administration.

A. Negative control group (Control): non-administration of Compound 9 and sunitinib
B. Compound 9 alone group: 50 mg/kg (twice a day × 5 days, days zero to 4)
C. Sunitinib alone group: 40 mg/kg (twice a day × 5 days, days zero to 4)
D. Compound 9 + sunitinib: 50 mg/kg (twice a day × 5 days, days zero to 4) for Compound 9, and 40 mg/kg (twice a day × 5 days, days 7 to 11) for sunitinib

The tumor volume was measured twice a week after day zero. In order to determine the antitumor effect, the average tumor volume was calculated for each group, and the ratios (V/V0) of change in tumor volume were compared, assuming that the tumor volume on day zero was V0. The ratios (V/V0) for the individual groups measured daily are shown in Fig. 7.

As shown in Fig. 7, the combined use of Compound 9 and sunitinib shows a higher growth inhibitory effect than single administration of Compound 9 or sunitinib.

The value (T/C) obtained by dividing the ratio V/V0 of each group by the ratio V/V0 of the negative control group on day 18 is shown in Table 13. In comparison with the theoretical T/C value in the case where the drug efficacy of Compound 9 and the drug efficacy of sunitinib are simply added, i.e., the value obtained by multiplying the T/C value of the Compound 9 alone group by the T/C value of the sunitinib alone group, the T/C in the group of actually combined use (D in Table) is lower (0.27) than 0.31 which is the theoretical value on day 18.

Table 13: T/C of individual groups

| A. Control | B. Compound 9 | C. Sunitinib | D. Compound 9 + Sunitinib | Theoretical value (B × C) |
|---|---|---|---|---|
| 1 | 0.50 | 0.62 | 0.27 | 0.31 |

As is evident from the above, the combined administration of Compound 9 and sunitinib has a higher antitumor effect than the single administration of each of the compounds, and shows a synergistic effect. This result suggests that combined administration of an Flt-3 inhibitor and a multi-tyrosine kinase inhibitor has higher antitumor activity than single administration of each of the compounds, and shows a synergistic effect.

<u>Test Example 9: Antitumor effect by combined use of Flt-3 inhibitor and Hsp90 inhibitor in mouse models transplanted with human acute myeloid leukemia MOLM-13 cells</u>

One day before the transplantation of MOLM-13 cells, an anti-asialo GM1 antibody was intraperitoneally injected to Fox C.B-17/Icr-scidJc1 mice (CLEA Japan) in an amount of 0.3 mg per mouse. MOLM-13 cells were cultured and grown in RPMI1640 medium containing 10% fetal calf serum (FCS) in a 5% carbon dioxide incubator at 37°C, and the cultured cells ($1 \times 10^7$ cells/mouse) were subcutaneously, ventrally transplanted into the mice. Five days after the transplantation, the major axis and minor axis of the tumors subcutaneously grown were measured with slide calipers,

and the tumor volume was determined according to the equation shown below.

$$\text{Tumor volume} \ (mm^3) = \text{major axis} \ (mm) \times \text{minor axis} \ (mm) \times \text{minor axis} \ (mm) \times \frac{1}{2}$$

At the same time, the body weight of each mouse was measured, and the mice were separated into the groups described below, each consisting of five mice, such that the groups had the similar average tumor volume and the similar average body weight. This day was defined as day zero of the administration test, and pharmaceutical administration was started in the following manner.

Compound P was dissolved in physiological saline (manufactured by Otsuka Pharmaceutical Co., Ltd.) at a concentration of 1.25 mg/mL. The resulting solution was intravenously administered from the caudal vein to each mouse in a dose of 0.01 mL per gram of the body weight of the mouse (12.5 mg/kg) daily, twice a day, from days zero to 4 after the start of administration.

Compound 9 was suspended at a concentration of 1 mg/kg in distilled water containing 0.5% Methyl Cellulose 400cp. The resulting suspension was orally administered to each mouse in a dose of 0.01 mL per gram of the body weight of the mouse (10 mg/kg) daily, twice a day, from days zero to 4 after the start of administration.

A. Negative control group (Control): non-administration of Compound P and Compound 9
B. Compound P alone group: 12.5 mg/kg (twice a day × 5 days)
C. Compound 9 alone group: 10 mg/kg (twice a day × 5 days)
D. Compound P + Compound 9: 12.5 mg/kg (twice a day × 5 days) for Compound P, and 10 mg/kg (twice a day × 5 days) for Compound 9

The tumor volume was measured twice a week after day zero. In order to determine the antitumor effect, the average tumor volume was calculated for each group, and the ratios (V/V0) of change in tumor volume were compared, assuming that the tumor volume on day zero was V0. The ratios (V/V0) for the individual groups measured daily are shown in Fig. 8.

As shown in Fig. 8, the combined use of Compound P and Compound 9 shows a higher growth inhibitory effect than single administration of Compound P or Compound 9.

The value (T/C) obtained by dividing the ratio V/V0 of each group by the ratio V/V0 of the negative control group on day 7 is shown in Table 14. In comparison with the theoretical T/C value in the case where the drug efficacy of Compound P and the drug efficacy of Compound 9 are simply added, i.e., the value obtained by multiplying the T/C value of the Compound P alone group by the T/C value of the Compound 9 alone group, the T/C in the group of actually combined use (D in Table) is lower (0.066) than 0.15 which is the theoretical value on day 14.

Table 14: T/C of individual groups

| A. Control | B. Compound P | C. Compound 9 | D. Compound P + Compound 9 | Theoretical value (B × C) |
|---|---|---|---|---|
| 1 | 0.53 | 0.29 | 0.066 | 0.15 |

As is evident from the above, the combined administration of Compound P and Compound 9 has a higher antitumor effect than the single administration of each of the compounds, and shows a synergistic effect. This result suggests that combined administration of an Flt-3 inhibitor and an Hsp90 inhibitor has higher antitumor activity than single administration of each of the compounds, and shows a synergistic effect.

<u>Test Example 10: Antitumor effect by combined use of Flt-3 inhibitor and daunorubicin in mouse models transplanted with human acute myeloid leukemia HL-60 cells</u>

One day before the transplantation of HL-60 cells, an anti-asialo GM1 antibody was intraperitoneally injected to Fox C.B-17/Icr-scidJc1 mice (CLEA Japan) in an amount of 0.3 mg per mouse. HL-60 cells were cultured and grown in IMDM medium containing 20% fetal calf serum (FCS) in a 5% carbon dioxide incubator at 37˚C, and the cultured cells ($1 \times 10^7$ cells/mouse) were subcutaneously, ventrally transplanted into the mice. Five days after the transplantation, the major axis and minor axis of the tumors subcutaneously grown were measured with slide calipers, and the tumor volume was determined according to the equation shown below.

$$\text{Tumor volume (mm}^3\text{)} = \text{major axis (mm)} \times \text{minor axis (mm)} \times \text{minor axis (mm)} \times \frac{1}{2}$$

At the same time, the body weight of each mouse was measured, and the mice were separated into the groups described below, each consisting of five mice, such that the groups had the similar average tumor volume and the similar average body weight. This day was defined as day zero of the administration test, and pharmaceutical administration was started in the following manner.

Compound 9 was suspended at a concentration of 2 mg/mL in distilled water containing 0.5% Methyl Cellulose 400cp. The resulting suspension was orally administered to each mouse in a dose of 0.01 mL per gram of the body weight of the mouse (20 mg/kg) daily, twice a day, from days zero to 13 after the start of administration.

Daunorubicin was dissolved in physiological saline (manufactured by Otsuka Pharmaceutical Co., Ltd.) at a concentration of 0.25 mg/mL. The resulting solution was intravenously administered from the caudal vein to each mouse in a dose of 0.01 mL per gram of the body weight of the mouse (2.5 mg/kg) daily, once a day, from days zero to 2 after the start of administration.

A. Negative control group (Control): non-administration of Compound 9 and daunorubicin
B. Compound 9 alone group: 20 mg/kg (twice a day × 14 days)
C. Daunorubicin alone group: 2.5 mg/kg (once a day × 3 days)
D. Compound 9 + daunorubicin: 20 mg/kg (twice a day × 14 days) for Compound 9, and 2.5 mg/kg (once a day × 3 days) for daunorubicin

The tumor volume was measured twice a week after day zero. In order to determine the antitumor effect, the average tumor volume was calculated for each group, and the ratios (V/V0) of change in tumor volume were compared, assuming that the tumor volume on day zero was V0. The ratios (V/V0) for the individual groups measured daily are shown in Fig. 9.

As shown in Fig. 9, the combined use of Compound 9 and daunorubicin shows a higher growth inhibitory effect than single administration of Compound 9 or daunorubicin.

The value (T/C) obtained by dividing the ratio V/V0 of each group by the ratio V/V0 of the negative control group on day 17 is shown in Table 15. In comparison with the theoretical T/C value in the case where the drug efficacy of Compound 9 and the drug efficacy of daunorubicin are simply added, i.e., the value obtained by multiplying the T/C value of the Compound 9 alone group by the T/C value of the daunorubicin alone group, the T/C in the group of actually combined use (D in Table) is lower (0.18) than 0.26 which is the theoretical value on day 17.

Table 15: T/C of individual groups

| A. Control | B. Compound 9 | C. Daunorubicin | D. Compound 9 + Daunorubicin | Theoretical value (B × C) |
|---|---|---|---|---|
| 1 | 0.42 | 0.61 | 0.18 | 0.26 |

As is evident from the above, the combined administration of Compound 9 and daunorubicin has a higher antitumor effect than the single administration of each of the compounds, and shows a synergistic effect. This result suggests that combined administration of an Flt-3 inhibitor and a DNA agonist has higher antitumor activity than single administration of each of the compounds, and shows a synergistic effect.

Test Example 11: Detection of apoptosis reaction using activation of Caspase-3 (DEVDase activity) as index

Measurement of Apoptosis-inducing intensity of test compounds against human acute myeloid leukemia cell line (MOLM-13) was performed by the method described below. As the index of apoptosis, activation reaction of Caspase 3, which is a typical apoptosis marker, was used. The activity of Caspase 3 was determined by measuring the fluorescence intensity of free AMC, generated as a result of hydrolysis (DEVDase) of Ac-DEVD-AMC peptide substrate including the recognition sequence (DEVD) by Caspase 3.

In the culture of MOLM-13 cells, Roswell Park Memorial Institute's Medium (RPMI) 1640 (Gibco Corp.) containing 10% fetal calf serum (FCS) was used. The suspension of MOLM-13 cells ($2.5 \times 10^5$ cells/mL) was seeded in a black flat bottom 96-well plate (Code No. 3916, Corning Inc.) in the amount of 60 μL ($6 \times 10^3$, $3 \times 10^3$, $1 \times 10^4$ cells/well), and preculturing was performed at 37°C under 5% $CO_2$ for 24 hours. A solution containing each test compound prepared by dilution with a medium for culturing cells was added in the amount of 30 μL, thus making the total of 90 μL/

well, and culturing was performed again in the 5% carbon dioxide incubator at 37°C for 8, 12, or 24 hours. Then, 30 μL of 4 × reaction buffer {80 mmol/L piperazine-N,N'-bis(2-ethanesulfonic acid) (PIPES) (Dojindo Laboratories)/NaOH (pH 7.2), 1 vol% Nonidet P40 (NP-40) (Nacalai Tesque), 4 mmol/L ethylene-diamine N,N,N',N'-tetraacetic acid (EDTA) (Dojindo Laboratories), 0.4 w/v% 3-[(3-cholamidopropyl)- dimethylammonio]-1-propanesulfonate (CHAPS) (Dojindo Laboratories), 20 mmol/L dithiothreitol (DTT) (Wako Pure Chemical Industries, Ltd.), 200 μmol/L acetyl-L-aspartyl-L-glutamyl-L-valyl-L-aspartic acid α-(4-methyl-coumaryl-7-amide)(Ac-DEVD-AMC) (Peptide Institute, Inc.)} was added to the culture solution, and mixing was performed for one minute with a MicroMixer (Code No. E-36, Taitec, Saitama), followed by incubation at 37°C under 5% $CO_2$ for 90 minutes. After adding a stop solution (0.8 mol/L acetic acid, Kanto chemical) in the amount of 50 μL to the reaction mixture, mixing was performed for one minute with the MicroMixer, and the reaction was stopped. The fluorescence intensity was measured at an excitation wavelength of 390 nm and at a detection wavelength of 460 nm, and the DEVDase activity (%) was calculated according to the equation shown below. The significance level P in the significant difference test was calculated using statistical analysis software SAS (Release 9.1.3, SAS Institute Inc.) on the basis of Student's t test.

Using the method described above, the DEVDase activity (%) was calculated in the case where Compound 9 or each of the compounds to be combined was used alone, and the DEVDase activity (%) was calculated in the case where both Compound 9 and each of the compounds to be combined were used. The results are shown in Figs. 10 to 16. As the compounds to be combined, SAHA, VPA, 17-AAG, 17-DMAG, decitabine, 5-azacitidine, and fludarabine were used. The incubation time was 8 hours when fludarabine was used, 12 hours when SAHA and VPA were used, and 24 hours when 17-AAG, 17-DMAG, decitabine, and 5-azacitidine were used. In any of the combinations of compounds, the apoptosis-inducing activity was statistically significantly enhanced compared with the case where Compound 9 or the compound to be combined was used alone. As is evident from the results, the effect of any of the compounds to be combined is enhanced by combination with Compound 9.

$$\text{DEVDase activity (\%)} = \left( \frac{F_{compound} - F_{blank}}{F_{vehicle} - F_{blank}} \right) \text{X } 100$$

$F_{compound}$ : Fluorescence intensity in the presence of cells, and in the presence of test compound
$F_{blank}$ : Fluorescence intensity in the absence of cells, and in the absence of test compound
$F_{vehicle}$ : Fluorescence intensity in the presence of cells, and in the absence of test compound

Example 1

Preparation Example 1 (Tablets)

Tablets having the composition shown below are prepared by an ordinary method.

| | |
|---|---|
| Compound 1 | 5 mg |
| Lactose | 60 mg |
| Potato starch | 30 mg |
| Polyvinyl alcohol | 2 mg |
| Magnesium stearate | 1 mg |
| Tar dye | Slight amount |

Example 2

Preparation Example 2 (Tablets)

Tablets having the composition shown below are prepared by an ordinary method.

| | |
|---|---|
| Compound 11 | 5 mg |
| Cytarabine | 10 mg |
| Lactose | 60 mg |
| Potato starch | 30 mg |

(continued)

| | |
|---|---|
| Polyvinyl alcohol | 2 mg |
| Magnesium stearate | 1 mg |
| Tar dye | Slight amount |

Example 3

Preparation Example 3 (Tablets)

Tablets having the composition shown below are prepared by an ordinary method.

| | |
|---|---|
| Compound P | 5 mg |
| Lactose | 60 mg |
| Potato starch | 30 mg |
| Polyvinyl alcohol | 2 mg |
| Magnesium stearate | 1 mg |
| Tar dye | Slight amount |

Example 4

Preparation Example 4 (Injection)

An injection having the composition shown below is prepared by an ordinary method.

| | |
|---|---|
| Compound 17 | 2 mg |
| D-mannitol | 10 mg |
| Aqueous hydrochloric acid solution | Proper amount |
| Aqueous sodium hydroxide solution | Proper amount |
| Distilled water for injection | Proper amount |

Example 5

Preparation Example 5 (Injection)

An injection having the composition shown below is prepared by an ordinary method.

| | |
|---|---|
| Cytarabine | 2 mg |
| D-mannitol | 10 mg |
| Aqueous hydrochloric acid solution | Proper amount |
| Aqueous sodium hydroxide solution | Proper amount |
| Distilled water for injection | Proper amount |

Industrial Applicability

The present invention provides pharmaceutical compositions, each including a combination of an Flt-3 inhibitor and at least one compound, and the like.

**Claims**

1. A pharmaceutical composition comprising a combination of an Fms like tyrosine kinase 3 (Flt-3) inhibitor and at least one compound.

2. A pharmaceutical composition for administering a combination of an Fms like tyrosine kinase 3 (Flt-3) inhibitor and

at least one compound.

3. A pharmaceutical composition for administering an Fms like tyrosine kinase 3 (Flt-3) inhibitor and at least one compound simultaneously or successively.

4. The pharmaceutical composition according to any of Claims 1 to 3, wherein the Fms like tyrosine kinase 3 (Flt-3) inhibitor is an indazole derivative represented by Formula (I)

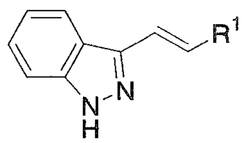

(I)

(wherein $R^1$ represents substituted or unsubstituted aryl or a substituted or unsubstituted heterocyclic group) or a pharmaceutically acceptable salt thereof.

5. The pharmaceutical composition according to any of Claims 1 to 3, wherein the Fms like tyrosine kinase 3 (Flt-3) inhibitor is an indazole derivative represented by Formula (Ia)

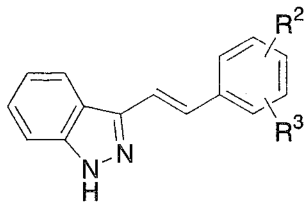

(Ia)

[wherein $R^2$ represents $CONR^{4a}R^{4b}$ (wherein $R^{4a}$ and $R^{4b}$ may be the same or different and each represents a hydrogen atom, substituted or unsubstituted lower alkyl, substituted or unsubstituted aryl, substituted or unsubstituted aralkyl, or a substituted or unsubstituted heterocyclic group, or $R^{4a}$ and $R^{4b}$ are combined together with the adjacent nitrogen atom thereto to form a substituted or unsubstituted heterocyclic group) or $NR^{5a}R^{5b}$ (wherein $R^{5a}$ represents substituted or unsubstituted lower alkylsulfonyl or substituted or unsubstituted arylsulfonyl and $R^{5b}$ represents a hydrogen atom or substituted or unsubstituted lower alkyl) and $R^3$ represents a hydrogen atom, halogen, cyano, nitro, hydroxy, carboxy, lower alkoxycarbonyl, substituted or unsubstituted lower alkyl, substituted or unsubstituted lower alkoxy, substituted or unsubstituted lower alkanoyl, $CONR^{6a}R^{6b}$ (wherein $R^{6a}$ and $R^{6b}$ may be the same or different and each represents a hydrogen atom, substituted or unsubstituted lower alkyl, substituted or unsubstituted aryl, substituted or unsubstituted aralkyl or a substituted or unsubstituted heterocyclic group, or $R^{6a}$ and $R^{6b}$ are combined together with the adjacent nitrogen atom thereto to form a substituted or unsubstituted heterocyclic group) or $NR^{7a}R^{7b}$ (wherein $R^{7a}$ and $R^{7b}$ may be the same or different and each represents a hydrogen atom, substituted or unsubstituted lower alkyl, substituted or unsubstituted lower alkanoyl, substituted or unsubstituted aroyl, substituted or unsubstituted heteroaroyl, substituted or unsubstituted aralkyl, substituted or unsubstituted lower alkylsulfonyl or substituted or unsubstituted arylsulfonyl)] or a pharmaceutically acceptable salt thereof.

6. The pharmaceutical composition according to Claim 5, wherein $R^2$ is $CONR^{4a}R^{4b}$ (wherein $R^{4a}$ and $R^{4b}$ have the same meanings as defined above, respectively) and $R^3$ is a hydrogen atom.

7. The pharmaceutical composition according to Claim 5, wherein $R^2$ is $NR^{5a}R^{5b}$ (wherein $R^{5a}$ and $R^{5b}$ have the same meanings as defined above, respectively) and $R^3$ is substituted or unsubstituted lower alkoxy.

8. The pharmaceutical composition according to Claim 5, wherein $R^2$ is $NR^{5a}R^{5b}$ (wherein $R^{5a}$ and $R^{5b}$ have the same meanings as defined above, respectively) and $R^3$ is a hydrogen atom.

9. The pharmaceutical composition according to any of Claims 1 to 3, wherein the Flt-3 inhibitor is an indazole derivative

represented by Formula (Ib)

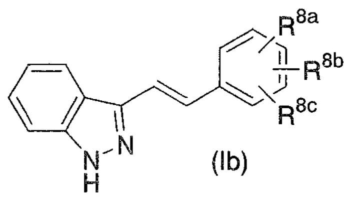

(Ib)

[wherein $R^{8a}$, $R^{8b}$ and $R^{8c}$ may be the same or different and each represents a hydrogen atom, halogen, nitro, nitroso, carboxy, cyano, substituted or unsubstituted lower alkyl, substituted or unsubstituted lower alkanoyl, substituted or unsubstituted lower alkoxycarbonyl, substituted or unsubstituted aryl, $NR^{9a}R^{9b}$ (wherein $R^{9a}$ and $R^{9b}$ may be the same or different and each represents a hydrogen atom, substituted or unsubstituted lower alkyl, substituted or unsubstituted lower alkenyl, substituted or unsubstituted lower alkynyl, substituted or unsubstituted lower alkoxy, substituted or unsubstituted lower alkanoyl, substituted or unsubstituted aryl, substituted or unsubstituted aroyl, a substituted or unsubstituted heterocyclic group or substituted or unsubstituted heteroaroyl, or $R^{9a}$ and $R^{9b}$ are combined together with the adjacent nitrogen atom thereto to form a substituted or unsubstituted heterocyclic group) or $OR^{10}$ (wherein $R^{10}$ represents a hydrogen atom, substituted or unsubstituted lower alkyl, substituted or unsubstituted aryl, substituted or unsubstituted aroyl or a substituted or unsubstituted heterocyclic group)] or a pharmaceutically acceptable salt thereof.

**10.** The pharmaceutical composition according to Claim 9, wherein at least one of $R^{8a}$, $R^{8b}$ and $R^{8c}$ is $NR^{9a}R^{9b}$ (wherein $R^{9a}$ and $R^{9b}$ have the same meanings as defined above, respectively).

**11.** The pharmaceutical composition according to Claim 9, wherein at least one of $R^{8a}$, $R^{8b}$ and $R^{8c}$ is $NR^{9c}R^{9d}$ (wherein $R^{9c}$ and $R^{9d}$ may be the same or different and each represents a hydrogen atom or substituted or unsubstituted lower alkanoyl, or $R^{9c}$ and $R^{9d}$ are combined together with the adjacent nitrogen atom thereto to form a substituted or unsubstituted heterocyclic group).

**12.** The pharmaceutical composition according to Claim 9, wherein at least one of $R^{8a}$, $R^{8b}$ and $R^{8c}$ is $OR^{10}$ (wherein $R^{10}$ has the same meaning as defined above).

**13.** The pharmaceutical composition according to Claim 9, wherein at least one of $R^{8a}$, and $R^{8c}$ is $OR^{10a}$ (wherein $R^{10a}$ represents substituted or unsubstituted lower alkyl).

**14.** The pharmaceutical composition according to any of Claims 1 to 3, wherein the Flt-3 inhibitor is an indazole derivative represented by Formula (Ib-1)

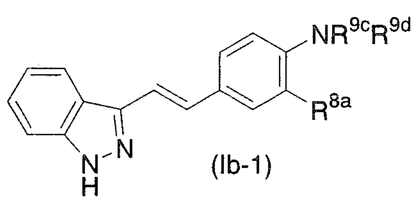

(Ib-1)

(wherein $R^{8a}$, $R^{9c}$ and $R^{9d}$ have the same meanings as defind above, respectively) or a pharmaceutically acceptable salt thereof.

**15.** The pharmaceutical composition according to any of Claims 1 to 3, wherein the Flt-3 inhibitor is an indazole derivative represented by Formula (Ib-2)

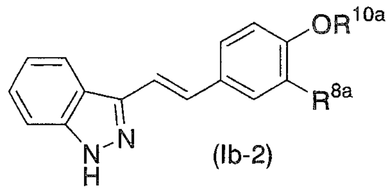

(Ib-2)

(wherein $R^{8a}$ and $R^{10a}$ have the same meanings as defind above, respectively) or a pharmaceutically acceptable salt thereof.

**16.** The pharmaceutical composition according to any of Claims 1 to 3, wherein the Flt-3 inhibitor is an indazole derivative represented by Formula (Ic)

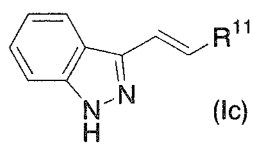

(Ic)

{wherein $R^{11}$ represents a substituted or unsubstituted heterocyclic group [substituent(s) in the substituted heterocyclic group may be the same or different, are 1 to 3 in number, and are oxo, formyl, carboxy, lower alkoxycarbonyl, substituted or unsubstituted lower alkyl), substituted or unsubstituted lower alkoxy, $CONR^{12a}R^{12b}$ (wherein $R^{12a}$ and $R^{12b}$ may be the same or different and each represents a hydrogen atom or substituted or unsubstituted lower alkyl), $NR^{13a}R^{13b}$ (wherein $R^{13a}$ and $R^{13b}$ may be the same or different and each represents a hydrogen atom, lower alkanoyl, lower alkoxycarbonyl, aralkyl, substituted or unsubstituted lower alkyl, substituted or unsubstituted aryl, substituted or unsubstituted aroyl or a substituted or unsubstituted heterocyclic group) or - O $(CR^{14a}R^{14b})_nO$- (wherein $R^{14a}$ and $R^{14b}$ may be the same or different and each represents a hydrogen atom or lower alkyl, n represents 2 or 3, and two terminal oxygen atoms are bonded to the same carbon atom in the heterocyclic group of the substituted heterocyclic group)]} or a pharmaceutically acceptable salt thereof.

**17.** The pharmaceutical composition according to Claim 16, wherein substituents in the substituted heterocyclic group are amino, oxo, lower alkoxy, lower alkoxycarbonylamino, aroylamino or lower alkoxycarbonyl-substituted lower alkyl.

**18.** The pharmaceutical composition according to Claim 16, wherein $R^{11}$ is 3-pyridyl.

**19.** The pharmaceutical composition according to any of Claims 1 to 3, wherein the Fms like tyrosine kinase 3 (Flt-3) inhibitor is a pyrimidine derivative represented by Formula (II)

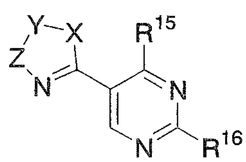

(II)

[wherein -X-Y-Z- represents -O-$CR^{17}$=N- (wherein $R^{17}$ represents a hydrogen atom, hydroxy, carboxy, lower alkyl, lower alkyl substituted with one to four substituents, which may be the same or different and selected from the following substituent group A [substituent group A: halogen, amino, aminosulfonyl, nitro, hydroxy, mercapto, cyano, formyl, carboxy, carbamoyl, lower alkanoyloxy, lower alkanoylamino, mono- or di-(lower alkyl)aminocarbonyl, lower alkoxycarbonyl, mono- or di-(lower alkyl)amino, N-aryl-N-(lower alkyl)amino, lower alkylsulfonyl, lower alkylsulfinyl, mono- or di-(lower alkylsulfonyl)amino, mono-or di-(arylsulfonyl)amino, tri-(lower alkyl)silyl, lower alkylthio, aromatic heterocycle-alkylthio, lower alkanoyl, lower alkanoyl substituted with one to three substituents, which may

be the same or different and selected from the following substituent group a (substituent group a: halogen and hydroxy), lower alkoxy, lower alkoxy substituted with one to three substituents, which may be the same or different and selected from the substituent group a, aryloxy, aryloxy substituted with one to three substituents, which may be the same or different and selected from the substituent group a, aralkyloxy, and aralkyloxy substituted with one to three substituents, which may be the same or different and selected from the substituent group a; wherein, when the substituted lower alkyl is substituted methyl, substituted ethyl, or substituted propyl, the substituent may be $-NR^{18a}R^{18b}$ (wherein $R^{18a}$ and $R^{18b}$ may be the same or different, and each represents a hydrogen atom, substituted or unsubstituted lower alkyl, substituted or unsubstituted cycloalkyl, substituted or unsubstituted lower alkenyl, substituted or unsubstituted lower alkynyl, substituted or unsubstituted aralkyl, substituted or unsubstituted aromatic heterocycle-alkyl, substituted or unsubstituted aliphatic heterocycle-alkyl, substituted or unsubstituted aryl, a substituted or unsubstituted aromatic heterocyclic group, or a substituted or unsubstituted aliphatic heterocyclic group)], lower cycloalkyl, lower cycloalkyl substituted with one to four substituents, which may be the same or different and selected from the above substituent group A, substituted or unsubstituted lower alkenyl, substituted or unsubstituted lower alkynyl, substituted or unsubstituted aralkyl, substituted or unsubstituted aromatic heterocycle-alkyl, substituted or unsubstituted aliphatic heterocycle-alkyl, substituted or unsubstituted aryl, a substituted or unsubstituted aromatic heterocyclic group, a substituted or unsubstituted aliphatic heterocyclic group, substituted or unsubstituted lower alkoxy, substituted or unsubstituted lower alkoxycarbonyl, substituted or unsubstituted lower alkylthio, substituted or unsubstituted lower alkanoyl, or $-C(=O)NR^{19a}R^{19b}$ (wherein $R^{19a}$ and $R^{19b}$ may be the same or different, and each represents a hydrogen atom, substituted or unsubstituted lower alkyl, substituted or unsubstituted lower cycloalkyl, substituted or unsubstituted lower alkenyl, substituted or unsubstituted lower alkynyl, substituted or unsubstituted aralkyl, substituted or unsubstituted aromatic heterocycle-alkyl, substituted or unsubstituted aliphatic heterocycle-alkyl, substituted or unsubstituted aryl, a substituted or unsubstituted aromatic heterocyclic group, or a substituted or unsubstituted aliphatic heterocyclic group, or $R^{19a}$ and $R^{19b}$ are combined together with the adjacent nitrogen atom thereto to form a substituted or unsubstituted aliphatic heterocyclic group)}, $-N=CR^{17a}-O-$ (wherein $R^{17a}$ has the same meaning as $R^{17}$ defined above), $-O-N=CR^{17b}-$(wherein $R^{17b}$ has the same meaning as $R^{17}$ defined above), $-O-C(=O)-NR^{20}-$ (wherein $R^{20}$ represents a hydrogen atom, lower alkyl, lower alkyl substituted with one to four substituents, which may be the same or different and selected from the above substituent group A, lower cycloalkyl, lower cycloalkyl substituted with one to four substituents, which may be the same or different and selected from the above substituent group A, or substituted or unsubstituted aliphatic heterocycle-alkyl), $-N=N-NR^{21}-$ (wherein $R^{21}$ represents substituted or unsubstituted lower alkyl, substituted or unsubstituted lower cycloalkyl or substituted or unsubstituted aliphatic heterocycle-alkyl), or $-NR^{21a}-N=N-$ (wherein $R^{21a}$ has the same definition as $R^{21}$ described above);

$R^{15}$ represents $-NR^{22a}R^{22b}$ (wherein $R^{22a}$ and $R^{22b}$ may be the same or different, and each represents a hydrogen atom, substituted or unsubstituted lower alkyl, substituted or unsubstituted lower cycloalkyl, substituted or unsubstituted lower alkenyl, substituted or unsubstituted lower alkynyl, substituted or unsubstituted aralkyl, substituted or unsubstituted aromatic heterocycle-alkyl, substituted or unsubstituted aliphatic heterocycle-alkyl, substituted or unsubstituted monocyclic aryl, a substituted or unsubstituted aromatic monoheterocyclic group, or a substituted or unsubstituted aliphatic heterocyclic group, or $R^{22a}$ and $R^{22b}$ are combined together with the adjacent nitrogen atom thereto to form a substituted or unsubstituted aliphatic heterocyclic group; wherein, when one of $R^{22a}$ and $R^{22b}$ is a hydrogen atom, the other of $R^{22a}$ and $R^{22b}$ is not a group selected from substituted or unsubstituted pyrazol-3-yl and substituted or unsubstituted 1,2,4-triazol-3-yl), or $-OR^{23}$ (wherein $R^{23}$ represents a hydrogen atom, substituted or unsubstituted lower alkyl, substituted or unsubstituted lower cycloalkyl, substituted or unsubstituted lower alkenyl, substituted or unsubstituted lower alkynyl, substituted or unsubstituted aralkyl, substituted or unsubstituted aromatic heterocycle-alkyl, substituted or unsubstituted aliphatic heterocycle-alkyl, substituted or unsubstituted aryl, a substituted or unsubstituted aromatic heterocyclic group, or a substituted or unsubstituted aliphatic heterocyclic group); and

$R^{16}$ represents $-NR^{24a}R^{24b}$ {wherein $R^{24a}$ and $R^{24b}$ may be the same or different, and each represents a hydrogen atom, lower alkyl, lower alkyl substituted with one to four substituents, which may be the same or different and selected from the following substituent group B [substituent group B: halogen, amino, aminosulfonyl, nitro, hydroxy, mercapto, cyano, formyl, carboxy, carbamoyl, lower alkanoyloxy, lower alkanoylamino, mono- or di-(lower alkyl) aminocarbonyl, lower alkoxycarbonyl, mono- or di-(lower alkyl)amino, N-aryl-N-(lower alkyl)amino, lower alkylsulfonyl, lower alkylsulfinyl, mono- or di-(lower alkylsulfonyl)amino, mono-or di-(arylsulfonyl)amino, tri-(lower alkyl) silyl, lower alkylthio, aromatic heterocycle-alkylthio, lower alkanoyl, lower alkanoyl substituted with one to three substituents, which may be the same or different and selected from the substituent group a, lower alkoxy, lower alkoxy substituted with one to three substituents, which may be the same or different and selected from the above substituent group a, aryloxy, aryloxy substituted with one to three substituents, which may be the same or different and selected from the above substituent group a, aralkyloxy, and aralkyloxy substituted with one to three substituents, which may be the same or different and selected from the above substituent group a], lower cycloalkyl,

lower cycloalkyl substituted with one to four substituents, which may be the same or different and selected from the above substituent group B, substituted or unsubstituted lower alkenyl, substituted or unsubstituted lower alkynyl, substituted or unsubstituted aliphatic heterocycle-alkyl, substituted or unsubstituted monocyclic aryl, or a substituted or unsubstituted aliphatic heterocyclic group, or $R^{24a}$ and $R^{24b}$ are combined together with the adjacent nitrogen atom thereto to form a substituted or unsubstituted aliphatic heterocyclic group or a substituted or unsubstituted aromatic heterocyclic group; wherein $R^{24a}$ and $R^{24b}$ do not simultaneously represent a hydrogen atom}, -$NR^{25}CR^{26a}R^{26b}$-Ar {wherein $R^{25}$ represents a hydrogen atom, lower alkyl or lower cycloalkyl, $R^{26a}$ and $R^{26b}$ may be the same or different, and each represents a hydrogen atom, lower alkyl, lower alkyl substituted with one to three substituents, which may be the same or different and selected from the following substituent group b (substituent group b: halogen, hydroxy and hydroxymethyl), lower cycloalkyl, or lower cycloalkyl substituted with one to three substituents, which may be the same or different and selected from the above substituent group b, and Ar represents aryl, aryl substituted with one to three substituents, which may be the same or different and selected from the following substituent group C [substituent group C: halogen, amino, nitro, hydroxy, mercapto, cyano, carboxy, aminosulfonyl, lower alkyl, lower alkyl substituted with one to three substituents, which may be the same or different and selected from the above substituent group b, lower cycloalkyl, lower cycloalkyl substituted with one to three substituents, which may be the same or different and selected from the above substituent group b, lower alkoxy, lower slkylthio, mono- or di-(lower alkyl)amino, lower alkanoylamino, mono- or di-(lower alkylsulfonyl) amino, lower alkoxycarbonylamino, aliphatic heterocycle-alkyloxy and alkylenedioxy], an aromatic heterocyclic group or an aromatic heterocyclic group substituted with one to three substituents, which may be the same or different and selected from the above substituent group C} or -$NR^{25}CR^{26a}R^{26b}CR^{27a}R^{27b}$-Ar (wherein $R^{25}$, $R^{26a}$, $R^{26b}$ and Ar have the same meanings as defined above, respectively, and $R^{27a}$ and $R^{27b}$ have the same meanings as $R^{26a}$ and $R^{26b}$ defined above, respectively)] or a pharmaceutically acceptable salt thereof.

**20.** The pharmaceutical composition according to any of Claims 1 to 3, wherein the Fms like tyrosine kinase 3 (Flt-3) inhibitor is a nitrogen-containing heterocyclic compound represented by Formula (III)

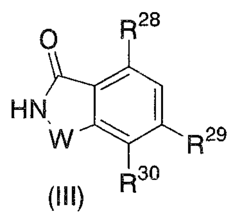

(III)

[wherein W represents -C(=O)- or -$CHR^{31}$- (wherein $R^{31}$ represents a hydrogen atom, hydroxy, substituted or unsubstituted lower alkyl, or substituted or unsubstituted lower alkoxy), $R^{28}$ represents

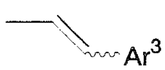

{wherein $Ar^3$ represents aryl, aryl substituted with one or two substituents, which may be the same or different and selected from the following substituent group D, an aromatic monoheterocyclic group, an aromatic monoheterocyclic group substituted with one or two substituents, which may be the same or different and selected from the following substituent group D [substituent group D: halogen, nitro, hydroxy, cyano, carboxy, lower alkoxycarbonyl, substituted or unsubstituted lower alkyl, substituted or unsubstituted lower alkoxy, substituted or unsubstituted lower alkanoyl, -$CONR^{32a}R^{32b}$ (wherein $R^{32a}$ and $R^{32b}$ may be the same or different, and each represents a hydrogen atom, substituted or unsubstituted lower alkyl, substituted or unsubstituted aryl, or substituted or unsubstituted aralkyl, or $R^{32a}$ and $R^{32b}$ are combined together with the adjacent nitrogen atom thereto to form a substituted or unsubstituted heterocyclic group) and -$NR^{33a}R^{33b}$ (wherein $R^{33a}$ and $R^{33b}$ may be the same or different, and each represents a hydrogen atom, substituted or unsubstituted lower alkyl, substituted or unsubstituted lower alkanoyl, substituted or unsubstituted lower alkylsulfonyl, substituted or unsubstituted aryl, substituted or unsubstituted aralkyl, substituted or unsubstituted aroyl, substituted or unsubstituted arylsulfonyl, or substituted or unsubstituted heteroaroyl)]}, $R^{29}$ represents a hydrogen atom or

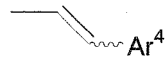

(wherein $Ar^4$ has the same meaning as $Ar^3$ defined above), and $R^{30}$ represents a hydrogen atom, halogen, substituted or unsubstituted lower alkyl, substituted or unsubstituted lower alkanoyl, substituted or unsubstituted aryl, $-NR^{34a}R^{34b}$ [wherein $R^{34a}$ and $R^{34b}$ may be the same or different, and each represents a hydrogen atom, substituted or unsubstituted lower alkyl, or - $C(=O)R^{35}$ (wherein $R^{35}$ represents a hydrogen atom, substituted or unsubstituted lower alkyl, substituted or unsubstituted lower alkoxy, or substituted or unsubstituted aralkyl)] or

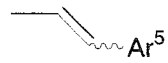

(wherein $Ar^5$ has the same meaning as $Ar^3$ defined above); provided that, when $R^{29}$ is a hydrogen atom and $Ar^3$ is aryl, aryl substituted with two lower alkoxy, or aryl substituted with one lower alkyl or one lower alkoxy only, $R^{30}$ is not a hydrogen atom] or a phrmaceutically acceptable salt thereof.

**21.** The pharmaceutical composition according to any of Claims 1 to 20, wherein the target disease is cancer.

**22.** The pharmaceutical composition according to Claim 21, wherein the cancer is cancer derived from hematopoietic tumor, breast cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, head and neck cancer, osteosarcoma, melanoma, or cancer derived from brain tumor.

**23.** The pharmaceutical composition according to Claim 21, wherein the cancer is leukemia, myeloma or lymphoma.

**24.** The pharmaceutical composition according to Claim 21, wherein the cancer is acute myeloid leukemia.

**25.** The pharmaceutical composition according to Claim 21, wherein the cancer is solid cancer.

**26.** The pharmaceutical composition according to Claim 21, wherein the cancer is pancreatic cancer.

**27.** The pharmaceutical composition according to Claim 21, wherein the cancer is renal cancer.

**28.** The pharmaceutical composition according to Claim 21, wherein the cancer is colon cancer.

**29.** The pharmaceutical composition according to any of Claims 3 to 28, wherein the compound to be administered in combination, simultaneously or successively, with the Flt-3 inhibitor is a protein or a low molecular compound.

**30.** The pharmaceutical composition according to Claim 29, wherein the compound to be combined with the Flt-3 inhibitor is a protein and the protein is an antibody.

**31.** The pharmaceutical composition according to Claim 30, wherein the antibody is Cetuximab.

**32.** The pharmaceutical composition according to Claim 29, wherein the compound to be combined with the Flt-3 inhibitor is a low molecular compound and the low molecular compound is a chemotherapeutic agent or a molecular targeted drug.

**33.** The pharmaceutical composition according to Claim 32, wherein the low molecular compound is a chemotherapeutic agent and the chemotherapeutic agent is selected from Cytarabine, Daunorubicin, Idarubicin, Vincristine, Etoposide, Vindesine, Doxorubicin, Mitoxantrone, Fluorouracil, Irinotecan and Gemcitabine.

**34.** The pharmaceutical composition according to Claim 32, wherein the low molecular compound is a molecular targeted drug and the molecular targeted drug is a farnesyltransferase inhibitor.

**35.** The pharmaceutical composition according to Claim 34, wherein the farnesyltransferase inhibitor is Zanestra.

**36.** The pharmaceutical composition according to Claim 32, wherein the low molecular compound is a molecular targeted drug and the molecular targeted drug is a proteasome inhibitor.

**37.** The pharmaceutical composition according to Claim 36, wherein the proteasome inhibitor is Bortezomib.

**38.** The pharmaceutical composition according to Claim 32, wherein the low molecular compound is a molecular targeted drug and the molecular targeted drug is a kinase inhibitor.

**39.** The pharmaceutical composition according to Claim 38, wherein the kinase inhibitor is Erlotinib.

**40.** The pharmaceutical composition according to Claim 32, wherein the low molecular compound is a molecular targeted drug and the molecular targeted drug is a heat shock protein 90 (Hsp90) inhibitor.

**41.** A method of treating cancer, which comprises the step of administering an Fms like tyrosine kinase 3 (Flt-3) inhibitor and at least one compound simultaneously or separately with an interval.

**42.** The method of treating cancer according to Claim 41, wherein the Fms like tyrosine kinase 3 (Flt-3) inhibitor is an indazole derivative represented by Formula (I)

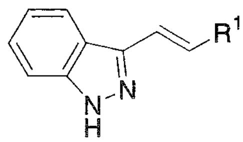

(I)

(wherein $R^1$ has the same meaning as defined above) or a pharmaceutically acceptable salt thereof.

**43.** The method of treating cancer according to Claim 41, wherein the Fms like tyrosine kinase 3 (Flt-3) inhibitor is an indazole derivative represented by Formula (Ia)

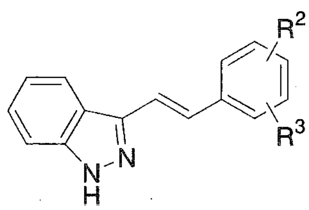

(Ia)

(wherein $R^2$ and $R^3$ have the same meanings as defined above, respectively) or a pharmaceutically acceptable salt thereof.

**44.** The method of treating cancer according to Claim 41, wherein the Fms like tyrosine kinase 3 (Flt-3) inhibitor is an indazole derivative represented by Formula (Ib)

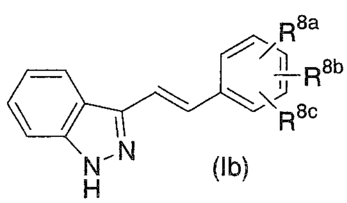

(Ib)

(wherein $R^{8a}$, $R^{8b}$ and $R^{8c}$ have the same meanings as defined above, respectively) or a pharmaceutically acceptable salt thereof.

**45.** The method of treating cancer according to Claim 41, wherein the Fms like tyrosine kinase 3 (Flt-3) inhibitor is an indazole derivative represented by Formula (Ib-1)

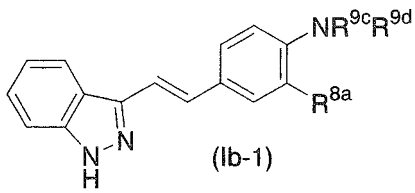

(Ib-1)

(wherein $R^{8a}$, $R^{9c}$ and $R^{9d}$ have the same meanings as defined above, respectively) or a pharmaceutically acceptable salt thereof.

**46.** The method of treating cancer according to Claim 41, wherein the Fms like tyrosine kinase 3 (Flt-3) inhibitor is an indazole derivative represented by Formula (Ib-2)

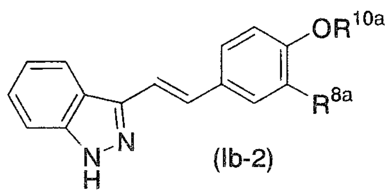

(Ib-2)

(wherein $R^{8a}$ and $R^{10a}$ have the same meanings as defined above, respectively) or a pharmaceutically acceptable salt thereof.

**47.** The method of treating cancer according to Claim 41, wherein the Fms like tyrosine kinase 3 (Flt-3) inhibitor is an indazole derivative represented by Formula (Ic)

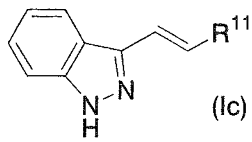

(Ic)

(wherein $R^{11}$ has the same meanings as defined above) or a pharmaceutically acceptable salt thereof.

**48.** The method of treating cancer according to Claim 41, wherein the Fms like tyrosine kinase 3 (Flt-3) inhibitor is a pyrimidine derivative represented by Formula (II)

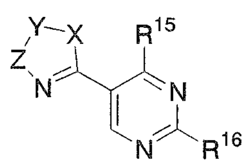

(II)

(wherein -X-Y-Z-, $R^{15}$ and $R^{16}$ have the same meanings as defined above, respectively) or a pharmaceutically acceptable salt thereof.

**49.** The method of treating cancer according to Claim 41, wherein the Fms like tyrosine kinase 3 (Flt-3) inhibitor is a nitrogen-containing heterocyclic compound represented by Formula (III)

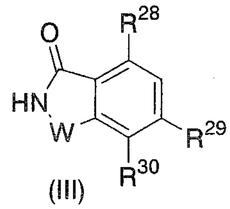

(III)

(wherein W, $R^{28}$, $R^{29}$ and $R^{30}$ have the same meanings as defined above, respectively) or a pharmaceutically acceptable salt thereof.

**50.** Use of an Fms like tyrosine kinase 3 (Flt-3) inhibitor and at least one compound for the manufacture of an anticancer agent.

**51.** The use according to Claim 50, wherein the Fms like tyrosine kinase 3 (Flt-3) inhibitor is an indazole derivative represented by formula (I)

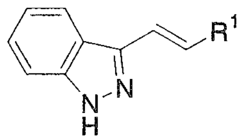

(I)

(wherein $R^1$ has the same meaning as defined above) or a pharmaceutically acceptable salt thereof.

**52.** The use according to Claim 50, wherein the Fms like tyrosine kinase 3 (Flt-3) inhibitor is an indazole derivative represented by Formula (Ia)

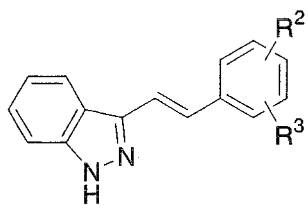

(Ia)

(wherein $R^2$ and $R^3$ have the same meanings as defined above, respectively) or a pharmaceutically acceptable salt thereof.

**53.** The use according to Claim 50, wherein the Fms like tyrosine kinase 3 (Flt-3) inhibitor is an indazole derivative represented by Formula (Ib)

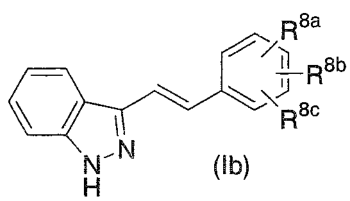

(Ib)

(wherein $R^{8a}$, $R^{8b}$ and $R^{8c}$ have the same meanings as defined above, respectively) or a pharmaceutically acceptable salt thereof.

**54.** The use according to Claim 50, wherein the Fms like tyrosine kinase 3 (Flt-3) inhibitor is an indazole derivative

represented by Formula (Ib-1)

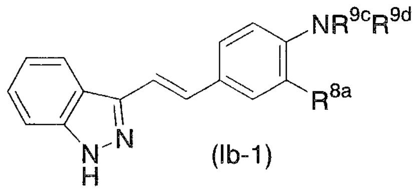

(Ib-1)

(wherein, $R^{8a}$, $R^{9c}$ and $R^{9d}$ have the same meanings as defined above, respectively) or a pharmaceutically acceptable salt thereof.

**55.** The use according to Claim 50, wherein the Fms like tyrosine kinase 3 (Flt-3) inhibitor is an indazole derivative represented by Formula (Ib-2)

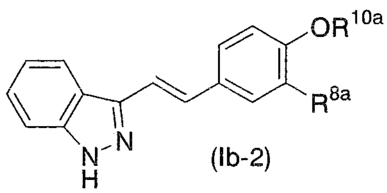

(Ib-2)

(wherein $R^{8a}$ and $R^{10a}$ have the same meanings as defined above, respectively) or a pharmaceutically acceptable salt thereof.

**56.** The use according to Claim 50, wherein the Fms like tyrosine kinase 3 (Flt-3) inhibitor is an indazole derivative represented by Formula (Ic)

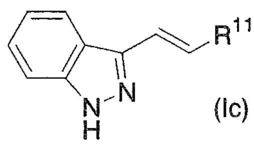

(Ic)

(wherein $R^{11}$ has the same meanings as defined above) or a pharmaceutically acceptable salt thereof.

**57.** The use according to Claim 50, wherein the Fms like tyrosine kinase 3 (Flt-3) inhibitor is a pyrimidine derivative represented by Formula (II)

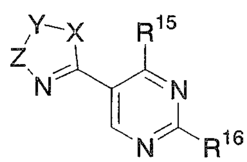

(II)

(wherein -X-Y-Z-, $R^{15}$ and $R^{16}$ have the same meanings as defined above, respectively) or a pharmaceutically acceptable salt thereof.

**58.** The use according to Claim 50, wherein the Fms like tyrosine kinase 3 (Flt-3) inhibitor is a nitrogen-containing heterocyclic compound represented by Formula (III)

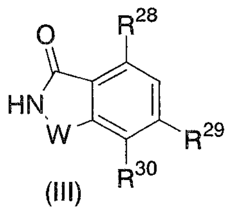

(III)

(wherein W, $R^{28}$, $R^{29}$ and $R^{30}$ have the same meanings as defined above, respectively) or a pharmaceutically acceptable salt thereof.

**59.** A kit which comprises a first component comprising an Fms like tyrosine kinase 3 (Flt-3) inhibitor and a second component comprising an antitumor agent.

**60.** The kit according to Claim 59, wherein the Fms like tyrosine kinase 3 (Flt-3) inhibitor is the indazole derivative described in any of Claims 4 to 18 or a pharmaceutically acceptable salt thereof.

**61.** The kit according to Claim 59, wherein the Fms like tyrosine kinase 3 (Flt-3) inhibitor is the pyrimidine derivative described in Claim 19 or a pharmaceutically acceptable salt thereof.

**62.** The kit according to Claim 59, wherein the Fms like tyrosine kinase 3 (Flt-3) inhibitor is the nitrogen-containing heterocyclic compound described in Claim 20 or a pharmaceutically acceptable salt thereof.

**63.** An antitumor agent for administering an Fms like tyrosine kinase 3 (Flt-3) inhibitor and at least one compound as active ingredients simultaneously or successively.

**64.** The antitumor agent according to Claim 63, wherein the Fms like tyrosine kinase 3 (Flt-3) inhibitor is the indazole derivative described in any of Claims 4 to 18 or a pharmaceutically acceptable salt thereof.

**65.** The antitumor agent according to Claim 63, wherein the Fms like tyrosine kinase 3 (Flt-3) inhibitor is the pyrimidine derivative described in Claim 19 or a pharmaceutically acceptable salt thereof.

**66.** The antitumor agent according to Claim 63, wherein the Fms like tyrosine kinase 3 (Flt-3) inhibitor is the nitrogen-containing heterocyclic compound described in Claim 20 or a pharmaceutically acceptable salt thereof.

Figure 1

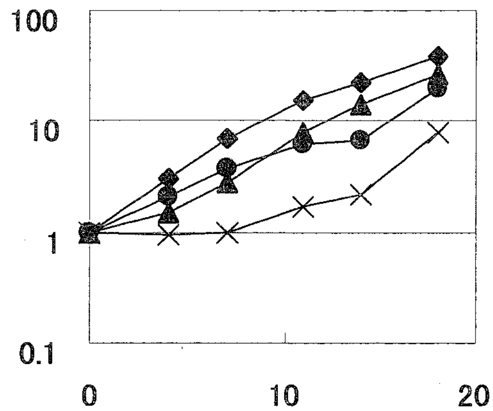


Figure 2

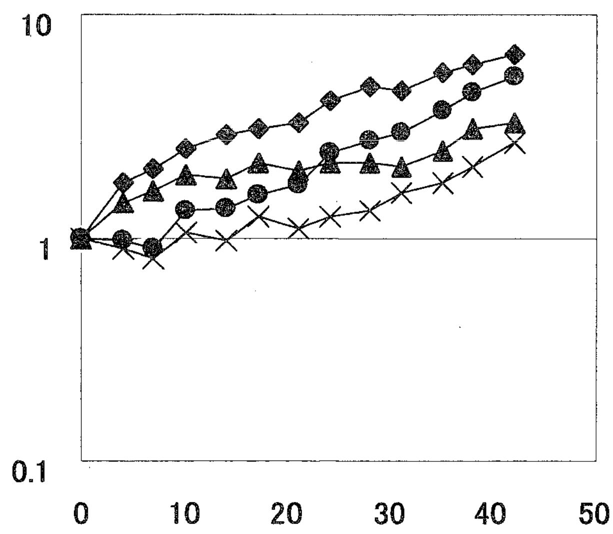

Figure 3

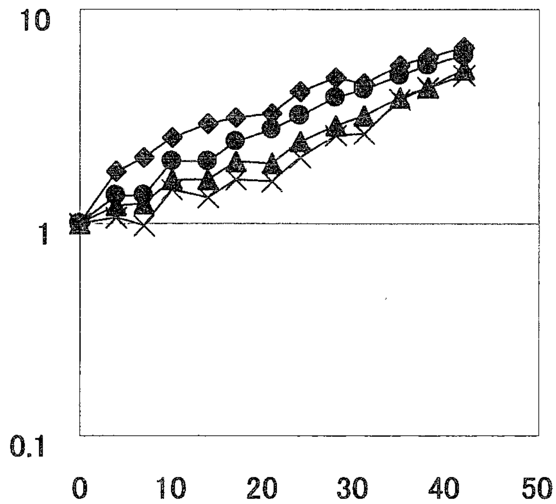


Figure 4

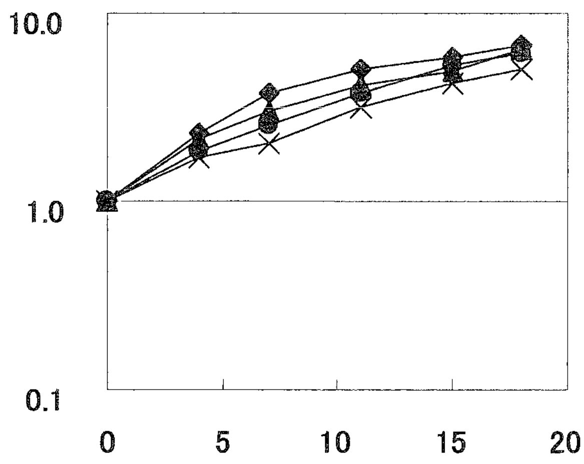

Figure 5

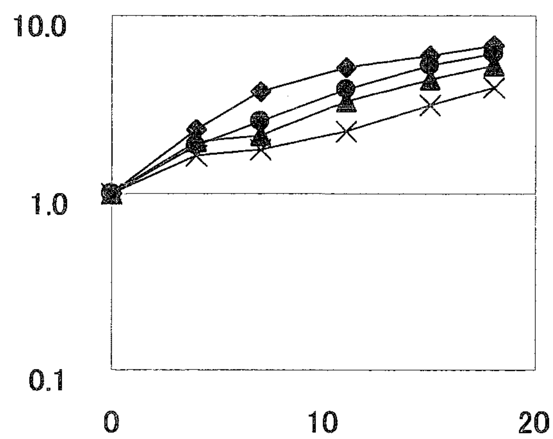


Figure 6

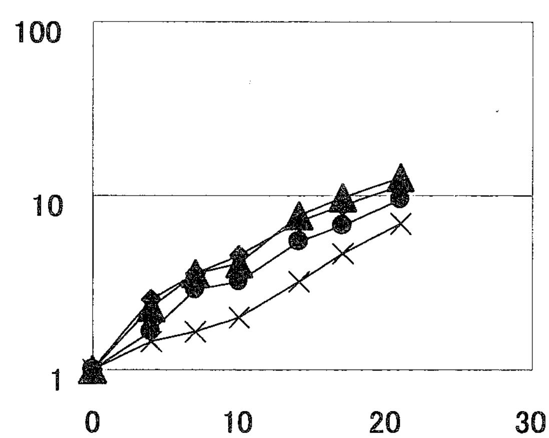

Figure 7

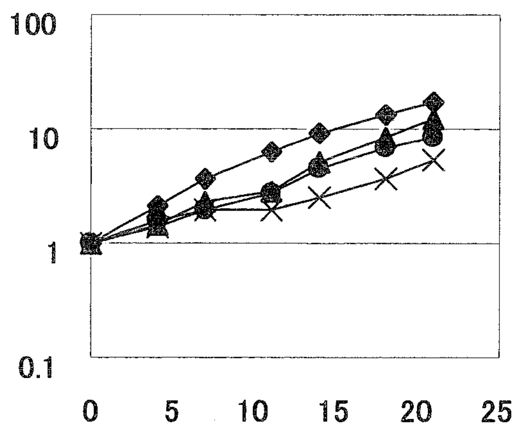


Figure 8

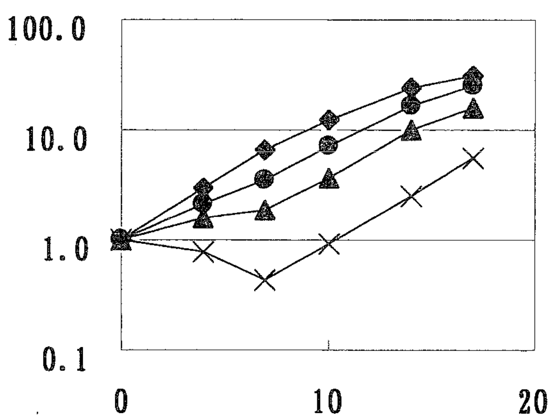

Figure 9

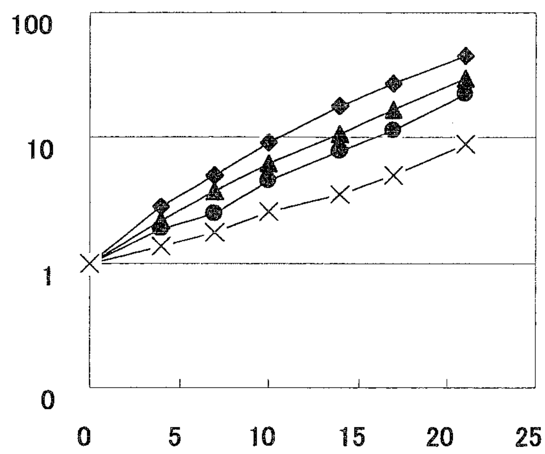


Figure 10

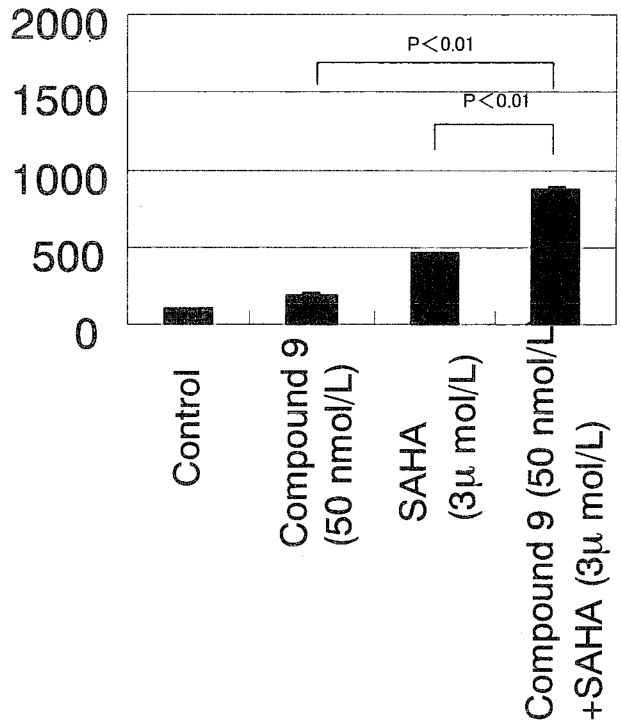

Figure 11

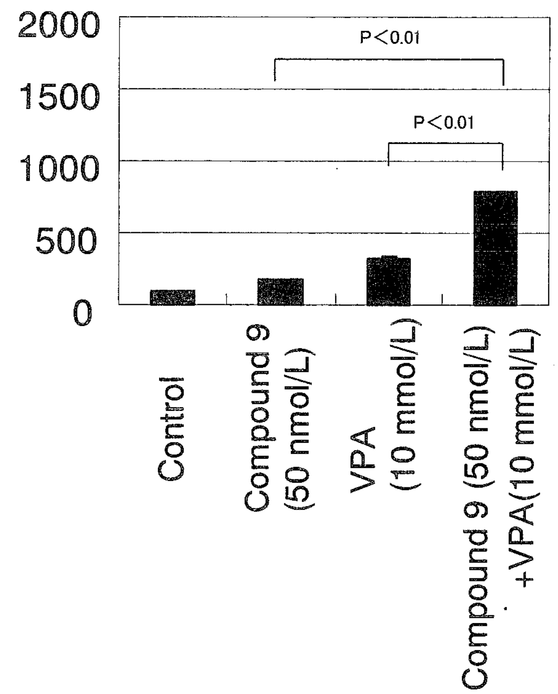


Figure 12

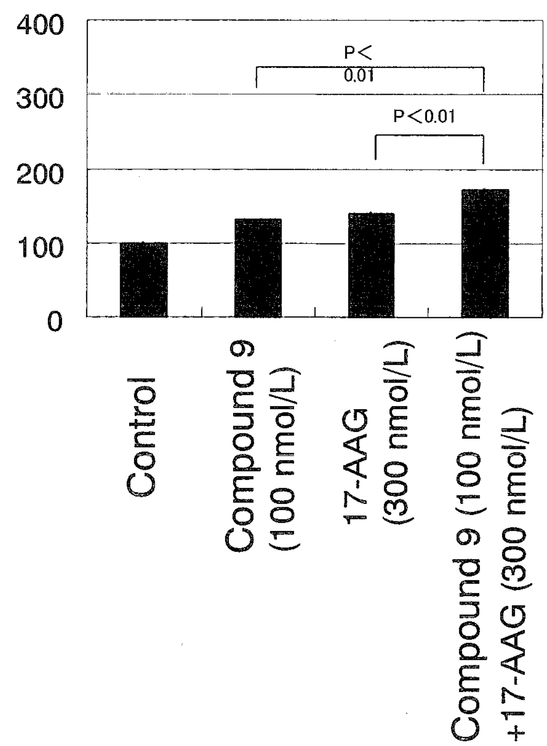

Figure 13

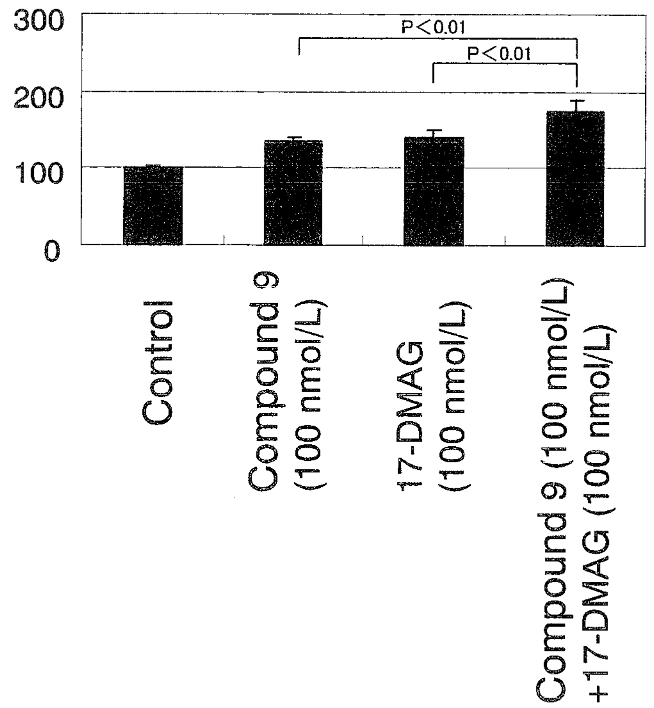


Figure 14

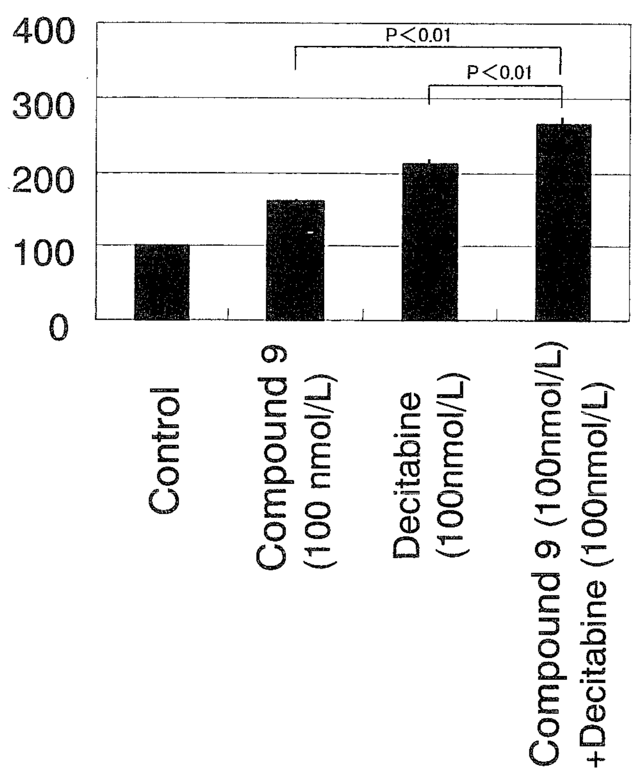

Figure 15

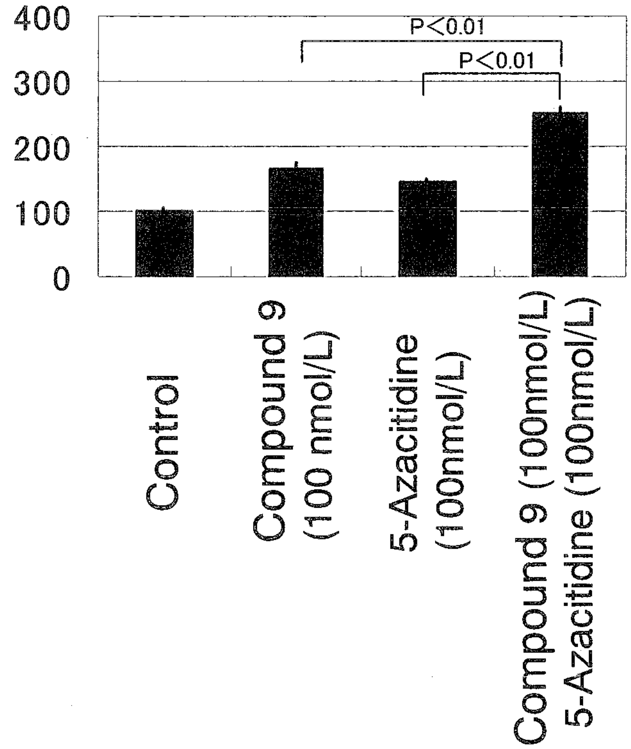


Figure 16

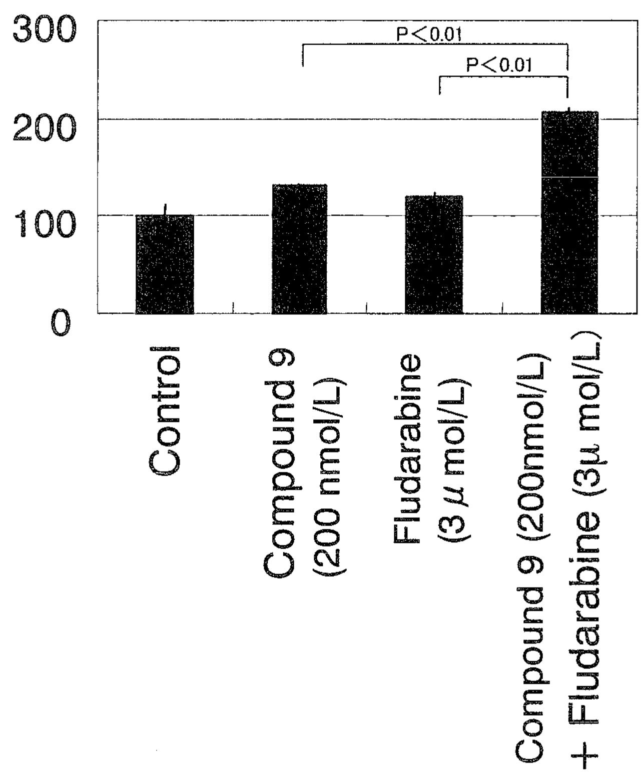

## INTERNATIONAL SEARCH REPORT

International application No.
PCT/JP2008/053909

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
A61K45/06, A61K31/136, A61K31/198, A61K31/416, A61K31/436, A61K31/4439, A61K31/454, A61K31/4745, A61K31/475, A61K31/496, A61K31/506, A61K31/513, A61K31/5377, A61K31/69, A61K31/704, A61K31/7048, A61K31/7068, A61K38/00,

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
BIOSIS(STN), CAplus(STN), EMBASE(STN), MEDLINE(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br><br>Y | ABRAMS, T.J. et al, Preclinical evaluation of the tyrosine kinase inhibitor SU11248 as a single agent and in combination with "standard of care" therapeutic agents for the treatment of breast cancer, Mol Cancer Ther, 2003, Vol.2, No.10, p.1011-21, full text, particularly, Abstract | 1-3,21-29, 32,33,50,59, 63<br>1-18,21-40, 50-56,59,60, 63,64 |
| X<br><br>Y | LEVIS, M. et al, In vitro studies of a FLT3 inhibitor combined with chemotherapy: sequence of administration is important to achieve synergistic cytotoxic effects, Blood, 2004, Vol.104, No.4, p.1145-50, full text, particularly, Abstract | 1-3,21-29, 32,33,50,59, 63<br>1-18,21-40, 50-56,59,60, 63,64 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 June, 2008 (04.06.08) | 17 June, 2008 (17.06.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2008/053909

C (Continuation). DOCUMENTS CONSIDERED TO BE RELEVANT

| Category* | Citation of document, with indication, where appropriate, of the relevant passages | Relevant to claim No. |
|---|---|---|
| X<br><br><br>Y | ALESKOG, A. et al, In vitro activity of the flt3-inhibitor su5614 and standard cytotoxic agents in tumour cells from patients with wild type and mutated flt3 acute myeloid leukaemia, Leuk Res, 2005, Vol.29, No.9, p.1079-81, full text, particularly, Abstract | 1-3,21-29,<br>32,33,50,59,<br>63<br>1-18,21-40,<br>50-56,59,60,<br>63,64 |
| X<br><br><br>Y | WO 2006/135629 A1 (JANSSEN PHARM NV, BE),<br>21 December, 2006 (21.12.06),<br>Full text<br>& US 2006/281788 A1 & WO 2006/138155 A1 | 1-3,21-29,<br>32,34,50,59,<br>63<br>1-18,21-40,<br>50-56,59,60,<br>63,64 |
| X<br><br><br>Y | GEORGE, P. et al, Cotreatment with 17-allylamino-demethoxygeldanamycin and FLT-3 kinase inhibitor PKC412 is highly effective against human acute myelogenous leukemia cells with mutant FLT-3, Cancer Res, 2004, Vol.64, No.10, p.3645-52, full text, particularly, Abstract | 1-3,21-29,<br>32,40,50,59,<br>63<br>1-18,21-40,<br>50-56,59,60,<br>63,64 |
| X<br><br>Y | MOHI, M.G. et al, Combination of rapamycin and protein tyrosine kinase (PTK) inhibitors for the treatment of leukemias caused by oncogenic PTKs, Proc Natl Acad Sci U S A, 2004, Vol.101, No.9, p.3130-5, full text, particularly, Abstract | 1-3,21-29,<br>32,50,59,63<br>1-18,21-40,<br>50-56,59,60,<br>63,64 |
| X<br><br>Y | WO 2007/010013 A2 (NOVARTIS AG, CH),<br>25 January, 2007 (25.01.07),<br>Full text<br>(Family: none) | 1-3,21-29,<br>32,50,59,63<br>1-18,21-40,<br>50-56,59,60,<br>63,64 |
| X<br><br>Y | BAGRINTSEVA, K. et al, FLT3-ITD-TKD dual mutants associated with AML confer resistance to FLT3 PTK inhibitors and cytotoxic agents by overexpression of Bcl-x(L), Blood, 2005, Vol.105, No.9, p.3679-85, full text, particularly, Abstract | 1-3,21-29,<br>32,50,59,63<br>1-18,21-40,<br>50-56,59,60,<br>63,64 |
| X<br><br>Y | BALI, P. et al, Superior activity of the combination of histone deacetylase inhibitor LAQ824 and the FLT-3 kinase inhibitor PKC412 against human acute myelogenous leukemia cells with mutant FLT-3, Clin Cancer Res, 2004, Vol.10, No.15, p.4991-7, full text, particularly, Abstract | 1-3,21-29,<br>32,50,59,63<br>1-18,21-40,<br>50-56,59,60,<br>63,64 |

Form PCT/ISA/210 (continuation of second sheet) (April 2007)

**INTERNATIONAL SEARCH REPORT**

International application No.
PCT/JP2008/053909

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2005/012258 A1 (KYOWA HAKKO KOGYO KABUSHIKI KAISHA), 10 February, 2005 (10.02.05), Full text & EP 1650194 A1 & US 2006/281789 A1 | 1-18,21-40, 50-56,59,60, 63,64 |
| Y | WO 2005/012257 A1 (KYOWA HAKKO KOGYO KABUSHIKI KAISHA), 10 February, 2005 (10.02.05), Full text & EP 1652842 A1 & US 2007/117856 A1 | 1-18,21-40, 50-56,59,60, 63,64 |
| Y | ROSS, J.S. et al, Targeted therapies for cancer 2004, Am J Clin Pathol, 2004, Vol.122, No.4, p.598-609, full text, particularly, Abstract | 1-18,21-31, 50-56,59,60, 63,64 |
| Y | YANAMANDRA, N. et al, Tipifarnib and bortezomib are synergistic and overcome cell adhesion-mediated drug resistance in multiple myeloma and acute myeloid leukemia, Clin Cancer Res, 2006, Vol.12, No.2, p.591-9, full text, particularly, Abstract | 1-18,21-29, 32,36,37, 50-56,59,60, 63,64 |

Form PCT/ISA/210 (continuation of second sheet) (April 2007)

**INTERNATIONAL SEARCH REPORT**

International application No.
PCT/JP2008/053909

Continuation of A. CLASSIFICATION OF SUBJECT MATTER
(International Patent Classification (IPC))

*A61K45/06*(2006.01)i, *A61K31/136*(2006.01)i, *A61K31/198*(2006.01)i, *A61K31/416*(2006.01)i, *A61K31/436*(2006.01)i, *A61K31/4439*(2006.01)i, *A61K31/454*(2006.01)i, *A61K31/4745*(2006.01)i, *A61K31/475*(2006.01)i, *A61K31/496*(2006.01)i, *A61K31/506*(2006.01)i, *A61K31/513*(2006.01)i, *A61K31/5377*(2006.01)i, *A61K31/69*(2006.01)i, *A61K31/704*(2006.01)i, *A61K31/7048*(2006.01)i, *A61K31/7068*(2006.01)i, *A61K38/00*(2006.01)i, *A61K39/395*(2006.01)i, *A61P35/00*(2006.01)i, *A61P35/02*(2006.01)i, *A61P43/00*(2006.01)i

(According to International Patent Classification (IPC) or to both national classification and IPC)

Continuation of B. FIELDS SEARCHED
Minimum documentation searched (International Patent Classification (IPC))

A61K39/395, A61P35/00, A61P35/02, A61P43/00

Minimum documentation searched (classification system followed by classification symbols)

Form PCT/ISA/210 (extra sheet) (April 2007)

**INTERNATIONAL SEARCH REPORT**

International application No.
PCT/JP2008/053909

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 41-49
because they relate to subject matter not required to be searched by this Authority, namely:
Claims 41 to 49 pertain to a method for treatment of a human body by therapy and thus relate to a subject matter which this International Searching Authority is not required, under the provisions of PCT Rule 39.1(iv), to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet.

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
Parts of 1-18, 21-40, 50-56, 59, 60, 63 and 64 which relate to a fms-like tyrosine kinase inhibitor represented by the formula (I).

**Remark on Protest** the
☐ The additional search fees were accompanied by the applicant's protest and, where applicable, payment of a protest fee.
☐ The additional search fees were accompanied by the applicant's protest but the applicable protest fee was not paid within the time limit specified in the invitation.

Form PCT/ISA/210 (continuation of first sheet (2)) (April 2007)

INTERNATIONAL SEARCH REPORT

International application No.
PCT/JP2008/053909

Continuation of Box No.III of continuation of first sheet(2)

Claims 1-40 and 50-66 disclose a pharmaceutical composition comprising a combination of a fms-like tyrosine kinase inhibitor represented by each of the distinct formulae (I)-(III) and at least one compound, and a common matter among the three inventions appears to be "a pharmaceutical composition comprising a combination of a fms-like tyrosine kinase inhibitor and at least one compound".

However, as disclosed in Documents 1-9 shown below, the common matter is not novel. Therefore, the distinct three inventions cannot be regarded as being so linked as to form a single general inventive concept.

Such being the case, the international search report is prepared only on the part of the inventions of claims 1-18, 21-40, 50-56, 59, 60, 63 and 64 which relate to a fms-like tyrosine kinase inhibitor represented by the formula (I).

Document 1: ABRAMS,T.J. et al, Preclinical evaluation of the tyrosine kinase inhibitor SU11248 as a single agent and in combination with "standard of care" therapeutic agents for the treatment of breast cancer, Mol Cancer Ther, 2003, Vol.2, No.10, p.1011-21

Document 2: LEVIS,M. et al, In vitro studies of a FLT3 inhibitor combined with chemotherapy: sequence of administration is important to achieve synergistic cytotoxic effects, Blood, 2004, Vol.104, No.4, p.1145-50

Document 3: ALESKOG,A. et al, In vitro activity of the flt3-inhibitor su5614 and standard cytotoxic agents in tumour cells from patients with wild type and mutated flt3 acute myeloid leukaemia, Leuk Res, 2005, Vol.29, No.9, p.1079-81

Document 4: WO 2006/135629 A1 (JANSSEN PHARM NV,BE) 21 December, 2006 (21.12.06)

Document 5: GEORGE,P. et al, Cotreatment with 17-allylamino-demethoxygeldanamycin and FLT-3 kinase inhibitor PKC412 is highly effective against human acute myelogenous leukemia cells with mutant FLT-3, Cancer Res, 2004, Vol.64, No.10, p.3645-52

Document 6: MOHI,M.G. et al, Combination of rapamycin and protein tyrosine kinase (PTK) inhibitors for the treatment of leukemias caused by oncogenic PTKs, Proc Natl Acad Sci U S A, 2004, Vol.101, No.9, p.3130-5

Document 7: WO 2007/010013 A2 (NOVARTIS AG,CH) 25 January, 2007 (25.01.07)

Document 8: BAGRINTSEVA,K. et al, FLT3-ITD-TKD dual mutants associated with AML confer resistance to FLT3 PTK inhibitors and cytotoxic agents by overexpression of Bcl-x(L), Blood, 2005, Vol.105, No.9, p.3679-85

Document 9: BALI,P. et al, Superior activity of the combination of histone deacetylase inhibitor LAQ824 and the FLT-3 kinase inhibitor PKC412 against human acute myelogenous leukemia cells with mutant FLT-3, Clin Cancer Res, 2004, Vol.10, No.15, p.4991-7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2032059 A
- WO 0153268 A
- WO 0210137 A
- WO 0102369 A
- WO 02083648 A
- WO 03101968 A
- WO 2004094388 A
- WO 2004050088 A
- WO 20050137171 A
- WO 2005094823 A
- WO 9201675 A
- WO 9414780 A
- JP 10087492 A
- WO 9919305 A
- WO 9932117 A
- US 5935966 A
- WO 9941253 A
- WO 0039101 A
- WO 0117995 A
- WO 0220495 A
- WO 0222601 A
- WO 0222602 A
- WO 0222608 A
- WO 0330909 A
- WO 0262789 A
- WO 0230358 A
- WO 0100213 A
- JP 2003523942 W
- DE 2141063
- WO 04108672 A
- WO 05039564 A
- WO 06120557 A
- WO 2005012257 A
- WO 2005012258 A
- WO 2005095382 A
- WO 2005095341 A
- WO 2005000778 A


### Non-patent literature cited in the description


- *Cell,* 1991, vol. 65, 1143
- *Leukemia,* 1997, vol. 11, 1447
- *Blood,* 2000, vol. 96, 3907
- *Blood,* 2001, vol. 97, 2434
- *Khimiya Geterotsiklicheskikh Soedinenii,* 1978, vol. 7, 957
- *Heterocycles,* 1997, vol. 45, 2217
- *Bioorganic & Medicinal Chemistry Letters,* 2004, vol. 14, 4505
- Clinical oncology. Japanese Journal of Cancer and Chemotherapy Publishers Inc
- *Clinical Cancer Research,* 2004, vol. 10, 4991
- *Blood,* 2004, vol. 104, 1145
- *Proceeding of National Academy of Science of the United States of America,* 2004, vol. 101, 3130
- *Blood,* 2004, vol. 104, 4202
- *J. Med. Chem.,* 1997, vol. 40, 2011-2016
- *Drug Dev. Res.,* 1995, vol. 34, 220-230
- *Advances in Drug Res.,* 1984, vol. 13, 224-331
- **Bundgaard.** Design of Prodrugs. Elsevier Press, 1985
- *International Journal of Radiation Oncology, Biology, Physics,* 1979, vol. 5, 85-91
- *International Journal of Radiation Oncology, Biology, Physics,* 1979, vol. 5, 85
- *International Journal of Radiation Oncology, Biology, Physics,* 1979, vol. 85, 1145